# EUROPEAN PATENT APPLICATION

(11) **EP 4 588 920 A1**
(43) Date of publication of application: **23.07.2025**
(21) Application number: 23870676.6
(22) Date of filing: 25.09.2023
(51) Int. Cl.: C07D 471/16, C07D 471/14, C07D 491/16, C07D 487/16, C07D 487/14, C07D 491/14, C07D 491/147, C07D 413/14, A61K 31/4353, A61K 31/5377, A61P 35/00

(54) **NOVEL PRMT5 INHIBITOR AND USE THEREOF**

(30) Priority: 26.09.2022 CN 202211173943; 22.11.2022 CN 202211473887; 17.01.2023 CN 202310080068; 14.04.2023 CN 202310402301
(71) Applicant: Shanghai Apeiron Therapeutics Company Limited, Shanghai 201210 (CN)
(72) Inventor: YAO, Bing, Shanghai 201210 (CN); GU, Xiaohui, Shanghai 201210 (CN); ZHANG, Junqing, Shanghai 201210 (CN); XIE, Shanshan, Shanghai 201210 (CN); CHENG, Kai, Shanghai 201210 (CN)
(74) Representative: Bayramoglu et al.
(86) International application number: PCT/CN2023/120946
(87) International publication number: WO 2024/067445

(57) **Abstract**

The present invention describes a novel molecule having protein arginine methyltransferase 5 inhibitory activity, and synthetic and use methods of the compound. Specifically, the present invention describes the compound of formula (I) or a pharmaceutically acceptable salt, a hydrate or a solvate thereof and synthetic and use methods of the compound.

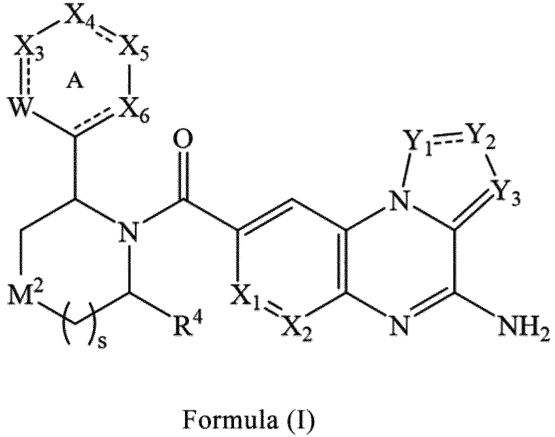

## Description

This application claims the priorities of the following Chinese patent applications: 1) A Chinese patent application, with the application number of 202211173943.5, filed to China National Intellectual Property Administration on September 26, 2022 and titled "Novel PRMT5 Inhibitor and Use thereof"; 2) A Chinese patent application, with the application number of 202211473887.7, filed to China National Intellectual Property Administration on November 22, 2022 and titled "Novel PRMT5 Inhibitor and Use thereof"; 3) A Chinese patent application, with the application number of 202310080068.4, filed to China National Intellectual Property Administration on January, 17, 2023 and tiled "Novel PRMT5 Inhibitor and Use thereof"; and 4) A Chinese patent application, with the application number of 202310402301.6, filed to China National Intellectual Property Administration on April, 14, 2023 and tiled "Novel PRMT5 Inhibitor and Use thereof". The entire contents of the above Chinese patent applications are incorporated herein by reference.

### TECHNICAL FIELD

This application belongs to the field of drug synthesis, specifically to a PRMT5 inhibitor and use thereof.

### BACKGROUND ART

Epigenetic alterations are key mediators that drive and maintain the malignant phenotype of tumors. Changes in DNA methylation, histone acetylation and methylation, noncoding RNA, and post-translational modifications are all epigenetic driving forces of cancer development, independent of changes in the DNA sequence. Arginine methylation is an important kind of post-translational modifications that affect cell growth and proliferation, apoptosis, angiogenesis and metastasis by regulating transcription and post-transcriptional RNA processing. There are three types of methylarginine, ω-NG, N'G-asymmetric dimethylarginine (ADMA) and ω-NG, N'G-symmetric dimethylarginine (SDMA). This modification is catalyzed by the protein arginine methyltransferase (PRMT) family, which transfers methyl from S-adenosylmethionine (AdoMet) to histone and nonhistone arginine side chains. Nine PRMT genes are annotated in the human genome, and classified as Type I (PRMT1, 2, 3, 4, 6 and 8), Type II (PRMT5 and PRMT9), and Type III enzyme (PRMT7) based on the type of methylarginine produced. PRMT5 is primarily a type II enzyme that catalyzes the symmetric dimethylation of arginine. PRMT5 was first discovered in a two-hybrid experiment that test the proteins interacting with Janus tyrosine kinase (Jak2).

PRMT5 is a universal transcriptional repressor that forms complexes with other transcription factors, including BRG 1 and Hbrm, Blimp1 and Snail. PRMT5 participates in different cell biological processes through methylation of substrates in the cytoplasm and nucleus, including histone H4 residue Arg3 (H4R3) and H3 residue Arg8 (H3R8). H4R3 methylation is associated with transcriptional repression, while H3R8 methylation is considered to be associated with both transcriptional activation and transcriptional repression. In addition to direct induction of inhibitory histone labeling by PRMT5, the role of this enzyme in gene silencing is mediated through the formation of multiple inhibitory protein complexes including NuRD fractions, HDACs, MDB proteins and DNA methyltransferase. PRMT5 affects its substrate specificity by interacting with some binding proteins. The core component of this protein complex is MEP50. MEP50 is required for the enzymatic activity of PRMT5. Studies have found that PRMT5 can methylate proteins participating RNA splicing, such as SmD3, which can be used to track the chemical activity of PRMT5 in cellular organism.

PRMT5 plays an important role in tumorigenesis. Studies have found that PRMT5 expression is up-regulated in a variety of tumors, including lymphoma, lung cancer, breast cancer and colorectal cancer. In addition, PRMT5 expression is increased in the samples of mantle cell lymphoma (MCL) patient, and PRMT5 knockout can inhibit MCL cell proliferation, indicating that PRMT5 plays an important role in MCL. Overexpression of PRMT5 promotes cell proliferation which is inhibited by PRMT5 knockout in the cell lines of melanoma, breast and lung cancer. Therefore, PRMT5 is a potential target for cancer therapy.

Loss of methylthioadenosine phosphorylase (MTAP) makes the cells selectively dependent on PRMT5 and its binding protein of WDR77. MTAP is often lost due to its proximity to the tumor suppressor gene of CDKN2A which is generally missing. The concentration of methionine adenosine (MTA, the metabolite cleaved by MTAP) is increased in the cells with MTAP deficiency. MTA has a similar structure to S-adenosylmethionine (SAM). With the increase of concentration, MTA acts as an intrinsic selective inhibitor to inhibit the binding of SAM to PRMT5, thus inhibiting the methyltransferase activity of PRMT5.

The most significant structural difference between MTAP-deficient cancer cells and MTAP wild-type cancer cells is the PRMT5-MTA complex produced in MTAP-deficient cancer cells due to accumulation of MTA concentration. The inhibitor developed for the PRMT5-MTA complex can selectively target cancer cells with MTAP deficiency, with little effect on normal cells, which greatly improves the therapeutic index.

Thus, the identification and development of small molecules that inhibit PRMT5 activity would be useful as a therapeutic method for treating various PRMT5-related diseases or disorders (e.g., cancer).

### CONTENT OF THE INVENTION

In order to solve the technical problem of the present invention, the present invention provides a type of novel structural compounds with excellent inhibitory activity against PRMT5.

Specifically, in one embodiment, the present invention provides a compound, a pharmaceutically acceptable salt, an ester, a prodrug, a stereoisomer or an isotopic derivative thereof, having the structure of formula (I): 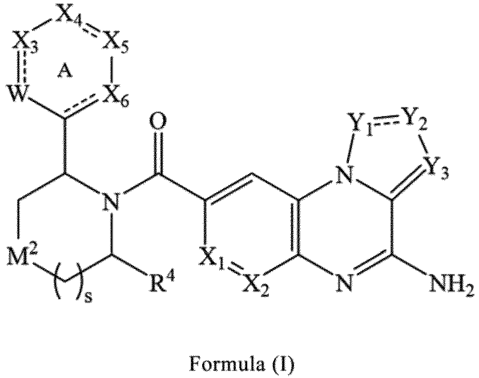
wherein W represents N or CR^{W};
wherein X₃ represents N or CR^{X3}; X₄ represents N or CR^{X4}; X₅ represents N or CR^{X5}; and X₆ represents N or CR^{X6};
Wherein, when X₃ represents CR^{X3}, R^{X3} represents hydrogen, deuterium, C₁-C₆ alkyl, deuterated C₁-C₆ alkyl, C₂-C₆ alkenyl, C₂-C₆ alkynyl, halogen, -OR^{a}, -SR^{a}, -S(O)₂R^{a}, -S(O)R^{a}, -P(O) R^{a}R^{b}, -CN, -SF₅, -NR^{a}R^{b}, C₁-C₆ haloalkyl, C₁-C₆ haloalkoxy, hydroxy (C₁-C₆ alkyl) or C₃-C₁₀ cycloalkyl, C₆-C₁₀ cycloalkenyl, 4- to 10-membered heterocycloalkyl, 6- to 10-membered heterocycloalkenyl, C₆-C₁₀ aryl, 5- to 10-membered heteroaryl, wherein the said C₃-C₁₀ cycloalkyl, C₆-C₁₀ cycloalkenyl, 4- to 10-membered heterocycloalkyl, 6-to 10-membered heterocycloalkenyl, C₆-C₁₀ aryl and 5- to 10-membered heteroaryl can be furtherly substituted with 0-4 substituents selected from: deuterium, halogen, C₁-C₆ alkyl, C₂-C₆ alkenyl, C₂-C₆ alkynyl, C₃-C₁₀ cycloalkyl, -OR^{a}, oxo, hydroxy(C₁-C₆ alkyl), NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, OC(O)NR^{a}R^{b}, -NR^{a}COR^{b} or -CONR^{a}R^{b};
wherein, when X₄ represents CR^{X4}, R^{X4} represents hydrogen, deuterium, C₁-C₆ alkyl, deuterated C₁-C₆ alkyl, C₂-C₆ alkenyl, C₂-C₆ alkynyl, halogen, -OR^{a}, -SR^{a}, -S(O)₂R^{a}, -S(O)R^{a}, -P(O) R^{a}R^{b}, -CN, -SF₅, -NR^{a}R^{b}, C₁-C₆ haloalkyl, C₁-C₆ haloalkoxy, hydroxy (C₁-C₆ alkyl) or C₃-C₁₀ cycloalkyl, C₆-C₁₀ cycloalkenyl, 4- to 10-membered heterocycloalkyl, 6- to 10-membered heterocycloalkenyl, C₆-C₁₀ aryl, 5- to 10-membered heteroaryl, wherein the said C₃-C₁₀ cycloalkyl, C₆-C₁₀ cycloalkenyl, 4- to 10-membered heterocycloalkyl, 6-to 10-membered heterocycloalkenyl, C₆-C₁₀ aryl and 5- to 10-membered heteroaryl can be furtherly substituted with 0-4 substituents selected from: deuterium, halogen, C₁-C₆ alkyl, C₂-C₆ alkenyl, C₂-C₆ alkynyl, C₃-C₆ cycloalkyl, -OR^{a}, oxo, hydroxy(C₁-C₆ alkyl), NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, OC(O)NR^{a}R^{b}, -NR^{a}COR^{b} or -CONR^{a}R^{b};
wherein, when X₅ represents CR^{X5}, R^{X5} represents hydrogen, deuterium, C₁-C₆ alkyl, deuterated C₁-C₆ alkyl, C₂-C₆ alkenyl, C₂-C₆ alkynyl, halogen, -OR^{a}, -SR^{a}, -S(O)₂R^{a}, -S(O)R^{a}, -P(O) R^{a}R^{b}, -CN, -SF₅, -NR^{a}R^{b}, C₁-C₆ haloalkyl, C₁-C₆ haloalkoxy, hydroxy (C₁-C₆ alkyl) or C₃-C₁₀ cycloalkyl, C₆-C₁₀ cycloalkenyl, 4- to 10-membered heterocycloalkyl, 6- to 10-membered heterocycloalkenyl, C₆-C₁₀ aryl, 5- to 10-membered heteroaryl, wherein the said C₃-C₁₀ cycloalkyl, C₆-C₁₀ cycloalkenyl, 4- to 10-membered heterocycloalkyl, 6-to 10-membered heterocycloalkenyl, C₆-C₁₀ aryl and 5- to 10-membered heteroaryl can be furtherly substituted with 0-4 substituents selected from: deuterium, halogen, C₁-C₆ alkyl, C₂-C₆ alkenyl, C₂-C₆ alkynyl, C₃-C₁₀ cycloalkyl, -OR^{a}, oxo, hydroxy(C₁-C₆ alkyl), NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, OC(O)NR^{a}R^{b}, -NR^{a}COR^{b} or -CONR^{a}R^{b};
wherein, when X₆ represents CR^{X6}, R^{X6} represents hydrogen, deuterium, C₁-C₆ alkyl, deuterated C₁-C₆ alkyl, C₂-C₆ alkenyl, C₂-C₆ alkynyl, halogen, -OR^{a}, -SR^{a}, -S(O)₂R^{a}, -S(O)R^{a}, -P(O) R^{a}R^{b}, -CN, -SF₅, -NR^{a}R^{b}, C₁-C₆ haloalkyl, C₁-C₆ haloalkoxy, hydroxy (C₁-C₆ alkyl) or C₃-C₁₀ cycloalkyl, C₆-C₁₀ cycloalkenyl, 4- to 10-membered heterocycloalkyl, 6- to 10-membered heterocycloalkenyl, C₆-C₁₀ aryl, 5- to 10-membered heteroaryl, wherein the said C₃-C₁₀ cycloalkyl, C₆-C₁₀ cycloalkenyl, 4- to 10-membered heterocycloalkyl, 6-to 10-membered heterocycloalkenyl, C₆-C₁₀ aryl and 5- to 10-membered heteroaryl can be furtherly substituted with 0-4 substituents selected from: deuterium, halogen, C₁-C₆ alkyl, C₂-C₆ alkenyl, C₂-C₆ alkynyl, C₃-C₁₀ cycloalkyl, -OR^{a}, oxo, hydroxy(C₁-C₆ alkyl), NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, OC(O)NR^{a}R^{b}, -NR^{a}COR^{b} or -CONR^{a}R^{b};
wherein ring A may also be optionally fused with a 5- to 6-membered saturated or unsaturated ring at the chemical bond between X₃ and X₄, which may contain 0-3 heteroatoms selected from O, N and S;
wherein ring A may also be optionally fused with a 5- to 6-membered saturated or unsaturated ring at the chemical bond between X₄ and X₅, which may contain 0-3 heteroatoms selected from O, N and S;
wherein ring A may also be optionally fused with a 5- to 6-membered saturated or unsaturated ring at the chemical bond between X₅ and X₆, which may contain 0-3 heteroatoms selected from O, N and S;
wherein, when W represents CR^{W}, R^{W} is selected from hydrogen, deuterium, C₁-C₆ alkyl, deuterided C₁-C₆ alkyl, halogen, -OR^{a}, -SR^{a}, -S(O)₂R^{a}, -S(O)R^{a}, -CN, -SF₅, -NR^{a}R^{b}, C₁-C₆ haloalkyl, hydroxy(C₁-C₆ alkyl), and C₁-C₆ haloalkoxy;
wherein X₁ represents N or CR^{X1};
wherein X₂ represents N or CR^{X2};
wherein Y₁ represents CR^{Y1}R^{Y1'}, NR^{Y1}, O, S, Se;
wherein Y₂ represents CR^{Y2}R^{Y2'}, NR^{Y2}, O, S, Se;
wherein Y₃ represents CR^{Y3}R^{Y3'}, NR^{Y3}, O, S, Se;
wherein R^{X1} and R^{X2} each independently represent hydrogen, deuterium, C₁-C₆ alkyl, deuterated C₁-C₆ alkyl, C₁-C₆ haloalkyl, C₁-C₆ haloalkoxy, hydroxy(C₁-C₆ alkyl), C₂-C₆ alkenyl, C₂-C₆ alkynyl, -OR^{a}, -SR^{a}, -S(O)₂R^{a}, -S(O)R^{a}, -CN, -OC(O)R^{a}, -OCONR^{a}R^{b}, halogen, -SO₃R^{a}, -NR^{a}R^{b} and -SF₅;
wherein R^{Y1}, R^{Y1'}, R^{Y2}, R^{Y2'}, R^{Y3}, and R^{Y3'} each independently represent absent, hydrogen, deuterium, C₁-C₆ alkyl, deuterated C₁-C₆ alkyl, C₁-C₆ haloalkyl, C₁-C₆ haloalkoxy, hydroxy(C₁-C₆ alkyl), C₂-C₆ alkenyl, C₂-C₆ alkynyl, C₃-C₆ cycloalkyl, -OR^{a}, -SR^{a}, -S(O)₂R^{a}, -S(O)R^{a}, -CN, -OC(O)R^{a}, -OCONR^{a}R^{b}, halogen, -SO₃R^{a}, -NR^{a}R^{b}, and -SF₅;
wherein M² represents CR^{M1}R^{M2}, S, O and NR^{M1};
wherein R^{M1} and R^{M2} each independently represent hydrogen, deuterium, C₁-C₆ alkyl, halogen, or R^{M1} and R^{M2}, together with the atom to which they are attached, form a 3- to 14-membered saturated or unsaturated ring which may optionally contain 0-2 heteroatoms selected from O, S, N;
wherein R⁴ represents hydrogen, C₁-C₆ alkyl, deuterated C₁-C₆ alkyl, C₃-C₆ cycloalkyl or halogen;
wherein -̅ -̅ -̅ represents a single bond or a double bond;
wherein s represents an integer of 0 to 3;
wherein R^{a} and R^{b} each independently represent hydrogen, deuterium, halogen, C₁-C₆ alkyl, C₃-C₆ cycloalkyl, C₁-C₆ haloalkyl, or R^{a} and R^{b}, together with the atom to which they are attached, form a 3- to 14-membered saturated or unsaturated ring which may optionally contain 0-2 heteroatoms selected from O, S, N.

In another embodiment, the present invention provides a compound, a pharmaceutically acceptable salt, an ester, a prodrug, a stereoisomer or an isotopic derivative thereof, having the structure of formula (II): 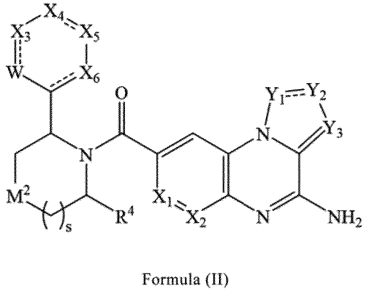
wherein W represents N or CR^{W};
wherein X₃ represents N or CR^{X3}; X₄ represents N or CR^{X4}; X₅ represents N or CR^{X5}; and X₆ represents N or CR^{X6},
Wherein, when X₃ represents CR^{X3}, R^{X3} represents hydrogen, deuterium, C₁-C₆ alkyl, deuterated C₁-C₆ alkyl, C₂-C₆ alkenyl, C₂-C₆ alkynyl, halogen, -OR^{a}, -SR^{a}, -S(O)₂R^{a}, -S(O)R^{a}, -P(O) R^{a}R^{b}, -CN, -SF₅, -NR^{a}R^{b}, C₁-C₆ haloalkyl, C₁-C₆ haloalkoxy, hydroxy (C₁-C₆ alkyl) or C₃-C₁₀ cycloalkyl, C₆-C₁₀ cycloalkenyl, 4- to 10-membered heterocycloalkyl, 6- to 10-membered heterocycloalkenyl, C₆-C₁₀ aryl, 5- to 10-membered heteroaryl, wherein the said C₃-C₁₀ cycloalkyl, C₆-C₁₀ cycloalkenyl, 4- to 10-membered heterocycloalkyl, 6-to 10-membered heterocycloalkenyl, C₆-C₁₀ aryl and 5- to 10-membered heteroaryl can be furtherly substituted with 0-4 substituents selected from: deuterium, halogen, C₁-C₆ alkyl, C₂-C₆ alkenyl, C₂-C₆ alkynyl, C₃-C₁₀ cycloalkyl, -OR^{a}, oxo, hydroxy(C₁-C₆ alkyl), NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, OC(O)NR^{a}R^{b}, -NR^{a}COR^{b} or -CONR^{a}R^{b};
wherein, when X₄ represents CR^{X4}, R^{X4} represents hydrogen, deuterium, C₁-C₆ alkyl, deuterated C₁-C₆ alkyl, C₂-C₆ alkenyl, C₂-C₆ alkynyl, halogen, -OR^{a}, -SR^{a}, -S(O)₂R^{a}, -S(O)R^{a}, -P(O) R^{a}R^{b}, -CN, -SF₅, -NR^{a}R^{b}, C₁-C₆ haloalkyl, C₁-C₆ haloalkoxy, hydroxy (C₁-C₆ alkyl) or C₃-C₁₀ cycloalkyl, C₆-C₁₀ cycloalkenyl, 4- to 10-membered heterocycloalkyl, 6- to 10-membered heterocycloalkenyl, C₆-C₁₀ aryl, 5- to 10-membered heteroaryl, wherein the said C₃-C₁₀ cycloalkyl, C₆-C₁₀ cycloalkenyl, 4- to 10-membered heterocycloalkyl, 6-to 10-membered heterocycloalkenyl, C₆-C₁₀ aryl and 5- to 10-membered heteroaryl can be furtherly substituted with 0-4 substituents selected from: deuterium, halogen, C₁-C₆ alkyl, C₂-C₆ alkenyl, C₂-C₆ alkynyl, C₃-C₆ cycloalkyl, -OR^{a}, oxo, hydroxy(C₁-C₆ alkyl), NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, OC(O)NR^{a}R^{b}, -NR^{a}COR^{b} or -CONR^{a}R^{b};
wherein, when X₅ represents CR^{X5}, R^{X5} represents hydrogen, deuterium, C₁-C₆ alkyl, deuterated C₁-C₆ alkyl, C₂-C₆ alkenyl, C₂-C₆ alkynyl, halogen, -OR^{a}, -SR^{a}, -S(O)₂R^{a}, -S(O)R^{a}, -P(O) R^{a}R^{b}, -CN, -SF₅, -NR^{a}R^{b}, C₁-C₆ haloalkyl, C₁-C₆ haloalkoxy, hydroxy (C₁-C₆ alkyl) or C₃-C₁₀ cycloalkyl, C₆-C₁₀ cycloalkenyl, 4- to 10-membered heterocycloalkyl, 6- to 10-membered heterocycloalkenyl, C₆-C₁₀ aryl, 5- to 10-membered heteroaryl, wherein the said C₃-C₁₀ cycloalkyl, C₆-C₁₀ cycloalkenyl, 4- to 10-membered heterocycloalkyl, 6-to 10-membered heterocycloalkenyl, C₆-C₁₀ aryl and 5- to 10-membered heteroaryl can be furtherly substituted with 0-4 substituents selected from: deuterium, halogen, C₁-C₆ alkyl, C₂-C₆ alkenyl, C₂-C₆ alkynyl, C₃-C₁₀ cycloalkyl, -OR^{a}, oxo, hydroxy(C₁-C₆ alkyl), NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, OC(O)NR^{a}R^{b}, -NR^{a}COR^{b} or -CONR^{a}R^{b};
wherein, when X₆ represents CR^{X6}, R^{X6} represents hydrogen, deuterium, C₁-C₆ alkyl, deuterated C₁-C₆ alkyl, C₂-C₆ alkenyl, C₂-C₆ alkynyl, halogen, -OR^{a}, -SR^{a}, -S(O)₂R^{a}, -S(O)R^{a}, -P(O) R^{a}R^{b}, -CN, -SF₅, -NR^{a}R^{b}, C₁-C₆ haloalkyl, C₁-C₆ haloalkoxy, hydroxy (C₁-C₆ alkyl) or C₃-C₁₀ cycloalkyl, C₆-C₁₀ cycloalkenyl, 4- to 10-membered heterocycloalkyl, 6- to 10-membered heterocycloalkenyl, C₆-C₁₀ aryl, 5- to 10-membered heteroaryl, wherein the said C₃-C₁₀ cycloalkyl, C₆-C₁₀ cycloalkenyl, 4- to 10-membered heterocycloalkyl, 6-to 10-membered heterocycloalkenyl, C₆-C₁₀ aryl and 5- to 10-membered heteroaryl can be furtherly substituted with 0-4 substituents selected from: deuterium, halogen, C₁-C₆ alkyl, C₂-C₆ alkenyl, C₂-C₆ alkynyl, C₃-C₁₀ cycloalkyl, -OR^{a}, oxo, hydroxy(C₁-C₆ alkyl), NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, OC(O)NR^{a}R^{b}, -NR^{a}COR^{b} or -CONR^{a}R^{b};
wherein, when W represents CR^{W}, R^{W} is selected from hydrogen, deuterium, C₁-C₆ alkyl, deuterated C₁-C₆ alkyl, halogen, -OR^{a}, -SR^{a}, -S(O)₂R^{a}, -S(O)R^{a}, -CN, -SF₅, -NR^{a}R^{b}, C₁-C₆ haloalkyl, hydroxy(C₁-C₆ alkyl), and C₁-C₆ haloalkoxy;
wherein X₁ represents N or CR^{X1};
wherein X₂ represents N or CR^{X2};
wherein Y₁ represents CR^{Y1}R^{Y1'}, NR^{Y1}, O, S, Se;
wherein Y₂ represents CR^{Y2}R^{Y2'}, NR^{Y2}, O, S, Se;
wherein Y₃ represents CR^{Y3}R^{Y3'}**,** NR^{Y3}, O, S, Se;
wherein R^{X1} and R^{X2} each independently represent hydrogen, deuterium, C₁-C₆ alkyl, deuterated C₁-C₆ alkyl, C₁-C₆ haloalkyl, C₁-C₆ haloalkoxy, hydroxy(C₁-C₆ alkyl), C₂-C₆ alkenyl, C₂-C₆ alkynyl, -OR^{a}, -SR^{a}, -S(O)₂R^{a}, -S(O)R^{a}, -CN, -OC(O)R^{a}, -OCONR^{a}R^{b}, halogen, -SO₃R^{a}, -NR^{a}R^{b} and -SF₅;
wherein R^{Y1}, R^{Y1'}, R^{Y2}, R^{Y2'}, R^{Y3}, and R^{Y3'} each independently represent absent, hydrogen, deuterium, C₁-C₆ alkyl, deuterated C₁-C₆ alkyl, C₁-C₆ haloalkyl, C₁-C₆ haloalkoxy, hydroxy(C₁-C₆ alkyl), C₂-C₆ alkenyl, C₂-C₆ alkynyl, C₃-C₆ cycloalkyl, -OR^{a}, -SR^{a}, -S(O)₂R^{a}, -S(O)R^{a}, -CN, -OC(O)R^{a}, -OCONR^{a}R^{b}, halogen, -SO₃R^{a}, -NR^{a}R^{b}, and -SF₅;
wherein M² represents CR^{M1}R^{M2}, S, O and NR^{M1};
wherein R^{M1} and R^{M2} each independently represent hydrogen, deuterium, C₁-C₆ alkyl, halogen, or R^{M1} and R^{M2}, together with the atom to which they are attached, form a 3- to 14-membered saturated or unsaturated ring which may optionally contain 0-2 heteroatoms selected from O, S, N;
wherein R⁴ represents hydrogen, C₁-C₆ alkyl, deuterated C₁-C₆ alkyl, C₃-C₆ cycloalkyl or halogen;
wherein -̅ -̅ -̅ represents a single bond or a double bond;
wherein s represents an integer of 0 to 3;
wherein R^{a} and R^{b} each independently represent hydrogen, deuterium, halogen, C₁-C₆ alkyl, C₃-C₆ cycloalkyl, C₁-C₆ haloalkyl, or R^{a} and R^{b}, together with the atom to which they are attached, form a 3- to 14-membered saturated or unsaturated ring which may optionally contain 0-2 heteroatoms selected from O, S, N.

In a preferred embodiment of the formula I or II, wherein X₁ represents CR^{X1} or N, wherein R^{X1} represents hydrogen, deuterium, halogen, -CN, C₁-C₆ alkyl, deuterated C₁-C₆ alkyl, C₁-C₆ alkoxy, halogenated C₁-C₆ alkyl.

In a preferred embodiment of formula I or II, wherein X₂ represents CH and CD.

In a preferred embodiment of formula I or II, wherein X₃ represents CH, CD or N.

In a preferred embodiment of formula I or II**,** wherein X₄ represents CR^{X4} or N, wherein R^{X4} represents hydrogen, deuterium, C₁-C₆ alkyl, deuterated C₁-C₆ alkyl, C₁-C₆ alkoxy, C₁-C₆ haloalkyl, C₁-C₆ haloalkoxy, halogen, SF₅, -SO₃R^{a}, -SR^{a}, -P(O)R^{a}R^{b}, or cyano or C₃-C₁₀ cycloalkyl, C₆-C₁₀ cycloalkenyl, 4- to 10-membered heterocycloalkyl, 6- to 10-membered heterocycloalkenyl, C₆-C₁₀ aryl, and 5- to 10-membered heteroaryl, wherein the said C₃-C₁₀ cycloalkyl, C₆-C₁₀ cycloalkenyl, 4- to 10-membered heterocycloalkyl, 6-to 10-membered heterocycloalkenyl, C₆-C₁₀ aryl, and 5- to 10-membered heteroaryl can be furtherly substituted with 0-4 substituents selected from: deuterium, halogen, C₁-C₆ alkyl, C₂-C₆ alkenyl, C₂-C₆ alkynyl, C₃-C₆ cycloalkyl, -OR^{a}, oxo, hydroxy(C₁-C₆ alkyl), NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, OC(O)NR^{a}R^{b}, -NR^{a}COR^{b} or -CONR^{a}R^{b}.

In a preferred embodiment of the formula I or II, wherein X₅ represents CR^{X5} or N, wherein R^{X5} represents hydrogen, deuterium, C₁-C₆ alkyl, C₁-C₆ alkoxyl, C₁-C₆ haloalkyl, C₁-C₆ haloalkoxy, halogen, SF₅, or cyano. In a preferred embodiment of formula I or II, wherein X₆ represents CH, CD or N.

In a preferred embodiment of formula I or II, wherein the chemical bond between Y₁ and Y₂ is a double bond.

In a preferred embodiment of formula I or II, wherein Y₁ represents CR^{Y1}, wherein R^{Y1} represents hydrogen, deuterium, C₁-C₆ alkyl, deuterated C₁-C₆ alkyl, C₃-C₆ cycloalkyl, C₁-C₆ alkoxy, C₁-C₆ haloalkyl, C₁-C₆ haloalkoxy, halogen, SF₅, -S(O)₂CH₃ or cyano.

In a preferred embodiment of formula I or II, wherein Y² represents N.

In a preferred embodiment of formula I or II, wherein Y³ represents CH or CD.

In a preferred embodiment of the formula I or II, wherein R⁴ represents hydrogen or C₁-C₆ alkyl or deuterated C₁-C₆ alkyl.

In a preferred embodiment of formula I or II, wherein M² represents O, NH, S.

In the preferred embodiment of formula I or II, wherein M² represents CR^{M1}R^{M2}; wherein R^{M1} and R^{M2} each independently represent hydrogen, deuterium, C₁-C₆ alkyl, halogen, or R^{M1} and R^{M2}, together with the atom to which they are attached, form a 3- to 14-membered saturated or unsaturated ring.

Specifically, the present invention provides the following compounds:

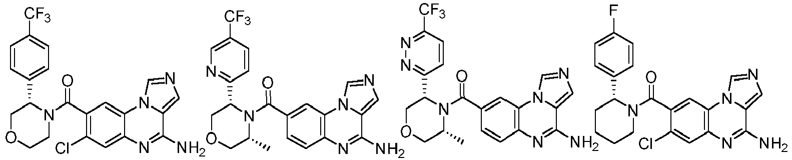

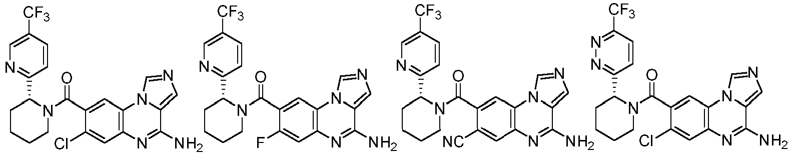

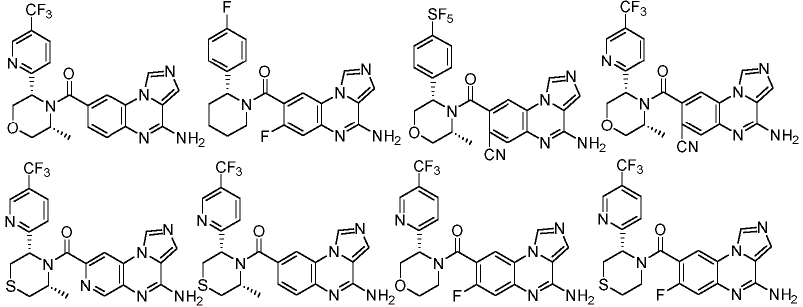

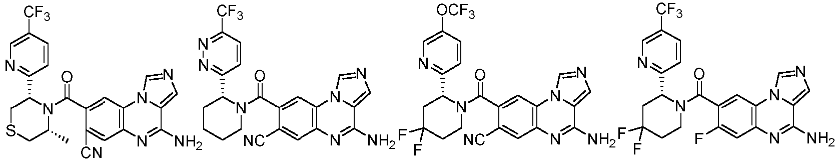

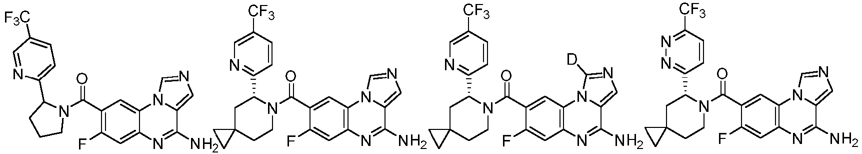

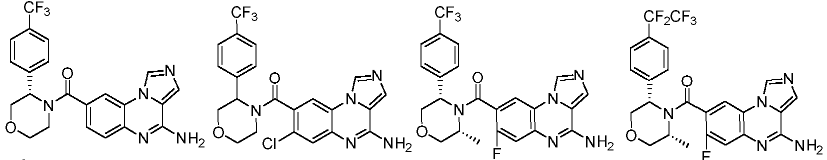

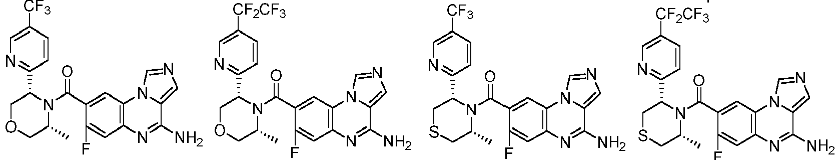

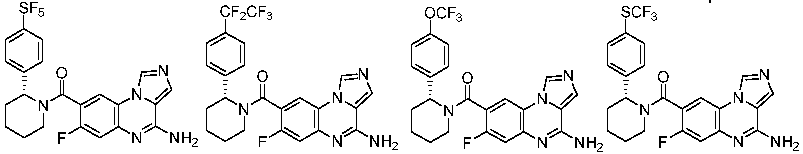

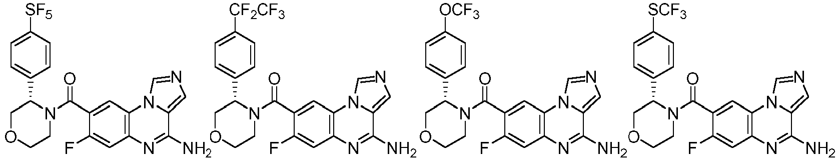

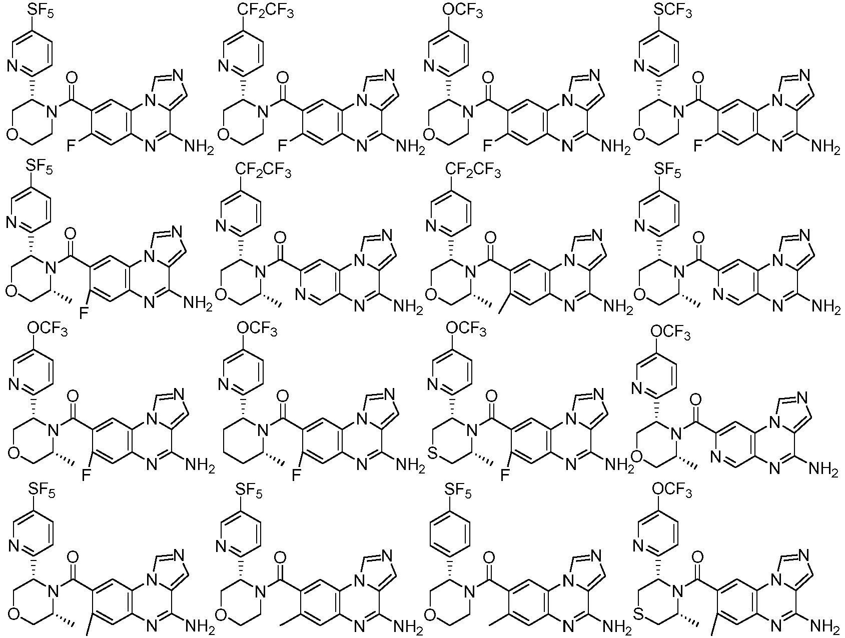

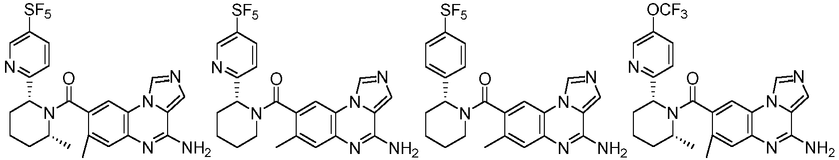

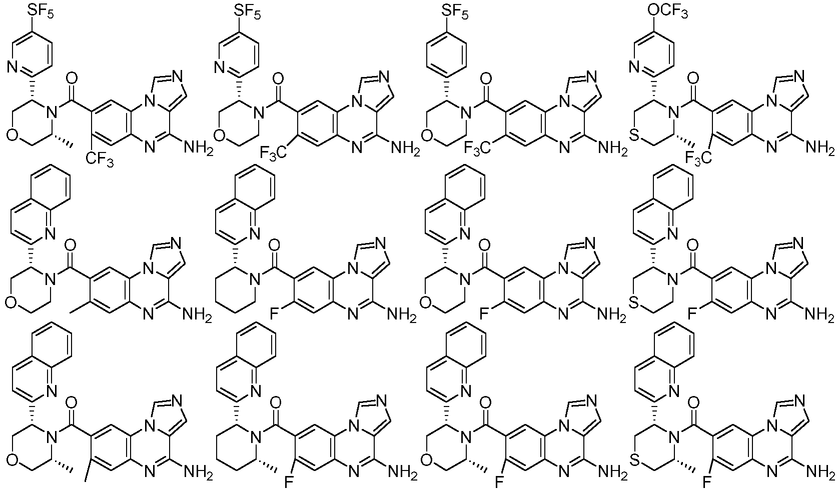

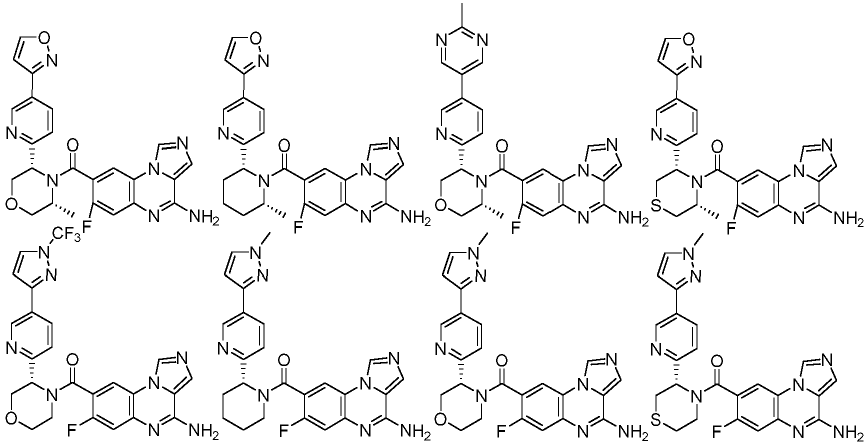

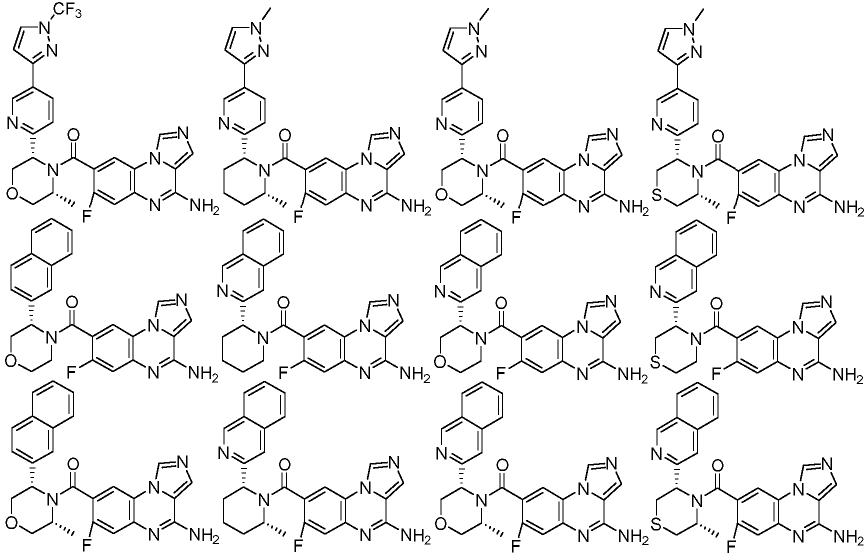

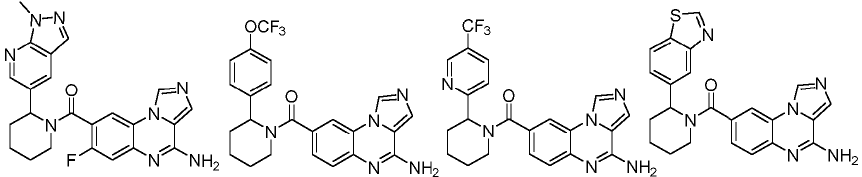

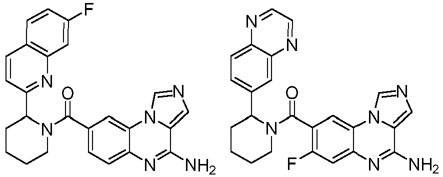

In the above preferred embodiments of the present invention, at least one or more hydrogen atoms in the isotopic derivative are deuterium atoms.

In additional embodiment, the present invention provides a pharmaceutical composition, comprising any one of the compounds mentioned above in the present invention, pharmaceutically acceptable salts, esters, prodrugs, stereoisomers or isotopic derivatives and pharmaceutically acceptable carriers thereof.

Unless otherwise specified, the compounds of the present invention may encompass, in addition to their specific structures, pharmaceutically acceptable salts, stereoisomers, isotopic isomers (e.g., deuterides), solvates, hydrates, prodrugs and metabolites thereof. Thus, the pharmaceutically acceptable salts, stereoisomers, isotope isomers, solvates, hydrates, prodrugs, and metabolites of these compounds are also considered within the scope of protection.

Preferably, the pharmaceutical composition of the present invention may further comprise a second active substance which is an anti-tumor drug including one or more of chemotherapeutic drugs, targeted tumor therapy drugs and tumor therapy antibody drugs.

In addition, the present invention also provides a method for treating diseases by inhibiting the action of PRMT5 with a compound and pharmaceutically acceptable salts, esters, prodrugs, stereoisomers or isotopic derivatives thereof. Preferably, said diseases are tumors.

### Definition:

Unless otherwise specified, the term "alkyl" itself or as part of another substituent refers to linear (i.e. unbranched) or branched, or cyclic hydrocarbyl, or combinations thereof, which may be saturated, mono- or polyunsaturated and may include divalent or multivalent groups having a defined number of carbon atoms (i.e., C₁-C₁₀ refers to one to ten carbon atoms). Examples of saturated hydrocarbyl include, but are not limited to, groups such as methyl, ethyl, n-propyl, isopropyl, n-butyl, t-butyl, isobutyl, sec-butyl, cyclohexyl, cyclohexylmethyl, and cyclopropylmethyl, homologs and isomers such as n-pentyl, n-hexyl, n-heptyl, and n-octyl. An unsaturated alkyl has one or more double or triple bonds. Examples of unsaturated alkyl include, but are not limited to, vinyl, 2-propenyl, crotonyl, 2-isopentenyl, 2-(butadienyl), 2,4-pentadienyl, 3-(1,4-pentadienyl), ethynyl, 1- and 3-propynyl, 3-butynyl, as well as higher homologs and isomers. The alkyl defined as the hydrocarbyl is called "(homoalkyl)". The alkyl is optionally substituted with one or more halogen atoms.

The term "haloalkyl" refers to alkyl as defined above, wherein one or more hydrogen atoms are substituted with halogen atoms.

The term "alkylene" itself or as part of another substituent refers to a divalent group derived from alkyl, such as, but not limited to, -CH₂CH₂CH₂CH₂-, -CH₂CH=CHCH₂-, - CH₂C≡CCH₂- and -CH₂CH₂CH(CH₂CH₂CH₃)CH₂-. The alkyl (or the alkylene) usually has 1 to 24 carbon atoms, and groups having 10 or less carbon atoms are preferred in the present invention. "Low alkyl" or "low alkylene" refers to shorter chain alkyl or alkylene, usually having eight or fewer carbon atoms. The alkylene is optionally substituted with one or more halogen atoms.

The term "alkynyl" refers to a carbon chain containing at least one carbon-carbon triple bond, which may be linear or branched, or a combination thereof. Examples of the alkynyl include ethynyl, propargyl, 3-methyl-1-pentynyl, 2-heptynyl. The alkynyl is optionally substituted with one or more halogen atoms.

The term "cycloalkyl" refers to a monocyclic or bicyclic saturated carbon ring, each having 3 to 10 carbon atoms. "Fused analogue" of cycloalkyl means that a monocyclic ring is fused with aryl or heteroaryl, wherein the linking site is in the non-aromatic portion. Examples of the cycloalkyl and fused analogues thereof include cyclopropyl, cyclobutyl, cyclopentyl, cyclohexyl, tetrahydronaphthyl, decahydronaphthyl, dihydroindenyl. The cycloalkyl is optionally substituted with one or more halogen atoms. Further, the term "cycloalkyl" in present invention includes both bridged ring systems and spiro ring systems.

The term "alkoxy" refers to a straight-chain or branched alkoxyl group having an indicated number of carbon atoms. C₁-₆ alkoxyl, for example, includes methoxyl, ethoxyl, propoxyl, isopropoxyl.

Unless otherwise specified, the term "heteroalkyl" itself or combination with another term refers to a stable straight-chain or branched, or cyclic hydrocarbon consisting of at least one carbon atom and at least one heteroatom selected from O, N, P, Si, S, or a combination thereof, wherein nitrogen atoms, phosphorus atoms or sulfur atoms may optionally be oxidized and the nitrogen atoms may optionally be quaternized. The heteroatoms O, N, P, S and Si may be placed at any position within the heteroalkyl or at positions where the alkyl is linked to the rest of the molecule. For example, the heteroatoms include, but are not limited to, -CH₂-CH₂-O-CH₃, -CH₂-CH₂-NH-CH₃, -CH₂-CH₂-N(CH₃)-CH₃, -CH₂-S-CH₂-CH₃, -CH₂-CH₂, -S(O)-CH₃, -CH₂-CH₂-S(O)₂-CH₃, -CH=CH-O-CH₃, -Si(CH₃)₃, -CH₂-CH=N-OCH₃, -CH=CH-N(CH₃)-CH₃, -O-CH₃, -O-CH₂-CH₃ and -CN. At most two or three heteroatoms may be contiguous. For example, -CH₂-NH-OCH₃ and -CH₂-O-Si(CH₃)₃. Similarly, the term "heteroalkylene" itself or combination with another term refers to a divalent group derived from heteroalkyl such as, but not limited to, -CH₂-CH₂-S-CH₂-CH₂- and -CH₂-S-CH₂-CH₂-NH-CH₂-. For heteroalkylene, the heteroatom may be at either or both ends of the chain (e.g., alkylene oxy, alkylene dioxy, alkylene amino, alkylene diamino). Furthermore, for alkylene and heteroalkylene linking groups, the direction in the molecular formula of the linking groups does not indicate the orientation of the linking groups. For example, the molecular formula -C(O)OR'- represents -C(O)OR'- and -R'OC(O)-. As mentioned above, the heteroalkyl as used herein includes those groups that are linked to the rest of the molecule via a heteroatom, for example -C(O)R', -C(O)NR', -NR'R", -OR', -SR' and/or -SO₂R'. Where reference is made to "heteroalkyl" followed by specific heteroalkyl such as -NR'R", it should be understood that the terms heteroalkyl and -NR'R" do not repeat and are not mutually exclusive. Instead, these specific heteroalkyl groups are referenced for greater clarity. Therefore, the term "heteroalkyl" should not be interpreted herein to exclude specific heteroalkyl such as -NR'R".

The term "cycloalkoxy" refers to cycloalkyl as defined above that is bonded to an oxygen atom, such as cyclopropoxy.

The term "haloalkoxy" refers to alkoxy as defined above in which one or more hydrogen atoms are substituted with halogen.

The term "aryl" refers to a monocycle or bicyclic aryl containing only carbon atoms. The "fused analogue" of aryl refers to the fusing of aryl with monocyclic cycloalkyl or monocyclic heterocyclyl in which the fusing point is located at the aryl. Examples of the aryl and fused ring analogues thereof include phenyl, naphthyl, indanyl, indenyl, tetrahydronaphthalene, 2,3-dihydrobenzofuryl, dihydrobenzopyranyl, 1,4-benzodioxalkyl. The term "heteroaryl" refers to a monocyclic or bicyclic aryl containing at least one heteroatom selected from N, O and S. The "fused analogue" of heteroaryl refers to the fusing of heteroaryl with monocyclic cycloalkyl or monocyclic heterocyclyl in which the fusing point is located at the aryl. Examples of the heteroaryl include pyrrolyl, isozolyl, isothiazolyl, pyrazolyl, pyridinyl, oxazolyl, oxadiazolyl, thiadiazolyl, thiazolyl, imidazolyl, triazolyl, tetrazolyl, furyl, triazinyl, thienyl, pyrimidinyl, pyridazinyl, pyrazinyl, benzoxazolyl, benzothiazolyl, benzimidazolyl, benzofuranyl, benzothiopheneyl, furano(2,3-b)pyridinyl, quinolinyl, indolyl, isoquinolinyl.

"Substituted or unsubstituted": the defined alkyl, aryl and heteroaryl are unsubstituted or substituted with at least one substituent selected from the group consisting of substituents. The substituents are selected from the group consisting of: halogen atoms, alkyl having 1 to 6 carbon atoms, alkoxy having 1 to 6 carbon atoms, haloalkyl having 1 to 6 carbon atoms, haloalkoxyl having 1 to 6 carbon atoms, -CN, alkynyl having 2 to 6 carbon atoms, alkanoyl having 1 to 6 carbon atoms, cycloalkyl having 3 to 7 ring atoms, heteroaryl, aryl, aralkyloxy having 7 to 10 carbon atoms, arylcarbonyl, aminocarbonyl, alkenyl having 2 to 5 carbon atoms, alkylthio having 1 to 6 carbon atoms, aminosulfinyl, aminosulphonyl, hydroxy, -SF₅, hydroxyalkyl having 1 to 4 carbon atoms, nitro, amino, carboxyl, alkoxycarbonyl having 2 to 5 carbon atoms, alkoxyalkyl having 1 to 4 carbon atoms, alkylsulfonyl having 1 to 4 carbon atoms, alkanoylamino having 1 to 4 carbon atoms, an alkanoyl (alkyl)amino having 1 to 6 carbon atoms, an alkanoylamidoalkyl group having 1 to 6 carbon atoms in each of the alkanoyl and alkyl portions, an alkanoyl (alkyl)aminoalkyl having 1 to 6 carbon atoms in each of the alkanoyl and alkyl portions, alkanoyl sulfonamide having 1 to 4 carbon atoms, monoalkylaminocarbonyl or dialkylaminocarbonyl having 1 to 6 carbon atoms, monoalkylaminosulfinyl or dialkylaminesulfinyl having 1 to 6 carbon atoms, monoalkylaminosulfonyl or dialkylaminosulfonyl having 1 to 6 carbon atoms, aminealkyl having 1 to 4 carbon atoms, a monoalkylamino or dialkylaminogroup having 1 to 6 carbon atoms, a monoalkylaminoalkyl or dialkylaminoalkyl having 1 to 6 carbon atoms in each of the alkyl moieties, an aralkyl group having 7 to 10 carbon atoms, a heteroaralkyl group having 1 to 4 carbon atoms in the alkyl portion, heteroarylalkoxy having from 1 to 4 carbon atoms in the alkoxy portion and alkylsulfonamido having from 1 to 4 carbon atoms.

As used herein, the term "heterocycle"or "heterocyclic" or "heterocycloalkyl" or "heterocyclyl" refers to a saturated, partially saturated or unsaturated group (but not aromatic) having a monocyclic or condensed ring (including bridged and spiro systems in which there are 1 to 10 carbon atoms and 1 to 4 heteroatoms selected from nitrogen, sulfur, or oxygen, where one or more of the rings may be cycloalkyl, aryl, or heteroaryl, as long as the linking point passes through a nonaromatic ring. In an example, nitrogen atoms and/or sulfur atoms of the heterocyclic group are optionally oxidized to provide N-oxide, sulfinyl and sulfonyl portions. Examples of the "heterocyclyl" and fused analogues include pyrrolidinyl, piperidinyl, piperazinyl, imidazolidinyl, 2,3-dihydrofuran(2,3-b)pyridyl, benzoxazinyl, tetrahydroquinolinyl, tetrahydroisoquinolinyl, dihydroindolyl. The term also includes non-aromatic, partially unsaturated monocyclic rings such as 2- or 4-pyridones linked via a nitrogen atom or N-substituted -(1H,3H)-pyrimidine-2,4-diones (N-substituted uracils).

As used herein, the term "substituted heterocyclic" or "substituted heterocycloalkyl" or "substituted heterocyclyl" refers to a heterocyclic group substituted with 1 to 5 (e.g., 1 to 3) substituents that are identical to those defined by substituted cycloalkyl.

Unless otherwise specified, the term "halogenated" or "halo" or "halogen" itself or as part of another substituent refers to a fluorine, chlorine, bromine or iodine atom. In addition, the term "halogenated alkyl" refers to including monohalogenated alkyl and polyhalogenated alkyl. For example, the term "halogenated (C₁-C₆) alkyl" refers to including, but not limited to, trifluoromethyl, 2,2,2-trifluoroethyl, 4-chlorobutyl, 3-bromopropyl.

"Prodrugs" refer to substances that are converted into the parent drug in vivo. In some cases, the prodrugs are often used because they are easier administered than the parent drugs. For example, the prodrugs may be bioavailable by oral administration while the parent drugs are not. The prodrugs may also have a higher solubility than the parent drugs in the pharmaceutical composition. An example of the prodrug, but not limited to, may be that any one of the compounds of Formula I is administered in the form of an ester (prodrug) to facilitate transport across cell membranes where water solubility is detrimental to migration and which subsequently metabolically hydrolyzes into the active substance (carboxylic acid) once within cells where water solubility is beneficial. Another example of the prodrug may be a short peptide (polyamino acid) that bonds to an acid group, where the peptide is metabolized to release the active portion.

Optical isomers-diastereomers-geometrical isomers-tautomers:
The compounds of formula (I) contain one or more asymmetric centers and are therefore used as racemates and racemic mixtures, single enantiomers, mixtures of diastereomers and single diastereomers. The present invention should comprise all these isomeric forms of the compounds of formula (I).

Some of the compounds described herein contain an olefinic (olefinic) double bond, which refers to including both E and Z geometrical isomers unless otherwise specified.

Some compounds of the present invention may comprise one or multiple ring systems, and thus cis- and trans-isomers may be present. The present invention aims to contain all of these cis- and trans-isomers.

Some of the compounds described herein may have different sites linked to a hydrogen atom, referred to as tautomers. Such examples may be ketones and enol forms thereof known as keto-enol tautomers. Individual tautomers as well as mixtures thereof are included in the compounds of the present invention.

The compounds of the present invention may be isolated into diastereoisomeric pairs of enantiomers, for example by fractional crystallization from a suitable solvent such as methanol or ethyl acetate or mixtures thereof. A pair of such enantiomers obtained can be separated into individual stereoisomers by conventional methods, for example using optically active amines or acids as resolution reagents or in a chiral HPLC column.

Alternatively, any enantiomers of the compounds of the present invention may be obtained by stereospecific synthesis using optically pure starting materials or reagents of known configuration.

Stable isotope-labeled analogues: one or more protons in the compounds of the present invention may be replaced with deuterium atoms, thereby providing deuterated analogues having improved pharmacological activity.

### Salts and dosage forms

It should be understood that, as used herein, reference to compounds of the present invention also includes pharmaceutically acceptable salts.

### Application

The compounds of the present invention can be used for treating PRMT5-associated diseases.

The compounds of the present invention can be prepared by the following reaction formula:

### Method A:

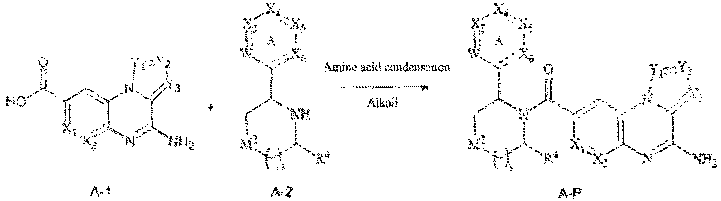

### Method A-SFC

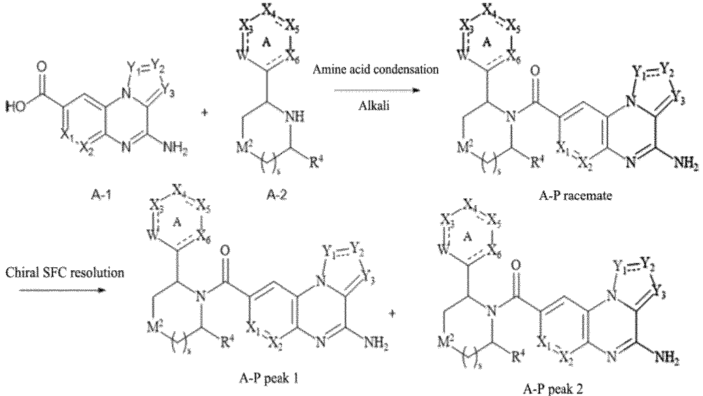

### Method B:

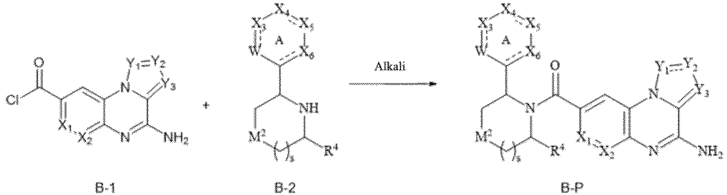

### Method B-SFC

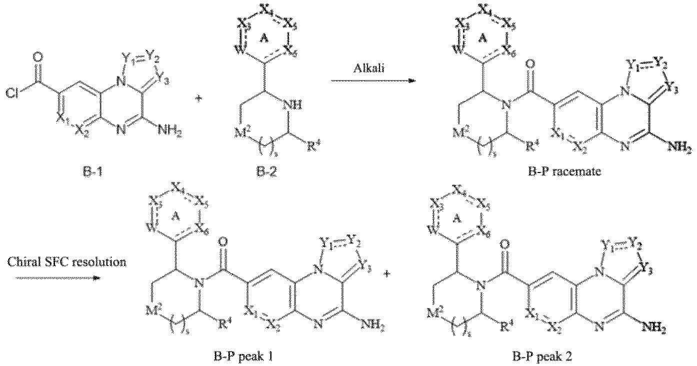

### Method C:

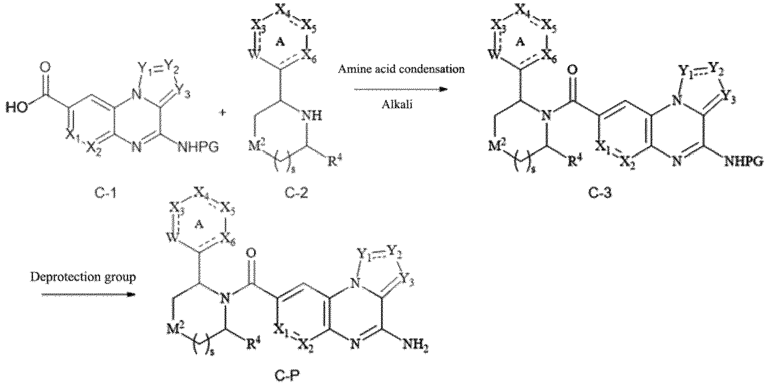

### Method C-SFC:

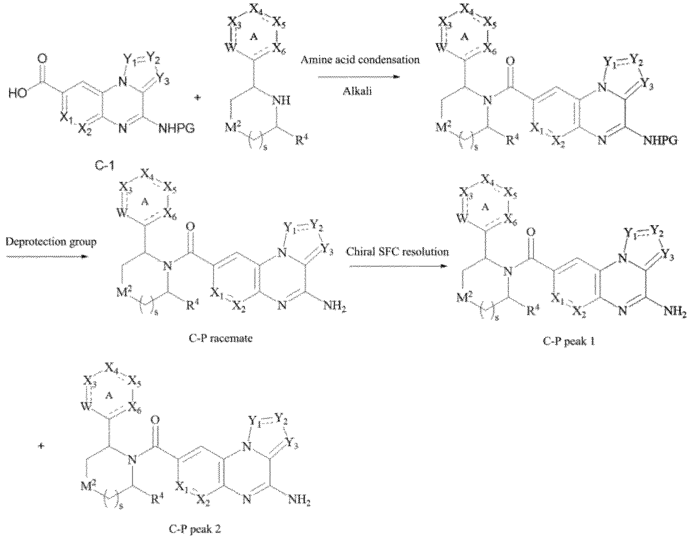

wherein R⁴, ring A, W, M², s, X₁, X₂, X₃, X₄, X₅, X₆, Y₁, Y₂ and Y₃ are as defined in claim 1, and PG is a protecting group.

Method A: Compounds A-P can be obtained by the amino acid condensation reaction of carboxylic acid A-1 and amine A-2. The condensing agent may be HATU or PyBrOP, the alkali may be DIPEA or TEA, and the solvent may be DMF or DMAc. If the amine used is a racemate, it will be resolved by chiral SFC and the stereochemistry of the obtained isomer will be assigned arbitrarily to R or S.

Method B: Compound B-P may be obtained by the reaction of acid chloride B-1 and amine B-2. The alkali used may be Et3N, DIPEA or pyridine, the solvent used may be THF or 1,4-dioxane or DCM or DCE, and some catalysts such as DMPK may also be added to promote the reaction. Similarly, if the amine used is a racemate, then the product will be resolved by chiral SFC and the stereochemistry of the obtained isomer will be assigned arbitrarily to R or S.

Method C: Compound C-P may be obtained by an amine acid condensation reaction of carboxylic acid C-1 and amine C-2 and a one-step deprotection reaction, wherein the condensing agent used in the condensation reaction may be HATU or PyBrOP, the alkali used may be DIPEA or TEA, and the solvent used may be DMF or DMAc; The selected protecting group is PMB or Boc, the reagent for removing PMB protecting group used is TFA or 5% methanesulfonic acid+TFA, and the reagent for removing Boc protecting group used is TFA or HCl/EA, HCl/1,4-dioxane or TMSOTf + 2,6-lutidine. Similarly, if the amine used is a racemate, then the product will be resolved by chiral SFC and the stereochemistry of the resulting isomer will be assigned arbitrarily to R or S.

### Analytical HPLC

Equipment: Agilent 1260; column specification: Agilent Poroshell HPH-C18 (3.0×50 mm, 2.7 µm); binary solvent system, mobile phase A: water (0.1% v/v ammonium bicarbonate), mobile phase B: acetonitrile; flow rate: 1 mL/min; gradient: from 10% B to 90% B; duration: 12 minutes; detector: DAD detector; wavelength: 254/220 nm;

### Preparation of HPLC-MS

HPLC equipment: Waters 2489; column specification: Ultimate µ XB-C18(130A, 5 µm, 30 mm×150 mm); binary solvent system, mobile phase A: water (0.1% v/v ammonium bicarbonate), mobile phase B: acetonitrile; flow rate: 60-100 mL/min; gradient: from 10 % B to 90 % B; detector: DAD detector; wavelength: 254/220 nm;
Mass spectrometer: Agilent G6125B.

The compound of the present invention may be prepared by chemical synthesis, examples of which are shown below. It will be understood that the sequence of steps in the process described may vary, those specifically mentioned reagents, solvents and reaction conditions may be substituted, and readily reactable sites may be protected and deprotected if necessary.

The following abbreviations have the meanings shown below. ACN refers to acetonitrile; EA refers to ethyl acetate; CDI refers to N, N'-carbonyldiimidazole; DBU refers to 1,8-diazabicyclo[5.4.0]undec-7-ene; DIBAL-H represents diisobutylaluminium hydride; DIEA refers to diisopropylethylamine; DMAP refers to N,N-dimethylaminopyridine; DME refers to 1,2-dimethoxyethane; DMF refers to N,N-dimethylformamide; DMA and DMAc refers to N,N-dimethylformamide; DMPE refers to 1,2-bis(dimethylphosphino)ethane; DMSO means dimethylsulfoxide; DPPB means 1,4-bis(diphenylphosphino)butane; DPPE refers to 1,2-bis(diphenylphosphino)ethane; DPPF means 1,1'-bis(diphenylphosphino)ferrocene; DPPM refers to 1,1'-bis(diphenylphosphino)methane; DIAD means diisopropyl azodicarboxylate; EDCI represents 1-(3-dimethylaminopropyl)-3-ethylcarbodiimide; HATU represents 2-(7-aza-1H-benzotriazol-1-yl)-1,1,3,3-tetramethylurea hexafluorophosphate; HMPA refers to hexamethylphosphoramide; IPA refers to isopropyl alcohol; LDA refers to lithium diisopropylamide; LHMDS refers to lithium di(trimethylsilyl)amide; LAH represents lithium aluminium hydride; NCS refers to N-chlorosuccinimide; NaHMDS refers to sodium di(trimethylsilyl)amide; PyBOP refers to benzotriazol-1-yl-oxytripyrrolidinophosphobenzotriazole hexafluorophosphate; PyBrOP refers to tripyrrolidylphosphonium bromide hexafluorophosphate; TDA-I refers to tris(2-(2-methoxyethoxy)ethyl)amine; DCM refers to dichloromethane; TEA refers to triethylamine and TFA means trifluoroacetic acid; THF refers to tetrahydrofuran; NCS refers to N-chlorosuccinimide; NMM means N-methylmorpholine; NMP refers to N-methylpyrrolidone; PPh₃ refers to triphenylphosphine; r.t. refers to room temperature; PMB means p-methoxybenzyl; Tosmic means p-Toluenesulfonylmethyl isonitrile; (Boc)₂O refers to di-tert-butyl dicarbonate; PE refers to petroleum ether; o/n refers to overnight reaction.

The following preparations and examples illustrate the present invention, but do not limit the present invention in any way.

The features and advantages of the subject matter of the present invention will become more apparent from a detailed description of selected examples. As will be appreciated, the disclosed and claimed subject matter can be modified in various aspects, and all the modifications will not depart from the scope of the claims. Therefore, the description should be treated as illustrative in nature rather than restrictive. The entire scope of the subject matter of the present invention is set forth in the claims.

The present invention may be more easily understood with reference to the following examples, which are only used for illustrating the present invention instead of limiting the scope of the present invention.

### Intermediates

### Intermediate 1:

### 4-{[(4-methoxylphenyl)methyl]amino}-10-hydroimidazo[1,5-a]quinoxalin-8-carboxylic acid

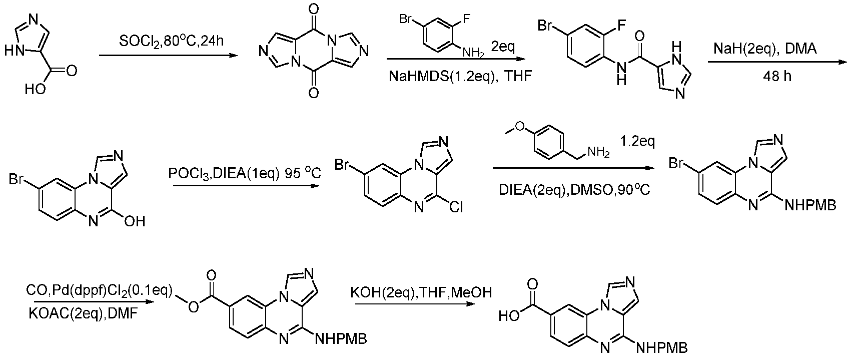

### Step I: Synthesis of imidazo[1,5-a]imidazo[1,5-d]1,4-diazapiperazine-5,10-dione

Imidazole-5-carboxylic acid (100 g, 892.14 mmol) was added to thionyl chloride (500 mL), and the mixture was stirred at 80 °C for 12 h and concentrated under vacuum to give imidazo[1,5-*a*]imidazo[1,5-*d*]1,4-diazapiperazine-5,10-dione (70 g, 0.37 mol, yield: 42%), which was a yellow solid.
LCMS (ESI) m/z: 189[M+H]⁺

### Step II: Synthesis of N-(4-bromo-2-fluorophenyl)imidazole-5-ylformamide

Sodium bistrimethylsilylamide (318.91 mL, 637.82 mmol, 2 mol/L) was slowly added dropwise to a tetrahydrofuran (600 mL) mixture of 4-bromo-2-fluorophenylamine (60.60 g, 318.91 mmol) at 0°C within 1 h, then imidazo[1,5-*a*]imidazo[1,5-*d*]1,4-diazapiperazine-5, 10-diketone (60 g, 318.91 mmol) was added, the mixture was stirred at room temperature for 12 h, then poured into water, filtered and the crude product was purified by silica gel column chromatography (petroleum ether: ethyl acetate=40%) to give *N*-(4-bromo-2-fluorophenyl)imidazole-5-ylformamide (60 g, 0.21 mol, yield: 66%), which was a white solid.
LCMS (ESI) m/z: 284[M+H]⁺

### Step III: Synthesis of 8-bromo-10-hydroimidazo [1,5-a]quinoxalin-4-ol

*N*-(4-bromo-2-fluorophenyl)imidazole-5-ylformamide (60 g, 211.20 mmol) and sodium hydride (16.88 g, 422.40 mmol, 60% content) were added in dimethylacetamide (600 mL) then the mixture was stirred at 140°C for 12 h, the mixture was poured into water, filtered to give 8-bromo-10-hydroimidazo[1,5-*a*]quinoxalin-4-ol (50 g, yield: 90%), which was a yellow solid.
LCMS (ESI) m/z: **264**[M+H]⁺

### Step IV: Synthesis of 8-bromo-4-chloro-10-hydroimidazo[1,5-a]quinoxaline

Phosphorus oxychloride (500 ml) was added to a mixture of 8-bromo-10-hydroimidazo[1,5-*a*]quinoxalin-4-ol (50 g, 189.34 mmol) and *N,N*-diisopropylethylamine (48.85 g, 378.67 mmol). The reaction system was stirred at 90°C for 2 h, and then concentrated, the residue was dissolved with acetonitrile, slowly dropped into ice water, a solid was precipitated, and the solid was filtered to give 8-bromo-4-chloro-10-hydroimidazo[1,5-*a*]quinoxaline (50 g, yield: 93%).
LCMS (ESI) m/z: **282**[M+H]⁺.

### Step V: Synthesis of (8-bromo(10-hydroimidazo[1,5-a]quinoxalin-4-yl)) [(4-methoxyphenyl)methyl]amine

8-bromo-4-chloro-10-hydroimidazo[1,5-*a*]quinoxaline (50 g, 176.98 mmol) and 4-methoxybenzylamine (29.13 g, 212.38 mmol) were added to dimethylsulfoxide (500 mL), then *N,N*-diisopropylethylamine (45.66 g, 353.96 mmol) was added, and the mixture was stirred at 80°C for 2 h, Then, the mixture was poured into water and filtered to give yellow oily (8-bromo(10-hydroimidazo[1,5-*a*]quinoxalin-4-yl))[(4-methoxylphenyl)methyl]amine (50 g, yield: 74%).
LCMS (ESI) m/z: **384**[M+H]⁺

### Step VI: Synthesis of methyl 4-{[(4-methoxylphenyl)methyl]amino}-10-hydroimidazo [1,5-a]quinoxalin-8-carboxylate

(8-bromo(10-hydroimidazo[1,5-*a*]quinoxalin-4-yl))[(4-methoxylphenyl)methyl]amine (50 g, 130.47 mmol), [1,1'-bis(diphenylphosphine)ferrocene]palladium dichloride (10.83 g, 13.05 mmol), and potassium acetate (25.57 g, 260.93 mmol) were added to a solution of dimethylformamide (50 mL) and methanol (250 mL). The system was stirred at 100°C for 12 h under an atmosphere of carbon monoxide (4 MPa), then poured into water and filtered, and purified by silica gel column chromatography (petroleum ether: ethyl acetate=80%) to give methyl 4-{[(4-methoxylphenyl)methyl]amino}-10-hydroimidazo[1,5-*a*]quinoxalin-8-carboxylate (30 g, yield: 63%), which was a yellow solid.
LCMS (ESI) m/z: 363 [M+H]⁺

### Step VII: Synthesis of methyl 4-{[(4-methoxylphenyl)methyl]amino}-10-hydroimidazo [1,5-a]quinoxalin-8-carboxylic acid

Methyl 4-{[(4-methoxyphenyl)methyl]amino}-10-hydroimidazo [1,5-*a*]quinoxalin-8-carboxylate (30 g, 82.87 mmol) was added to a mixed solution of methanol, water and tetrahydrofuran (1: 1: 1, 150 mL), potassium hydroxide (92.40 g, 165.0 mmol) was added to the mixed solution, the mixed solution was stirred at 60°C for 12 h and concentrated in vacuum to remove the organic solvent, and then poured into water, extracted with ethyl acetate (100 mL × 3) and concentrated in vacuum to give 4-{[(4-methoxylphenyl)methyl]amino}-10-hydroimidazo[1,5-*a*]quinoxalin-8-carboxylic acid (25 g, yield: 86%).
LCMS (ESI) m/z: 349 [M+H]⁺

The intermediate carboxylic acids in the following table may be prepared using the synthesis step described for **Intermediate** 1, only replacing the corresponding starting materials:

| Intermediate No. | Chemical Structure | Chemical Name | m/z(ESI): (M+H)⁺ |
|---|---|---|---|
| 2 | 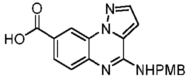 | 4-((4-methoxybenzyl)amino)imi dazo[1,2-*a*]quinoxalin-8-carbox ylic acid | 349.12 |
| 3 | 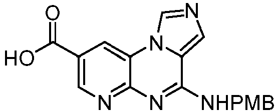 | 4-((4-methoxybenzyl)amino)imi dazo[1,5-*a*]pyrido[2,3-*e*] pyrazin-8-carboxylic acid | 350.2 |
| 4 | 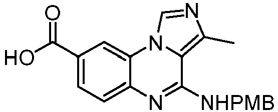 | 4-((4-methoxybenzyl)amino)-3-methylimidazo[1,5-*a*]quinoxalin -8-carboxylic acid | 363.1 |
| 5 | 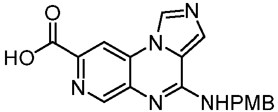 | 4-((4-methoxybenzyl)amino)imi dazo[1,5-*a*]pyrido[3,4-*e*]pyrazin -8-carboxylic acid | 350.1 |
| 6 | 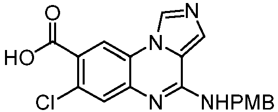 | 7-Chloro-4-((4-methoxybenzyl) amino)imidazo[1,5-*a*]quinoxalin -8-carboxylic acid | 383.1 |
| 7 | 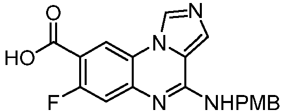 | 7-fluoro-4-((4-methoxybenzyl)a mino)imidazo[1,5-*a*]quinoxalin-8-carboxylic acid | 367.1 |
| 8 | 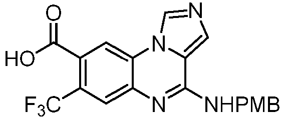 | 4-((4-methoxybenzyl)amino)-7-t rifluoromethylimidazo[1,5-*a*]qui noxalin-8-carboxylic acid | 417.1 |

### Intermediate 9: 4-((4-methoxylbenzyl)amino)pyrrolo[1,2-a]quinoxalin-8-carboxylic acid

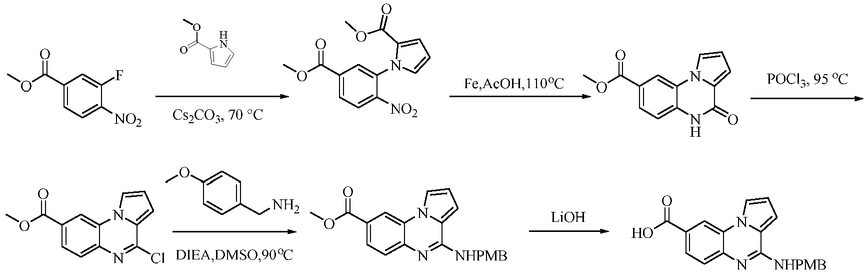

### Step I: Synthesis of methyl 1-(5-(methoxycarbonyl)-2-nitrophenyl)-1H-pyrrol-2-carboxylate

Methyl 3-amino-4-nitromethyl benzoate (5 g, 25.12 mmol) and 1H-pyrrol-2-carboxylate (4.711 g, 37.68 mmol) were first added to a 250 mL round bottom flask at room temperature. Then, *N,N*-dimethylformamide (100 mL) was poured into the flask and cesium carbonate (24.5 g, 75.37 mmol) was added. After the resulting mixture was stirred at 70°C for 6 h, the reaction solution was filtered and the filtrate was concentrated in vacuum and purified by silica gel column chromatography (petroleum ether: ethyl acetate=2:1) to give a yellow solid methyl 1-(5-(methoxylcarbonyl)-2-nitrophenyl)-1*H*-pyrrol-2-carboxylate (3.0 g, yield: 39.2%).
LCMS (ESI) m/z: 305.2[M+H]⁺

### Step II: Synthesis of methyl 4-oxo-4,5-dihydropyrrolo[1,2-a]quinoxalin-8-carboxylate

At room temperature, methyl 1-(5-(methoxycarbonyl)-2-nitrophenyl)-1*H*-pyrrol-2-carboxylate (3.0 g, 22.94 mmol) was dissolved in acetic acid (60 mL), then iron powder (7.7 g, 137.64 mmol) was successively added to the system, and the resulting reaction system was stirred at 110°C for 4 h. After the reaction was completed, the reaction solution was poured into water (100 mL) and filtered, the resulting filter cake was washed with water and dried to give a gray solid methyl **4-oxo-4,5-dihydropyrrolo[1,2-*a*]quinoxalin-8-carboxylate** (1.8 g, yield: 32.4%).
LCMS (ESI) m/z: 243.1[M+H]⁺

### Step III: Synthesis of methyl 4-chloropyrrolo[1,2-a] quinoxalin-8-carboxylate

Methyl **4-oxo-4,5-dihydropyrrolo[1,2-*a*]quinoxalin-8-carboxylate** (1.8 g, 7.43 mmol) was dissolved in phosphorus oxychloride (20 mL) at room temperature, then the mixture was heated to 90°C and reacted for 3 h while maintaining this temperature. After the reaction was completed, the system was poured into water (50 mL) and filtered, and the resulting filter cake was washed with water and dried to give a brown solid methyl 4-chloropyrrolo[1,2-*a*]quinoxalin-8-carboxylate (1.3 g, yield: 67.2%).
LCMS (ESI) m/z: 261.2[M+H]⁺

### Step IV: Synthesis of methyl 4-((4-methoxybenzyl)amino)pyrrolo[1,2-a]quinoxalin-8-carboxylate

At room temperature, methyl 4-chloropyrrolo[1,2-*a*]quinoxalin-8-carboxylate (1.3 g, 5.000 mmol) was dissolved in DMSO (25 mL), and then 4-methoxylbenzylamine (1.027 g, 7.500 mmol) and DIEA (1.935 g, 15.00 mmol) were successively added to the reaction system. After the resulting mixture was stirred at 90°C for 8 h, the reaction was completed. The reaction solution was poured into water (50 mL) and extracted with ethyl acetate (50 mL×3), the organic phases were combined, dried over anhydrous sodium sulfate and filtered, and the filtrate was concentrated under reduced pressure to obtain a crude product. The crude product was purified by silica gel column chromatography (the chromatography of petroleum ether: ethyl acetate=1:1) to give a yellow solid methyl 4-((4-methoxybenzyl)amino)imidazo[1,5-*a*]quinoxalin-8-carboxylate (1.4 g, yield: 77.7%).
LCMS (ESI) m/z: 361.1 [M+H]⁺

### Step V: Synthesis of 4-((4-methoxybenzyl)amino)pyrrolo[1,2-a]quinoxalin-8-carboxylic acid

At room temperature, methyl 4-((4-methoxybenzyl)amino)pyrrolo[1,2-*a*]quinoxalin-8-carboxylate (130 mg, 0.3601 mmol) was dissolved in a mixed system of tetrahydrofuran, methanol and water (4 mL, v/v: 2: 2:1), then lithium hydroxide monohydrate (25.9 mg, 1.080 mmol) was added, and the resulting mixture was reacted at 25°C for 16 h to complete the reaction. The pH of the reaction solution was adjusted to 6 with 4M hydrochloric acid and then concentrated under reduced pressure to give the crude product 4-((4-methoxylbenzyl)amino)pyrrolo[1,2-*a*]quinoxalin-8-carboxylic acid (100 mg, crude product), which was used directly in the next step without purification.
LCMS (ESI) m/z: 348.1[M+H]⁺

The intermediate carboxylic acids in the following table may be prepared using the synthesis step described for **Intermediate 9,** only replacing the corresponding starting materials:

| Intermediate No. | Chemical Structure | Chemical Name | m/z(ESI): (M+H)⁺ |
|---|---|---|---|
| 10 | 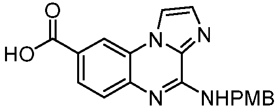 | 4-((4-methoxybenzyl)amino) imidazo[1,2-*a*]quinoxalin-8-carboxylic acid | 349.1 |
| 11 | 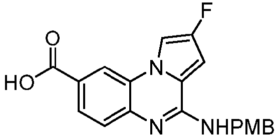 | 2-fluoro-4-((4-methoxybenz yl)amino)pyrrolo[1,2-*a*]quin oxalin-8-carboxylic acid | 366.1 |
| 12 | 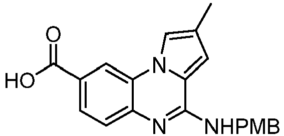 | 4-((4-methoxybenzyl)amino) -2-methylpyrrolo[1,2-*a*]quin oxalin-8-carboxylic acid | 362.1 |
| 13 | 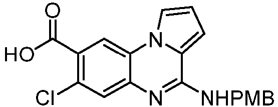 | 7-chloro-4-((4-methoxybenz yl)amino)pyrrolo[1,2-*a*]quin oxalin-8-carboxylic acid | 382.1 |
| 14 | 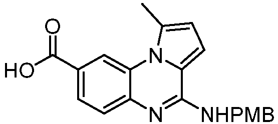 | 4-((4-methoxybenzyl)amino) -1-methylpyrrolo[1,2-*a*]quin oxalin-8-carboxylic acid | 362.1 |
| 15 | 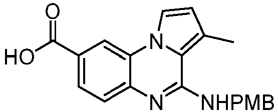 | 4-((4-methoxybenzyl)amino) -3-methylpyrrolo[1,2-*a*]quin oxalin-8-carboxylic acid | 362.1 |

### Intermediate 16: 4-((4-methoxylbenzyl)amino)-1-methylimidazo[1,5-a]quinoxalin-8-carboxylic acid

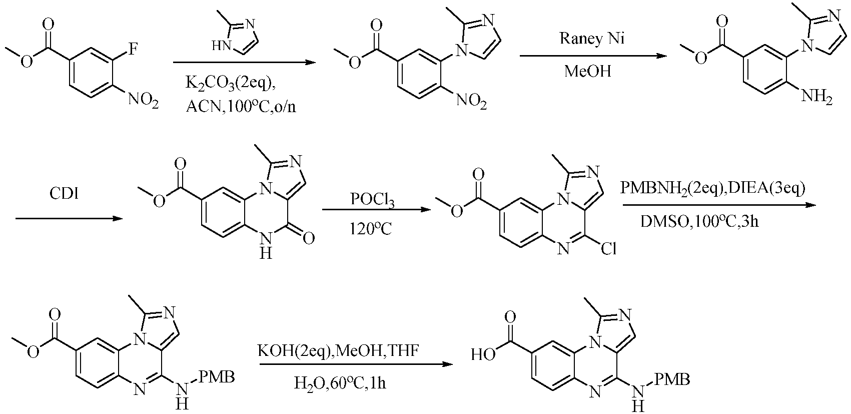

### Step I: Synthesis of 3-(2-methyl-1H-imidazol-1-yl)-4-nitromethyl benzoate

3-fluoro-4-nitromethyl benzoate (20.0 g, 100 mmol) was dissolved in acetonitrile (100 mL), and then 2-methyl-1*H*-imidazole (8.2 g, 100 mmol) and potassium carbonate (27.6 g, 200 mmol) were added. The mixture was stirred at 100°C for 12 h. The reaction was monitored by the LCMS. The reaction solution was poured into water (300 mL), extracted with ethyl acetate (200 mL×3), and the organic phases were combined, dried over anhydrous sodium sulfate, concentrated and purified by silica gel column chromatography on(petroleum ether:ethyl acetate=10%) to give a yellow solid 3-(2-methyl-1*H*-imidazol-1-yl)-4-nitromethyl benzoate (25.0 g, yield: 95%).
LCMS (ESI) m/z: 262 [M+H]⁺

### Step II: Synthesis of methyl4-amino-3-(2-methyl-1H-imidazol-1-yl)benzoate

At room temperature, 3-(2-methyl-1*H*-imidazol-1-yl)-4-nitromethyl benzoate (25 g, 95.8 mmol) was dissolved in methanol (300 mL), and then Raney nickel (2 g) was added. The mixture was stirred in a hydrogen atmosphere at room temperature for 12 h. The reaction was monitored by the LCMS. The residue was filtered to give a filtrate, and the filtrate was concentrated and purified by silica gel column chromatography (petroleum ether:ethyl acetate=20%) to give a yellow solid methyl 4-amino-3-(2-methyl-1*H*-imidazol-1-yl)benzoate (20.5 g, 93%).
LCMS (ESI) m/z: 232 [M+H]⁺

### Step III: Synthesis of methyl 1-methyl-4-oxo-4,5-dihydroimidazo[1,5-a] quinoxalin-8- carboxylate

At room temperature, methyl 4-amino-3-(2-methyl-1*H*-imidazol-1-yl)benzoate (2.0 g, 8.7 mmol) was dissolved in o-dichlorobenzene (40 mL), and then carbonyldiimidazole (2.8 g, 17.4 mmol) was added. The mixture was stirred at 180°C for 12 h. The reaction was monitored by LC-MS. The mixture was filtered to give a filter cake, the filter cake was slurried with ethyl acetate (5 mL) to give a black solid methyl 1-methyl-4-oxo-4,5-dihydroimidazo[1,5-*a*]quinoxalin-8-carboxylate (920.0 mg, yield: 41%).
LCMS (ESI) m/z: 258 [M+H]⁺

### Step IV: Synthesis of methyl 4-chloro-1-methylimidazo[1,5-a]quinoxalin-8-carboxylate

Methyl 1-methyl-4-oxo-4,5-dihydroimidazo[1,5-*a*]quinoxalin-8-carboxylate (100.0 mg, 0.39 mmol) was dissolved in phosphorus oxychloride (5 mL) at room temperature and the mixture was stirred at 120°C for 4 h. The reaction was monitored by LC-MS, the reaction solution was concentrated under reduced pressure, diluted with a small amount of acetonitrile, poured into water (5 mL), and filtered, the filter cake was washed with water, and vacuum dried to give methyl **4-chloro-1-methylimidazo[1,5-*a*]quinoxalin-8-carboxylate** (60.0 mg, yield: 56%), which was a black solid.
LCMS (ESI) m/z: 276 [M+H]⁺

### Step V: Synthesis of methyl 4-((4-methoxybenzyl)amino)-1-methylimidazo[1,5-a]quinoxalin-8-carboxyla te

At room temperature, methyl **4-chloro-1-methylimidazo[1,5-*a*]quinoxalin-8-carboxylate** (60 mg, 0.22 mmol) was dissolved in dimethylsulfoxide (2 mL), and then (4-methoxyphenyl)methanamine (60 mg, 0.44 mmol) and N,N-diisopropylethylamine (67 mg, 0.52 mmol) were added. The mixture was stirred at 100°C for 1 h. The reaction was monitored by LC-MS. The reaction solution was poured into (10 mL) water and extracted with ethyl acetate (10 mL×3), the organic phases were combined, dried over anhydrous sodium sulfate, concentrated and purified by silica gel column chromatography (petroleum ether: ethyl acetate=40%) to give a black solid methyl 4-((4-methoxybenzyl)amino)-1-methylimidazo[1,5-*a*]quinoxalin-8-carboxylate (60 mg, yield: 73%).
LCMS (ESI) m/z: 377 [M+H]⁺

### Step VI: Synthesis of 4-((4-methoxybenzyl)amino)-1-methylimidazo[1,5-a] quinoxalin-8-carboxylic acid

Methyl 4-((4-methoxybenzyl)amino)-1-methylimidazo[1,5-*a*]quinoxalin-8-carboxylate (60 mg, 0.16 mmol) was dissolved in a mixed solvent of methanol (1 mL), tetrahydrofuran (1 mL) and water (1 mL) at room temperature, and then potassium hydroxide (26 mg, 0.44 mmol) was added. The mixture was stirred at room temperature for 1 h. The reaction was monitored by LC-MS. The reaction solution was poured into (10 mL) water, formic acid was added to adjust the reaction solution to be acidic, 10 mL of ethyl acetate was added to extract the reaction solution three times, the organic phases were combined, dried over anhydrous sodium sulfate and concentrated to give a white solid 4-((4-methoxylbenzyl)amino)-1-methylimidazo[1,5-*a*]quinoxalin-8-carboxylic acid (50 mg, yield: 86%).
LCMS (ESI) m/z: 363 [M+H]⁺

The intermediate carboxylic acids in the following table may be prepared using the synthesis step described for **Intermediate 16,** only replacing the corresponding starting materials:

| Intermediate No. | Chemical Structure | Chemical Name | m/z(ESI): (M+H)⁺ |
|---|---|---|---|
| 17 | 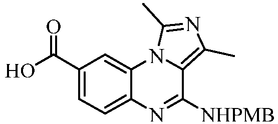 | 4-((4-methoxybenzyl)ami no)-1,3-dimethylimidazo[ 1,5-*a*]quinoxalin-8-carbo xylic acid | 377.1 |

### Intermediate 18: 4-amino-7-cyanoimidazo[1,5-a]quinoxalin-8-carboxylic acid

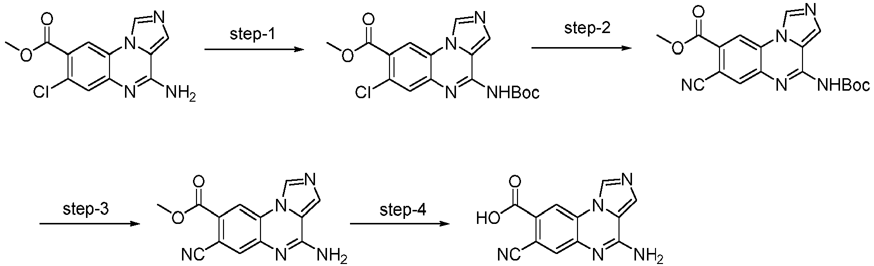

### Step I: Synthesis of methyl 4-((tert-butoxycarbonyl)amino)-7-chloroimidazo[1,5-a]quinoxalin-8-carboxylate

A solution of methyl 4-amino-7-chloroimidazo[1,5-*a*]quinoxalin-8-carboxylate (1.20 g, 4.34 mmol), di-tert-butyl dicarbonate (1.89 g, 8.67 mmol), triethylamine (1.30 g, 13.01 mmol) in methylene chloride (10 mL) was stirred at room temperature for 2 h and after the reaction was completed, the reaction solution was poured into water (5 mL) and extracted with ethyl acetate (5 mL), the organic layer was dried over anhydrous sodium sulfate and filtered, then concentrated and purified by column chromatography (petroleum ether: ethyl acetate=70:30) to give methyl 4-((tert-butoxycarbonyl)amino)-7-chloroimidazo[1,5-a]quinoxalin-8-carboxylate (1.00 g, yield: 61%), which was a white solid.
LCMS (ESI): 376 [M+H]⁺

### Step II: Synthesis of methyl 4-((tert-butoxycarbonyl)amino)-7-cyanoimidazo[1,5-a]quinoxalin-8-carboxylate

A mixture of methyl 4-((tert-butoxycarbonyl)amino)-7-chloroimidazo[1,5-*a*]quinoxalin-8-carboxylate)carbon ylamino]-7-chloro-10-hydroimidazo[1,5-*a*]quinoxalin-8-carboxylate (0.20 g, 0.53 mmol), potassium ferricyanide (0.05 g, 0.16 mmol), 2-di-tert-butylphosphono-2',4',6'-triisopropylbiphenyl (0.09 g, 0.21 mmol), methanesulfonic acid (2-di-tert-butylphosphino-2',4',6'-triisopropyl-1,1'-biphenyl)(2'-amino-1,1'-biphenyl-2-yl) palladium(II) (0.17 g, 0.21 mmol), and potassium acetate (0.01 g, 0.07 mmol) in dioxane/water (4.00 mL) were stirred at 100°C for 2 h, and then the mixture was concentrated and purified by column chromatography (petroleum ether: ethyl acetate=70:30) to give a white solid methyl 4-((tert-butoxycarbonyl)amino)-7-cyanoimidazo[1,5-*a*]quinoxalin-8-carboxylate (0.10 g, 51%).
LCMS (ESI): 368[M+H]⁺

### Step III: Synthesis of methyl 4-amino-7-cyanoimidazo[1,5-a]quinoxalin-8-carboxylate

Trifluoroacetic acid (0.10 mL) was added to a solution of methyl 4-((tert-butoxycarbonyl)amino)-7-cyanoimidazo[1,5-*a*]quinoxalin-8-carboxylate (0.20 g, 0.54 mmol) in dichloromethane (0.50 mL), and the reaction solution was stirred at room temperature for 2 h and then concentrated to give methyl 4-amino-7-cyanoimidazo[1,5-*a*]quinoxalin-8-carboxylate (0.05 g, crude product).
LCMS (ESI): 268[M+H]⁺

### Step IV: Synthesis of 4-amino-7-cyanoimidazo[1,5-a] quinoxalin-8-carboxylic acid

A mixture of methyl 4-amino-7-cyanoimidazo[1,5-*a*]quinoxalin-8-carboxylate (0.20 g, 0.75 mmol), potassium hydroxide (0.07 g, 1.50 mmol), and tetrahydrofuran/methanol/water (1 mL/1 mL/1 mL) was stirred at room temperature for 2 h. After the reaction was completed, the system was concentrated to give a crude product 4-amino-7-cyanoimidazo[1, 5-*a*]quinoxalin-8-carboxylic acid crude (210 mg, crude product). The crude product was directly used in the next step without purification.
LCMS (ESI): 254[M+H]⁺

### Intermediate 19: 4-amino-7-methylimidazo[1,5-a]quinoxalin-8-carboxylic acid

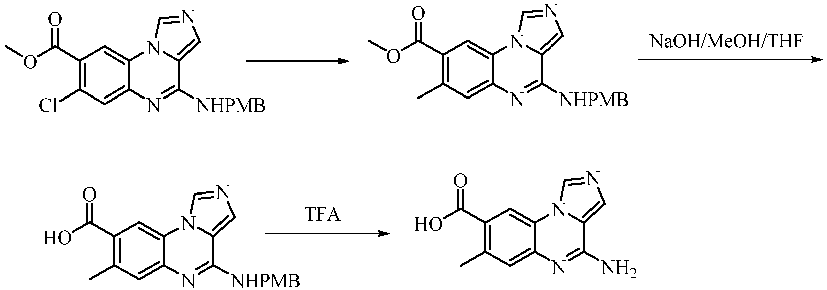

### Step I: Synthesis of methyl 4-{[(4-methoxyphenyl)methyl]amino}-7-methyl-10-hydroimidazo [1,5-a]quinoxalin-8-carboxylate

A mixture of methyl 7-chloro-4-{[(4-methoxylphenyl)methyl]amino}-10-hydroimidazo[1,5-*a*]quinoxalin-8-c arboxylate (200 mg, 0.5 mmol), potassium carbonate (300 mg, 2 mmol), dichloro[1,1'-bis(tert-butylphosphine)ferrocene palladium (II) (0.08 g, 0.1 mmol), and trimethylboronoxane (0.01 g, 0.10 mmol) was stirred in dioxane (2.00 mL) at 100°C for 12 h, then the mixture was poured into water, extracted with ethyl acetate (5 mL), dried over anhydrous sodium sulfate, filtered and purified by column chromatography (petroleum ether: ethyl acetate=30%) to give methyl 4-{[(4-methoxyphenyl)methyl]amino}-7-methyl-10-hydroimidazo [1,5-*a*]quinoxalin-8-carboxylate (100 mg, yield: 53%).
LCMS (ESI): 268 [M+H] ⁺

### Step II: Synthesis of 4-((4-methoxybenzyl)amino)-7-methylimidazo[1,5-a] quinoxalin-8-carboxylic acid

Methyl 4-{[(4-methoxyphenyl)methyl]amino}-7-methyl-10-hydroimidazo[1,5-*a*]quinoxalin-8-carboxylate (500 mg, 1.86 mmol) was dissolved in a mixed solution of methanol: tetrahydrofuran: potassium hydroxide (1 mL: 1 mL: 1 mL), the reaction solution was reacted at 60°C for 12 h and then concentrated in vacuum, the pH was adjusted to 3 with formic acid, and then the reaction solution was filtered to give 4-((4-methoxylbenzyl)amino)-7-methylimidazo[1,5-*a*]quinoxalin-8-carboxylic acid (52 mg, yield: 12%).
LCMS (ESI):363 [M+H]⁺

### Step III: Synthesis of 4-amino-7-methylimidazo [1,5-a]quinoxalin-8-carboxylic acid

4-((4-methoxylbenzyl)amino)-7-methylimidazo[1,5-*a*]quinoxalin-8-carboxylic acid (500 mg, 1.86 mmol) was dissolved in a trifluoroacetic acid solution (5 mL), and the reaction mixture was stirred at 100°C for 12 h to give 4-amino-7-methylimidazo[1,5-*a*]quinoxalin-8-carboxylic acid (52 mg, 0.23 mmol).
LCMS (ESI):363 [M+H]⁺

Using the method used for the **Intermediate** 19 in step 3, the following intermediate acid was prepared:

| Intermediate No. | Chemical Structure | Chemical Name | m/z(ESI): (M+H)⁺ |
|---|---|---|---|
| 20 | 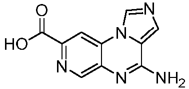 | 4-aminoimidazo[1,5-*a*]pyrido[3, 4-*e*] pyrazin-8-carboxylic acid | 230.1 |
| 21 | 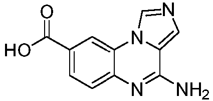 | 4-amino-10-hydroimidazo[1,5-*a* ]quinoxalin-8-carboxylic acid | 229.1 |
| 22 | 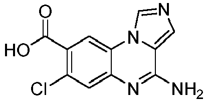 | 4-amino-10-hydroimidazo[1,5-*a* ]quinoxalin-8-carboxylic acid | 263.1 |
| 23 | 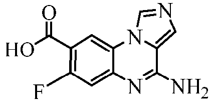 | 4-amino-7-fluimidazo[1,5-*a*]qui noxalin-8-carboxylic acid | 247.1 |
| 24 | 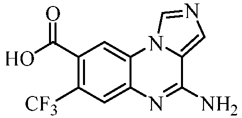 | 4-amino-7-(trifluoromethyl)imid azo[1,5-*a*]quinoxalin-8-carboxyl ic acid | 297.1 |
| 25 | 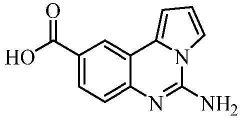 | 5-aminopyrrolo[1,2-*c*]quinoxali n-9-carboxylic acid | 228.1 |

### Example 1 Synthesis of (R)-(4-aminopyrrolo[1,2-a]quinoxalin-8-yl)(2-(4-fluorophenyl)piperidin-1-yl)me thanone

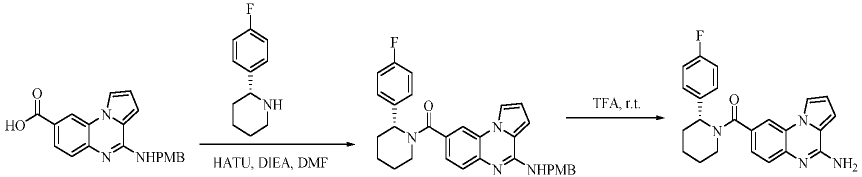

### Step I: Synthesis of (R)-(4-aminopyrrolo[1,2-a]quinoxalin-8-yl)(2-(4-fluorophenyl)piperidin-1-yl)methan one

4-((4-methoxylbenzyl)amino)pyrrolo[1,2-*a*]quinoxalin-8-carboxylic acid (40 mg, 0.12 mmol) and (*R*)-2-(4-fluorophenyl)piperidine (20 mg, 0.12 mmol) were dissolved in *N,N*-dimethylformamide (1 mL), and then 2-(7-azabenzotriazole)-*N,N,N',N*'-tetramethylurea hexafluorophosphate (130 mg, 0.34 mmol) and *N,N*-diisopropylethylamine (44 mg, 0.34 mmol) were added. The mixed solution was stirred at room temperature for 2 h. After the reaction was completed, the reaction solution was poured into water (10 mL), extracted with ethyl acetate (10 mL×3) and the combined organic phases were washed with a saturated brine solution and dried over anhydrous sodium sulfate. The anhydrous sodium sulfate was removed by filtration and the filtrate is concentrated to give a crude product. The crude product was purified by silica gel column chromatography (dichloromethane:methanol=10:90) to give (*R*)-(4-aminopyrrolo[1,2-a]quinoxalin-8-yl)(2-(4-fluorophenyl)pi peridin-1-yl)methanone (20 mg, yield: 34.15%), which was a white solid.
LCMS (ESI) *m*/*z:* 509[M+H]⁺

### Step II: Synthesis of (R)-(4-aminopyrrolo[1,2-a]quinoxalin-8-yl)(2-(4-fluorophenyl)piperidin-1-yl)metha none

(R)-(4-aminopyrrolo[1,2-*a*]quinoxalin-8-yl)(2-(4-fluorophenyl)piperidin-1-yl)methanone (20 mg, 0.039 mmol) was dissolved in trifluoroacetic acid (1 mL), and stirred at 60 °C for 4 h. After the reaction was completed, the reaction solution was poured into a saturated aqueous sodium bicarbonate solution (30 mL), extracted with ethyl acetate (30 mL×3), with the organic phases combined, washed with a saturated brine solution (30 mL) and dried over anhydrous sodium sulfate. The anhydrous sodium sulfate was removed by filtration, the filtrate was concentrated and the resulting crude product was purified by a preparative liquid phase (column: XBridge Prep OBD C₁₈, 30 x 150 mm 5 µm, mobile phase A: 10 mmol/L aqueous ammonium bicarbonate solution, mobile phase B: acetonitrile, flow rate: 60 mL/min, elution gradient: from 15% to 60% of mobile phase B in 8 min, detection wavelength: 220 nm, retention time: 8.38 min) to give (*R*)-(4-aminopyrrolo[1,2-*a*]quinoxalin-8-yl)(2-(4-fluorophenyl)piperidin-1-yl)methanone (0.35 mg, yield: 2.29%), which was a white solid.

¹H NMR (400 MHz, DMSO-*d₆*): δ *ppm* 8.21 (br, 1H), 8.06 (s, 1H), 7.36-7.31 (m, 3H), 7.24 (d, *J =* 7.6 Hz, 1H), 7.19-7.14 (m, 2H), 7.00 (d, *J* = 4.8 Hz, 3H), 6.69-6.67 (m, 1H), 3.12-3.09 (m, 1H), 2.34 (br, 2H), 1.92-1.85 (m, 1H), 1.62-1.56 (m, 1H), 1.48 (br, 2H), 1.38-1.29 (m, 1H). LCMS (ESI) *m*/*z:* 388.85 [M+H]⁺

### Example 2

### Synthesis of 4-amino-N-(6-bromo-2,3-dihydrobenzofuran-3-yl)-N-methylimidazo[1,5-a]quinoxalin-8-for mamide

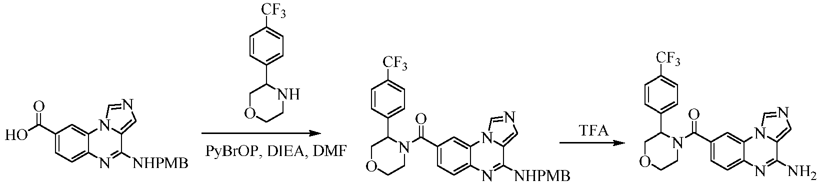

### Step I: Synthesis of (4-((4-methoxylbenzyl)amino)imidazo[1,5-a]quinoxalin-8-yl)(3-(4-(trifluoromethyl)p henyl)morpholino)methanone

At room temperature, 4-((4-methoxylbenzyl)amino)imidazo[1,5-*a*]quinoxalin-8-carboxylic acid (200 mg, 0.5741 mmol) was dissolved in *N,N*-dimethylacetamide (5 mL). 3-(4-(trifluoromethyl)phenyl)morpholine (159 mg, 0.6889 mmol), tripyrrolidinylphosphonium bromide hexafluorophosphate (541 mg, 1.148 mmol) and *N,N*-diisopropylethylamine (227 mg, 1.722 mmol) were added successively to the system. The resulting mixture was stirred at 50°C for 3 h, and after the reaction was completed, the reaction solution was poured into water (20 mL) and extracted with ethyl acetate (10 mL×3), the organic phases were combined, dried over anhydrous sodium sulfate, and filtered, the resulting filtrate was concentrated under reduced pressure to obtain a crude product, and the crude product was purified by silica gel column chromatography (the volume ratio of petroleum ether to ethyl acetate 1:1) to give a yellow solid (4-((4-methoxyjbenzyl)amino)imidazo[1,5-*a*]quinoxalin-8-yl)(3-(4-(trifluoromethyl)phenyl)morpholino)meth anone (100 mg, yield: 75%).
LCMS (ESI) m/z: 561.3[M+H]⁺

### Step II: Synthesis of 4-{[(4-methoxylphenyl)methyl]amino}(10-hydroimidazo [1,5-a]quinoxalin-8-yl) 3-[4-(trifluoromethyl)phenyl] morpholin-4-yl ketone

At room temperature, (4-((4-methoxylbenzyl)amino)imidazo[1,5-*a*]quinoxalin-8-yl)(3-(4-(trifluoromethyl)ph enyl)morpholino) methanone (200 mg, 0.3561 mmol) was dissolved in TFA (5 mL), and the resulting mixture was stirred at 50°C for 3 h. After the reaction was completed, the reaction solution was concentrated under reduced pressure and poured into water (10 mL). Then the pH was adjusted to 8 with a saturated sodium bicarbonate solution, the reaction solution was extracted with ethyl acetate (10 mL×3), with the organic phases combined, dried over anhydrous sodium sulfate, filtered and the resulting filtrate was concentrated under reduced pressure to give a crude product, and the crude product was purified by column chromatography (dichloromethane: methanol 20:1) to give a white solid 4-{[(4-methoxylphenyl)methyl]amino}(10-hydroimidazo [1,5-*a*]quinoxalin-8-yl) 3-[4-(trifluoromethyl) phenyl]morpholin-4-ylone (71.0 mg, yield: 45.2%).
LCMS (ESI) m/z: 441.3[M+H]⁺
¹H NMR (400 MHz, DMSO-*d*₆) δ 9.17 (s, 1H), 8.30 (d, *J =* 1.7 Hz, 1H), 7.91 (s, 1H), 7.78 (d, *J =* 7.9 Hz, 2H), 7.71 (s, 2H), 7.52 - 7.37 (m, 4H), 5.57 (s, 1H), 4.49 (d, *J* = 12.4 Hz, 1H), 3.94 (d, *J* = 12.3 Hz, 1H), 3.80 (s, 1H), 3.70 - 3.56 (m, 1H), 3.23 (s, 2H), 0.88 - 0.79 (m, 1H).

### Example 2A: (R)-4-amino-N-(6-bromo-2,3-dihydrobenzofuran-3-yl)-N-methylimidazo[1,5-a]quinoxalin-8-f ormamide and Example 2B: (S)-4-amino-N-(6-bromo-2,3-dihydrobenzofuran-3-yl)-N-methylimidazo[1,5-a] quinoxalin-8-formamide

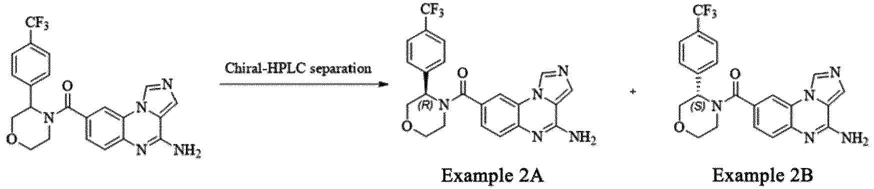

### Example 2A (R)-4-Amino-N-(6-bromo-2,3-dihydrobenzofuran-3-yl)-N-methylimidazo[1,5-a]quinoxalin-8-carboxam ide

Column: CHIRALPAK IF-3, 4.6×50 mm, 3 µm; Mobile Phase A: Hex (0.1% D ethyl acetate): (EtOH: DCM=1: 1)=65: 35; Flow rate: 1 mL/min; Injection Volume: 5 µl
¹H NMR (400 MHz, DMSO-*d₆)* δ 9.17 (s, 1H), 8.30 (d, *J =* 1.7 Hz, 1H), 7.91 (s, 1H), 7.78 (d, *J =* 8.0 Hz, 2H), 7.70 (s, 2H), 7.43 (d, *J* = 8.9 Hz, 4H), 4.49 (d, *J =* 12.2 Hz, 1H), 3.94 (d, *J* = 12.5 Hz, 1H), 3.80 (s, 1H), 3.62 (t*, J =* 11.3 Hz, 1H).
LCMS (ESI) m/z:**442.30**[M+H]⁺

### Example 2B

### (S)-4-amino-N-(6-bromo-2,3-dihydrobenzofuran-3-yl)-N-methylimidazo[1,5-a]quinoxalin-8-carboxami de

Column: CHIRALPAK IF-3, 4.6×50 mm, 3 µm; Mobile Phase A: Hex(0.1%D ethyl acetate): (EtOH: DCM=1: 1)=65: 35; Flow rate: 1 mL/min; Injection Volume: 5 µl
¹H NMR (400 MHz, DMSO-*d*₆) δ 9.17 (s, 1H), 8.29 (d, *J=* 1.8 Hz, 1H), 7.91 (s, 1H), 7.78 (d, *J =* 8.0 Hz, 2H), 7.70 (s, 2H), 7.43 (d, *J =* 9.3 Hz, 4H), 5.61 (s, 1H), 4.49 (d, *J =* 12.4 Hz, 1H), 3.94 (d, *J =* 12.1 Hz, 1H), 3.80 (s, 1H), 3.62 (t, *J =* 11.5 Hz, 1H).
LCMS (ESI) m/z:**442.35**[M+H]⁺

### Example 3:

### Synthesis of (4-((4-methoxybenzyl)amino)pyrrolo[1,2-a]quinoxalin-8-yl)(3-(4-(trifluoromethyl)phenyl) morpholino)methanone

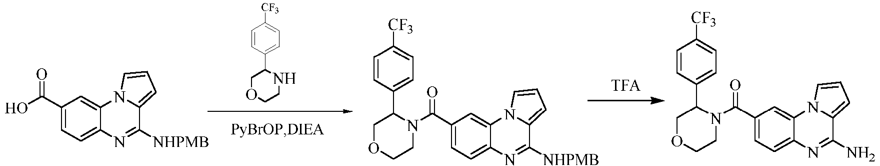

### Step I: Synthesis of N-cyclopropyl-4-((4-methoxylbenzyl)amino)-N-((5-(trifluoromethyl)pyridin-2-yl)me thyl)pyrrolo[1,2-a]quinoxalin-8-formamide

At room temperature, 4-((4-methoxylbenzyl)amino)pyrrolo[1,2-*a*]quinoxalin-8-carboxylic acid (100 mg, 0.288 mmol) was dissolved in N,N-dimethylacetamide (3 mL) first, and then 3-(4-(trifluoromethyl)phenyl)morpholine (95.04 mg, 0.432 mmol), tripyrrolidinylphosphonium bromide hexafluorophosphate (135.1 mg, 0.5762 mmol) and *N,N*-diisopropylethylamine (112.23 mg, 0.864 mmol) were added successively to the system. The resulting mixture was stirred at 50°C for 3 hours. After the reaction, the reaction solution was poured into water (15 mL) and extracted with ethyl acetate (10 mL×3), withthe organic phases combined, dried over anhydrous sodium sulfate and filtered. The resulting filtrate was concentrated under reduced pressure to give a crude product, and the crude product was purified by silica gel column chromatography (volume ratio of petroleum ether to ethyl acetate: 1:1) to give *N*-cyclopropyl-4-((4-methoxylbenzyl)amino)-*N*-((5-(trifluoromethyl)pyridin-2-yl)methyl)pyrrolo[1,2-*a*]quino xalin-8-formamide (90 mg, yield: 57%).
LCMS (ESI) m/z: **546.3** [M+H]⁺

### Step II: Synthesis of (4-aminopyrrolo[1,2-a]quinoxalin-8-yl)(3-(4-(trifluoromethyl)phenyl)morpholino)m ethanone

At room temperature, (4-((4-methoxybenzyl)amino)pyrrolo[1,2-*a*]quinoxalin-8-yl)(3-(4-(trifluoromethyl)phe nyl)morpholino) methanone (90 mg, 0.1830 mmol) was dissolved in TFA (2 mL). The resulting mixture was stirred at 50°C for 3 h. After the reaction was completed, the reaction solution was concentrated under reduced pressure and poured into water (10 mL). Then the pH was adjusted to 8 with a saturated sodium bicarbonate solution, the reaction solution was extracted with ethyl acetate (10 mL×3), with the organic phases combined, dried over anhydrous sodium sulfate and filtered, and the resulting filtrate was concentrated under reduced pressure to give a crude product. The crude product was purified by reversed-phase column chromatography (the volume ratio of water to acetonitrile: 5:95) to give a white solid (4-aminopyrrolo[1,2-*a*]quinoxalin-8-yl)(3-(4-(trifluoromethyl)phenyl) morpholino)methanone (10.3 mg, yield: 14.0%).

¹H NMR (400 MHz, DMSO-*d₆*): δ 8.28 (s, 1H), 8.14 (d, *J =* 1.8 Hz, 1H), 7.78 (d, *J* = 8.0 Hz, 2H), 7.71 (s, 2H), 7.42 (d, *J =* 8.2 Hz, 1H), 7.33 (dd, *J =* 8.3, 1.7 Hz, 1H), 7.12 (s, 2H), 7.08 (dd, *J =* 3.9, 1.3 Hz, 1H), 6.75 (dd, *J* = 3.9*,* 2.8 Hz, 1H), 5.57 (s, 1H), 4.49 (d, *J* = 12.3 Hz, 1H), 3.95 (dd, *J* = 12.3, 3.5 Hz, 1H), 3.86 - 3.74 (m, 1H), 3.63 (td, *J* = 11.5, 2.8 Hz, 1H), 3.22 (s, 2H).
LCMS (ESI) m/z: **441.4** [M+H]⁺

### Example 4:

### Synthesis of (4-amino-7-chloroimidazo[1,5-a]quinoxalin-8-yl)(3-(4-(trifluoromethyl)phenyl)morpholino) methanone

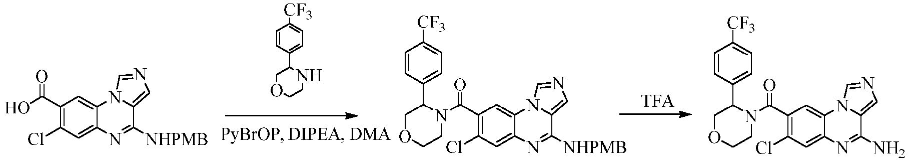

### Step I: Synthesis of (7-chloro-4-((4-methoxybenzyl)amino)imidazo[1,5-a]quinoxalin-8-yl)(3-(4-(trifluoro methyl)phenyl)morpholino)methanone

At room temperature, 7-chloro-4-((4-methoxylbenzyl)amino)imidazo[1,5-*a*]quinoxalin-8-carboxylic acid (200 mg, 0.5225 mmol) was dissolved in N,N-dimethylacetamide (5 mL), and then 3-(4-(trifluoromethyl)phenyl)morpholine (145 mg, 0.6270 mmol), tripyrrolidylphosphonium bromide hexafluorophosphate (369 mg, 0.7837 mmol) and *N,N*-diisopropylethylamine (207 mg, 1.567 mmol) were added successively to a flask. The mixture was stirred at 50°C for 3 h, after the reaction was completed, the reaction solution was poured into water (40 mL), extracted with ethyl acetate (20 mL×3), the organic phases were combined and dried over anhydrous sodium sulfate and filtered, the filtrate was concentrated under reduced pressure to give a crude product. The crude product was purified by silica gel column chromatography (DCM: MeOH=10:1) to give a yellow solid 7-chloro-*N*-cyclopropyl-4-((4-methoxybenzyl)amino)-*N*-((5-(trifluoromethyl)pyridin-2-yl)methyl)imidazo[1, 5-*a*]quinoxalin-8-formamide (160 mg, yield: 51.4%).
LCMS (ESI) m/z: 597 [M+H]⁺

### Step IX: Synthesis of

### (4-amino-7-chloroimidazo[1,5-a]quinoxalin-8-yl)(3-(4-(trifluoromethyl)phenyl)morpholino)methanone

At room temperature, (7-chloro-4-((4-methoxylbenzyl)amino)imidazo[1,5-*a*]quinoxalin-8-yl)(3-(4-(trifluoro methyl)phenyl)morpholino) methanone (160 mg, 0.252 mmol) was dissolved in TFA (5 mL), and the mixture was stirred at 50°C for 3 h. After the reaction was completed, the reaction solution was concentrated under reduced pressure, and then poured into water (10 mL). The pH was adjusted to 8 with a saturated sodium bicarbonate solution, the reaction solution was extracted with ethyl acetate (10 mL×3), with the organic phases combined, dried over anhydrous sodium sulfate and filtered, and the filtrate was concentrated under reduced pressure to give a crude product. The crude product was purified by column chromatography (DCM: MeOH=20:1) to give a white solid (4-amino-7-chloroimidazo[1,5-*a*]quinoxalin-8-yl)(3-(4-(trifluoromethyl)phenyl)morpholino)methanone (75 mg, yield: 62.6%).
LCMS (ESI) m/z: **476** [M+H]⁺
¹H NMR (400 MHz, DMSO-*d*₆) δ 9.24 - 9.15 (m, 1H), 8.40 - 8.22 (m, 1H), 7.95 (s, 1H), 7.82 (s, 3H), 7.71 (t, *J* = 8.4 Hz, 1H), 7.61 (s, 2H), 7.54 - 7.46 (m, 1H), 5.78 (d, *J =* 15.8 Hz, 1H), 4.65 - 4.53 (m, 1H), 3.98 - 3.87 (m, 1H), 3.81 - 3.68 (m, 1H), 3.67 - 3.55 (m, 1H), 3.27 - 3.16 (m, 2H).

### Example 4A: Synthesis of (3R)-3-[4-(trifluoromethyl)phenyl]morpholin-4-yl 4-amino-7-chloro(10-hydroimidazo[1,5-a]quinoxalin-8-yl)one and Example 4B: (3S)-3-[4-(trifluoromethyl)phenyl]morpholin-4-yl 4-amino-7-chloro(10-hydroimidazo[1,5-a]quinoxalin-8-yl) one

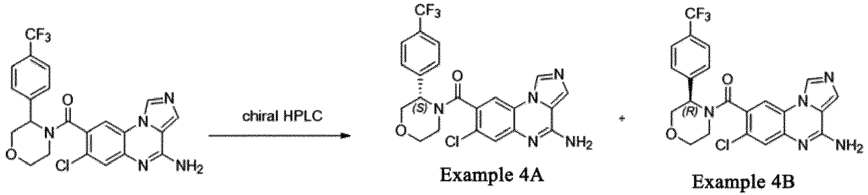

4-amino-7-chloro(10-hydroimidazo[1,5-*a*]quinoxalin-8-yl)3-[4-(trifluoromethyl)phenyl]morpholin-4-ylone (70 mg, 0.15 mmol) was separated by chiral HPLC (Chromatographic column: CHIRALPAK I 2×25 cm; Mobile phase A: methyl tert-butyl ether (0.5% 2 M/L ammonia-methanol solution), Mobile phase B: methanol; Flow rate: 20 mL/min; Gradient: isocratic; Wavelength: 254/220 nm; Prepeak retention time: 5.647 min; Postpeak retention time: 11.537 min; Injection volume: 2.7 mL; Number of operations: 10) to give prepeak

(3*R*)-3-[4-(trifluoromethyl)phenyl]morpholin-4-yl4-amino-7-chloro(10-hydroimidazo[1,5-*a*]quinoxalin-8-yl) one(8.44 mg, 12%), which was a white solid, and postpeak (3*S*)-3-[4-(trifluoromethyl)phenyl]morpholin-4-yl4-amino-7-chloro(10-hydroimidazo[1,5-*a*]quinoxalin-8-yl) one(7.54 mg, 11%), which was a white solid.

### Prepeak Example 4A (3R)-3-[4-(trifluoromethyl)phenyl]morpholin-4-yl4-amino-7-chloro(10-hydroimidazo[ 1,5-a]quinoxalin-8-yl)one

LCMS (ESI) m/z: **476.05**[M+H]⁺
¹H NMR (400 MHz, DMSO-*d*₆) δ 9.20-8.95 (m, 1H), 8.39-8.23 (m, 1H),7.93-7.87 (m, 1H), 7.83-7.39 (m, 7H), 5.79-4.94 (m, 1H), 4.59-4.41 (m, 1H), 4.29-3.77 (m, 2H), 3.69-3.55 (m, 1H),3.25-3.03 (m, 2H). Postpeak **Example 4B** (3*S*)-3-[4-(trifluoromethyl)phenyl]morpholin-4-yl 4-amino-7-chloro(10-hydroimidazo[1,5-*a*]quinoxalin-8-yl)one
LCMS (ESI) m/z: **476.05**[M+H]⁺
¹H NMR (400 MHz, DMSO-*d*₆) δ 9.20-8.95 (m, 1H), 8.39-8.23 (m, 1H),7.93-7.40 (m, 8H), 5.79-4.93 (m, 1H), 4.59-4.11 (m,2H), 3.91-3.88 (m, 1H), 3.74-3.69 (m, 1H),3.60-3.58(m,1H),3.25-3.03 (m,1H).

### Example 5:

### Synthesis of (4-amino-7-chloroimidazo[1,5-a]quinoxalin-8-yl)(2-(4-fluorophenyl)pyrrolidin-1-yl)methanone

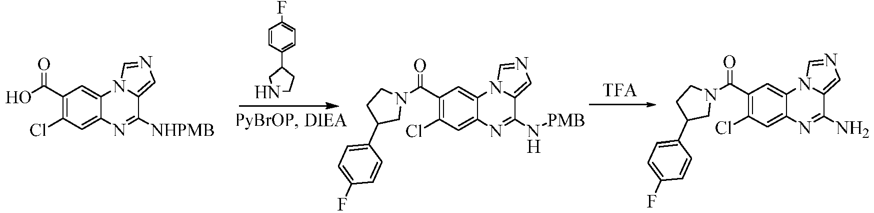

### Step I: Synthesis of (7-chloro-4-((4-methoxybenzyl)amino)imidazo[1,5-a]quinoxalin-8-yl)(2-(4-fluorophe nyl)pyrrolidin-1-yl)methanone

At room temperature, 7-chloro-4-((4-methoxylbenzyl)amino)imidazo[1,5-*a*]quinoxalin-8-carboxylic acid (200 mg, 0.522 mmol) was dissolved in *N,N*-dimethylacetamide (5 mL), and then 2-(4-fluorophenyl)pyrrolidine (104 mg, 0.627 mmol), tripyrrolidylphosphonium bromide hexafluorophosphate (369 mg, 0.7837 mmol) and *N,N*-diisopropylethylamine (207 mg, 1.567 mmol) were added successively to a flask. The mixture was stirred at 50°C for 3 h, after the reaction was completed, the reaction solution was poured into water (40 mL), extracted with ethyl acetate (20 mL×3), with the organic phases combined, dried over anhydrous sodium sulfate and filtered, the filtrate was concentrated under reduced pressure to obtain a crude product. The crude product was purified by to column chromatography on silica gel (DCM: MeOH=10:1) to obtain a yellow solid 7-chloro-*N*-cyclopropyl-4-((4-methoxybenzyl)amino)-*N*-((5-(trifluoromethyl)pyridin-2-yl)methy l)imidazo[1,5-*a*]quinoxalin-8-formamide (150 mg, yield: 54.2%).
LCMS (ESI) m/z: 531[M+H]⁺

### Step II: Synthesis of (4-amino-7-chloroimidazo[1,5-a]quinoxalin-8-yl)(2-(4-fluorophenyl)pyrrolidin-1-yl) methanone

At room temperature, (7-chloro-4-((4-methoxybenzyl)amino)imidazo[1,5-*a*]quinoxalin-8-yl)(2-(4-fluorophen yl)pyrrolidin-1-yl) methanone (150 mg, 0.2830 mmol) was dissolved in TFA (5 mL), and the mixture was stirred at 50°C for 3 h. After the reaction was completed, the reaction solution was concentrated under reduced pressure, then poured into water (10 mL). The pH was adjusted to 8 with a saturated sodium bicarbonate solution, the reaction solution was extracted with ethyl acetate (10 mL×3), with the organic phases combined, dried over anhydrous sodium sulfate and filtered, and the filtrate was concentrated under reduced pressure to obtain a crude product. The crude product was purified by column chromatography (DCM: MeOH=20:1) to obtain a white solid **(4-amino-7-chloroimidazo[1,5-*a*]quinoxalin-8-yl)(3-(4-(trifluoromethyl)phenyl)morpholino)methanone** (70 mg, yield: 60.4%).
LCMS (ESI) m/z: **410** [M+H]⁺
¹H NMR (400 MHz, DMSO-*d*₆) δ 9.14 (d, *J =* 7.5 Hz, 1H), 8.31 (d, *J =* 6.6 Hz, 1H), 7.93 (d, *J =* 7.2 Hz, 1H), 7.58 (d, *J =* 8.3 Hz, 2H), 7.48 (d, *J =* 12.9 Hz, 1H), 7.44 - 7.38 (m, 1H), 7.34 - 7.27 (m, 1H), 7.22 - 7.15 (m, 1H), 7.14 - 7.07 (m, 1H), 4.10 - 3.73 (m, 1H), 3.67 - 3.55 (m, 1H), 3.49 - 3.40 (m, 1H), 3.38 (dd, *J* = 8.7, 4.9 Hz, 1H), 3.23 (t, *J* = 9.8 Hz, 1H), 2.42 - 2.22 (m, 1H), 2.00 (ddq, *J* = 21.3, 11.8, 9.1 Hz, 1H).

### Example 6:

### Synthesis of (2R)-2-(4-fluorophenyl)piperidinyl4-amino-7-chloro(10-hydroimidazo[1,5-a]quinoxalin-8-yl)on e

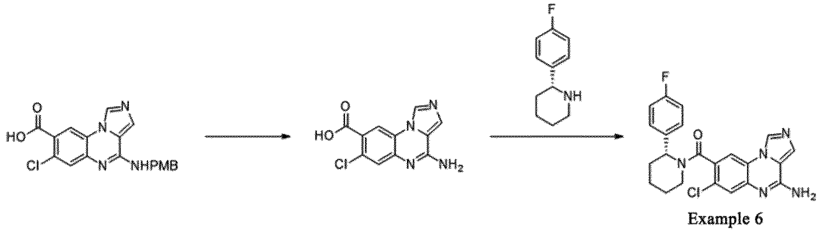

Step I, 7-chloro-4-{[(4-methoxylphenyl)methyl]amino}-10-hydroimidazo [1,5-*a*]quinoxalin-8-carboxylic acid (0.10 g, 0.26 mmol) was dissolved in trifluoroacetic acid (1 mL), and the reaction solution was stirred at 60°C for 4 h. After the reaction was completed, the mixture was concentrated under reduced pressure and diluted with ethyl acetate (5 mL) and a saturated sodium bicarbonate (5 mL). The aqueous layer was separated and extracted with ethyl acetate (10 × mL 3). The combined organic extracts were dried over anhydrous sodium sulfate, filtered and concentrated in vacuum to give 4-amino-7-chloro-10-hydroimidazo[1,5-*a*]quinoxalin-8-carboxyli c acid (0.05 g, 0.26 mmol, 73%), which was a brown solid.
LCMS (ESI): 262 [M+H]⁺

Step II: A solution of 4-amino-7-chloro-10-hydroimidazo[1,5-*a*]quinoxalin-8-carboxylic acid (20.0 mg, 0.08 mmol), (2*R*)-2-(4-fluorophenyl)piperidine (15.01 mg, 0.08 mmol), *N,N',N*'-tetramethyl-O-(7-azabenzotriazol-1-yl)urea hexafluorophosphate (43.43 mg, 0.11 mmol) and *N,N*-diisopropylethylamine (29.52 mg, 0.23 mmol) in dimethylformamide (1.00 mL) was stirred at room temperature for 2h. The reaction mixture was diluted with ethyl acetate (5 mL) and a saturated sodium bicarbonate solution (5 mL). The layers were separated and the aqueous layer was extracted with ethyl acetate (10× 3 mL). The combined organic phases were dried over anhydrous sodium sulfate, filtered and concentrated in vacuum and purified by PREP_HPLC (Columns: XBridge Shield RP18 OBD columns, 30 x 150 mm, 5 µm; Mobile phase A: Water (10 mmol/L NH₄HCO₃), Mobile Phase B: Acetonitrile; Flow rate: 60 mL/min; Gradient: 20% B to 55% B, 55% B in 10 minutes; Wavelength: 220 nm RT1 (min): 8.48; Number of operations: 0) to give (2*R*)-2-(4-fluorophenyl)piperidyl4-amino-7-chloro(10-hydroimidazo[1,5-*a*]quinoxalin-8-yl)one(14.12 mg, 0.076 mmol, yield: 44%), which was a white solid.

¹HNMR (400 MHz, DMSO-*d*₆) δ 9.25-8.98 (m, 1H), 8.37-8.28 (m, 1H), 7.94-7.87 (m, 1H), 7.59-7.57 (m, 2H), 7.55-7.48 (m, 2H), 7.41-7.17 (m, 3H), 5.93-4.55 (m, 1H), 3.27-3.24 (m, 1H), 3.00-2.90 (m, 1H), 2.71-2.54 (m, 1H), 2.40-2.25 (m, 1H),2.15-1.88 (m, 1H), 1.73-1.62 (m, 1H), 1.54-1.42 (m, 1H), 1.39-1.35 (m, 1H).
LCMS (ESI) m/z: 424.00[M+H]⁺

### Example 7

### Synthesis of (R)-(4-amino-7-fluorimidazo[1,5-a]quinoxalin-8-yl)(2-(4-fluorophenyl)piperidin-1-yl)metha none

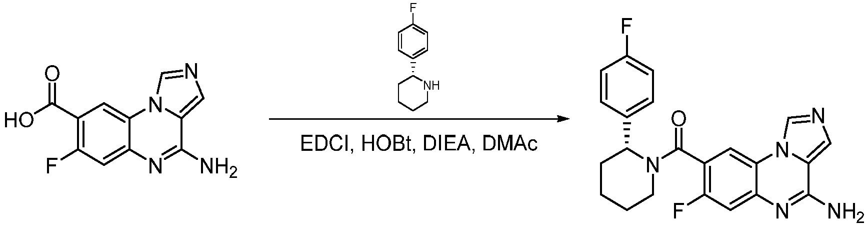

1-hydroxybenzotriazole (60.37 mg, 0.45 mmol), 1-ethyl-(3-dimethylaminopropyl)carbodiimide (85.65 mg, 0.45 mmol), diisopropylethylamine (209.98 mg, 1.62 mmol) and (*S*)-*N*-methyl-6-(trifluoromethyl)-2,3-dihydrobenzofuran-3-amine (73 mg, 0.41 mmol) were added successively to a solution of 4-amino-7-fluorimidazo[1,5-*a*]quinoxalin-8-carboxylic acid (100 mg, 0.41 mmol) in dimethylacetamide (4 mL). The reaction mixture was stirred at room temperature for 2 h. After the reaction was completed, the reaction solution was poured into water (5 mL), extracted with ethyl acetate (5 mL×3), dried over anhydrous sodium sulfate, concentrated by suction filtration, and purified by a column: Kinetex 5 m EVO C18, 30 mm ×150 mm; Mobile phase A: Water (10 mmol/L NH₄HCO₃), Mobile Phase B: Acetonitrile; Flow rate: 60 mL/min.mL/min; Gradient: from 20% B to 49% B in 8 min; Wavelength: 254 nm/220 nm; RT1(min):7.77 to give (*R*)-(4-amino-7-fluoroimidazo[1,5-a]quinoxalin-8-yl)(2-(4-fluorophe nyl)piperidin-1-yl)methanone (23.74 mL, 13.92 %), which was a white solid.
LCMS (ESI) m/z: 408.15[M+H]⁺
¹H NMR (400 MHz, Methanol-*d₄*) δ 9.18 (s, 1H), 8.35 (s, 1H), 7.92(m, 1H), 7.57-7.56 (m, 2H), 7.40-7.25 (m, 5H), 5.93 - 4.53 (m, 1H), 3.44-3.41 (m, 1H),2.97-2.67 (m, 1H),2.36-2.32 (m, 1H), 2.06-1.87 (m, 1H), 1.66-1.63(m,1H),1.49-1.23(m,3H).

### Example 8

### Synthesis of (4-amino-7-fluorimidazo[1,5-a]quinoxalin-8-yl)(3-(5-(trifluoromethyl)pyridin-2-yl)pyrrolid in-1-yl)methanone

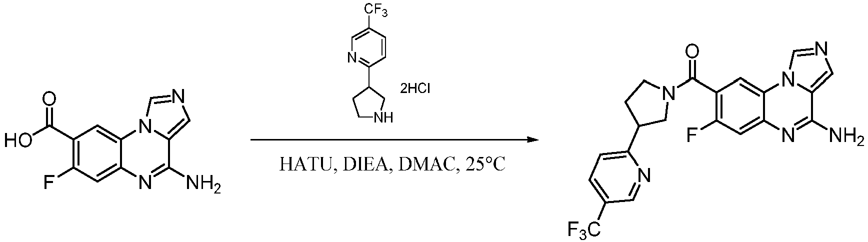

### Step I Preparation of (4-Amino-7-fluorimidazo[1,5-a]quinoxalin-8-yl)(3-(5-(trifluoromethyl)pyridin-2-yl) pyrrolidin-1-yl)methanone

At room temperature, 4-amino-7-fluoroimidazo[1,5-*a*]quinoxalin-8-carboxylic acid (200 mg, 0.80 mmol), 2-(pyrrolidin-3-yl)-5-(trifluoromethyl) pyridine hydrochloride (238 mg, 0.80 mmol), 2-(7-azobenzotriazole)-*N,N,N',N'*-tetramethylurea hexafluorophosphate (456 mg, 1.20 mmol) and DIEA (515 mg, 4.0 mmol) were placed in a flask and N,N-dimethylacetamide (5.0 mL) was added. The mixture was stirred to react at 25°C for 16 h. After the reaction was completed, a sodium bicarbonate aqueous solution (10 mL) was added and the reaction solution was extracted with ethyl acetate (20 mL). The organic phases were dried over sodium sulfate, filtered, concentrated under reduced pressure to give a crude product. The crude product was purified by column chromatography to give a target compound 4-amino-7-fluoroimidazo[1,5-*a*]quinoxalin-8-yl)(3-(5-(trifluoromethyl)pyridin-2-yl)pyrrolidin-1-yl)methanon e (90.0 mg, yield: 25%).
LCMS (ESI) m/z:445.2[M+H]⁺
¹H NMR (400 MHz, DMSO-*d*₆) δ 9.14 (d, *J* = 3.3 Hz, 1H), 9.00 - 8.84 (m, 1H), 8.33 (dd, *J =* 6.7, 3.5 Hz, 1H), 8.17 (ddd, *J =* 27.5, 8.3, 2.5 Hz, 1H), 7.92 (dd, *J =* 5.6, 0.7 Hz, 1H), 7.63 (dd, *J =* 35.8, 7.1 Hz, 3H), 7.23 (dd, *J =* 11.2, 8.6 Hz, 1H), 4.10 - 3.70 (m, 3H), 3.66 - 3.47 (m, 2H), 2.34 - 2.08 (m, 2H).

### Example 9

### Synthesis of (4-Amino-7-Fluorimidazo[1,5-a]quinoxalin-8-yl)(3-(5-(trifluoromethyl)phenyl)pyrrolidin-1-yl)methano ne

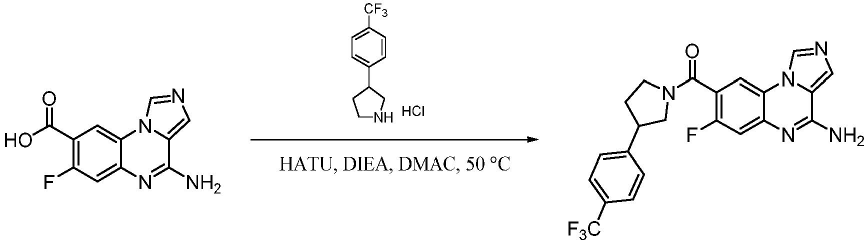

### Step I: Preparation of (4-amino-7-fluorimidazo[1,5-a]quinoxalin-8-yl)(3-(5-(trifluoromethyl)phenyl)pyrr olidin-1-yl)methanone

At room temperature, the compound 4-amino-7-fluoroimidazo[1,5-*a*]quinoxalin-8-carboxylic acid (200 mg, 0.8124 mmol), 2-(pyrrolidin-3-yl)-5-(trifluoromethyl)phenyl hydrochloride (159 mg, 0.7385 mmol), 2-(7-azobenzotriazole)-*N,N,N',N'*-tetramethylurea hexafluorophosphate (344 mg, 0.8862 mmol) and DIEA (292 mg, 2.216 mmol) were placed in a flask and N,N-dimethylacetamide (3 mL) was added. The mixture was stirred to react at 50°C for 16 h. After the reaction was completed, water (30 mL) was added and the reaction solution was extracted with ethyl acetate (20 mL). The organic phases were dried over sodium sulfate, filtered and the filtrate concentrated under reduced pressure to give a crude product. The crude product was purified by column chromatography (dichloromethane: methanol=10:1) to give a target compound 4-amino-7-fluoroimidazo[1,5-*a*]quinoxalin-8-yl) (3-(5-(trifluoromethyl)phencyclo-2-yl)pyrrolidin-1-yl)methanone (42 mg, yield: 12.9%).
LCMS (ESI) m/z:444.4[M+H]⁺
¹H NMR (400 MHz, DMSO-*d*₆) δ 9.14 (d, *J =* 7.8 Hz, 1H), 8.34 (dd, *J =* 6.6, 3.0 Hz, 1H), 7.93 (d, *J =* 7.1 Hz, 1H),7.73 (d, *J* = 8.0 Hz, 1H), 7.65 (d, *J =* 8.2 Hz, 1H), 7.63 - 7.55 (m, 3H), 7.51 (d, *J =* 8.1 Hz, 1H), 7.23 (dd, *J* = 14.8, 11.2 Hz, 1H),3.86 - 3.73 (m, 1H), 3.68 - 3.58 (m, 1H), 3.52 (td, *J* = 8.7, 3.3 Hz, 2H), 3.44 (q, *J* = 10.4, 9.7 Hz, 1H), 2.46 - 2.26 (m, 1H),2.18 - 1.91 (m, 1H).

### Example 10

### Synthesis of (4-amino-7-fluorimidazo[1,5-a]quinoxalin-8-yl) 3-(5-(trifluoromethyl)pyridin-2-yl)azetidin-1-yl)methanone

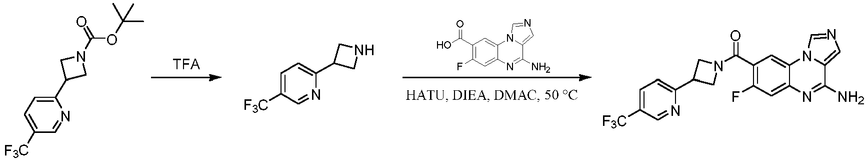

### Step I: preparation of 2-azetidin-3-yl-5-trifluoromethylpyridine

At room temperature, tert-butyl 3-(5-(trifluoromethyl)pyridin-2-yl)azetidin-1-carboxylate (200 mg, 0.6623 mmol) was dissolved in dichloromethane (5 mL), then trifluoroacetic acid (2 mL) was added and the mixture was stirred to react for 2 h at room temperature. The reaction was monitored by LCMS for completeness, and the reaction solution was concentrated under reduced pressure to give a crude product 2-azetidin-3-yl-5-trifluoromethylpyridine (150 mg, crude product), which was used directly in the next step without purification.
LCMS (ESI) m/z: 203.1 [M+H]⁺

### Step II: Preparation of (4-amino-7-fluorimidazo[1,5-a]quinoxalin-8-yl) 3-(5-(trifluoromethyl)pyridin-2-yl)azetidin-1-yl) methanone.

At room temperature, the compound 4-amino-7-fluoroimidazo[1,5-*a*]quinoxalin-8-carboxylic acid (200 mg, 0.8124 mmol), 2-azetidin-3-yl-5-trifluoromethylpyridine (181 mg, 0.8936 mmol), 2-(7-azobenzotriazole)-*N,N,N',N*'-tetramethylurea hexafluorophosphate (378 mg, 0.9748 mmol) and DIEA (321 mg, 2.437 mmol) were placed in a flask, *N,N-*dimethylacetamide (3 mL) was added, and the reaction mixture was stirred to react at 50°C for 16 h. After the reaction was completed, water (30 mL) was added and the reaction solution was extracted with ethyl acetate (20 mL). The organic phases were dried over sodium sulfate, filtered and the filtrate concentrated under reduced pressure to obtain a crude product. The crude product was purified by column chromatography (dichloromethane: methanol=10:1) to give a target compound 4-amino-7-fluoroimidazo[1,5-*a*]quinoxalin-8-yl) (3-(5-(trifluoromethyl)phencyclo-2-yl)pyrrolidin-1-yl)methanone (20 mg, yield: 6%).
LCMS (ESI) m/z:431.4[M+H]⁺
¹H NMR (400 MHz, DMSO-*d*₆) δ 9.25 - 9.17 (m, 1H), 9.07 - 8.93 (m, 1H), 8.63 - 8.36 (m, 1H), 8.19 (dt, *J =* 8.5, 2.6 Hz, 1H), 8.00 - 7.89 (m, 1H), 7.71 - 7.58 (m, 2H), 7.45 - 7.33 (m, 1H), 7.22 (dd, *J =* 11.6, 2.5 Hz, 1H), 4.59 - 4.36 (m, 2H), 4.35 - 4.20 (m, 2H).

### Example 11

### (S)-(4-amino-7-fluoroimidazo[1,5,a]quinoxalin-8-yl)(3-(4-trifluoromethoxy)phenyl)morpholino)methan one

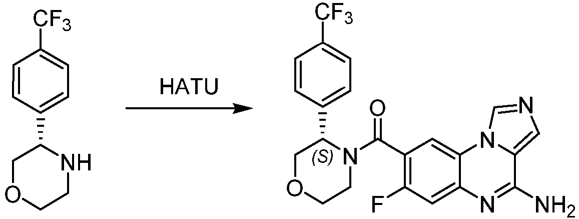

At room temperature, the compound (*S*) 3-(4-trifluoromethoxy)phenyl)morpholine (200 mg, 0.80 mmol), 2-(pyrrolidin-3-yl)-5-(trifluoromethyl)pyridine hydrochloride (238 mg, 0.80 mmol), 2-(7-azobenzotriazole)-*N,N,N',N'*-tetramethylurea hexafluorophosphate (456 mg, 1.20 mmol) and DIEA (515 mg, 4.0 mmol) were placed in a flask and *N, N*-dimethylacetamide (5.0 mL) was added. The mixture was stirred to react at 25°C for 16 h. After the reaction was completed, an aqueous sodium bicarbonate solution (10 mL) was added and the reaction solution was extracted with ethyl acetate (20 mL). The organic phases were dried over sodium sulfate, filtered and concentrated under reduced pressure to give a crude product. The crude product was purified by column chromatography to give a target compound (*S*)-(4-amino-7-fluoroimidazo[1,5,*a*]quinoxalin-8-yl)(3-(4-trifluor omethoxy)phenyl)morpholino) methanone (90.0 mg, yield: 21%).
LCMS (ESI) m/z: 460.2 [M+H]⁺

### Example 12

### (4-amino-7-fluorimidazo[1,5-a]quinoxalin-8-yl)((3R,5S)-3-methyl-5-(4-(trifluoromethyl)phenyl)morpho linyl)methanone

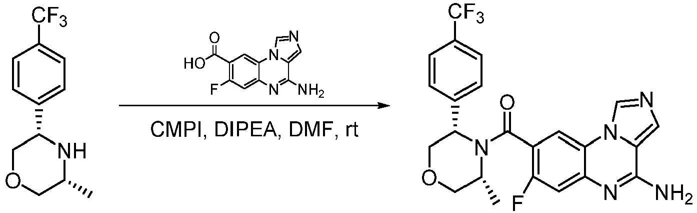

Compounds 4-amino-7-fluoroimidazo[1,5-*a*]quinoxalin-8-carboxylic acid (100.0 mg, 0.41 mmol) and (3*R*,5*S*)-3-methyl-5-(4-(trifluoromethyl)phenyl)morpholine (150.0 mg, 0.61 mmol) were dissolved in DMF (3.0 mL), and triethylamine (124 mg, 1.23 mmol) and CMPI (156 mg, 0.61 mmol) were added, and the reaction solution was stirred at room temperature for 72 h. The reaction solution was diluted with ethyl acetate (20 mL), filtered with diatomaceous earth, washed with water (10 mL × 2) and a saturated saline solution (10 mL), dried over anhydrous sodium sulfate and filtered, and the filtrate was evaporated to dryness, separated and purified by column chromatography (0-5% methanol/dichloromethane) to give the target compound (21.0 mg, yield: 11%).
LCMS (ESI) m/z: 474.20[M+H]⁺
¹H NMR (400 MHz, DMSO-*d*₆) δ 9.16 (d, *J =* 11.1 Hz, 1H), 8.34 (d, *J =* 9.1 Hz, 1H), 7.92 (s, 1H),7.79 (d, *J* = 14.7 Hz, 4H), 7.59 (s, 2H), 7.26 (d, *J =* 10.8 Hz, 1H), 4.76 (s, 1H), 3.75 (d, *J =* 47.5 Hz,4H), 2.68 (s, 1H), 1.24 (d, *J =* 5.9 Hz, 3H).

### Example 13

### (4-amino-7-fluimidazo[1,5-a]quinoxalin-8-yl)(3-(4-(trifluoromethyl)phenyl)piperidin-1-yl)methanone

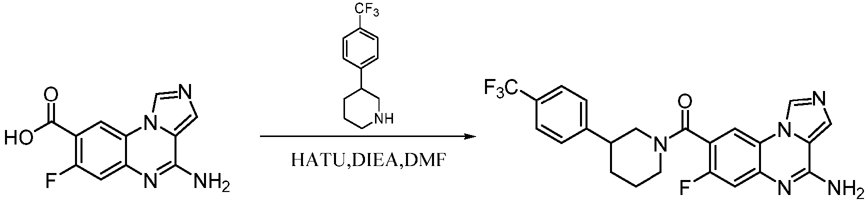

Compounds 4-amino-7-fluoroimidazo[1,5-*a*]quinoxalin-8-carboxylic acid (210.0 mg, 0.85 mmol) and 3-(4-(trifluoromethyl)phenyl)piperidine (226.0 mg, 0.85 mmol) were dissolved in DMF (8.0 mL), then HATU (496.0 mg, 1.28 mmol) and DIEA (337.0 mg, 2.56 mmol) were added, and the mixture was stirred to react at room temperature for 16 h. After the reaction was completed, water (20 mL) was added thereto and the reaction solution was extracted with ethyl acetate (30 mL ×2), with the organic phases combined, washed with water (20 mL×3) and a saturated salt (20 mL), dried over anhydrous sodium sulfate and filtered, and the filtrate was evaporated to dryness to give a crude product. The crude product was separated and purified by column chromatography (0-5% methanol/dichloromethane solution) to give (4-amino-7-fluoroimidazo[1,5-*a*]quinoxalin-8-yl)(3-(4-(trifluoromethyl)phenyl)piperidin-1-yl)methanone (130.0 mg, yield: 33%).
LCMS (ESI) m/z:458.2[M+H]⁺
¹H NMR (400 MHz, DMSO-*d*₆) δ 9.14 (s, 1H), 8.31 (d, *J =* 6.5 Hz, 1H), 7.92 (d, *J =* 10.2 Hz, 1H),7.73 (d, *J* = 8.1 Hz, 1H), 7.66 - 7.49 (m, 4H), 7.40 (d, *J =* 7.9 Hz, 1H), 7.22 (dd, *J =* 23.0, 11.0 Hz, 1H),4.62 (t, *J =* 15.0 Hz, 1H), 3.55 (t, *J =* 16.0 Hz, 1H), 3.22 - 2.76 (m, 3H), 2.09 - 1.47 (m, 4H).

### Example 13A: Synthesis of (R)-4-amino-7-fluorimidazo[1,5-a]quinoxalin-8-yl)(3-(4-(trifluoromethyl)phenyl) piperidin-1-yl)methanone and Example 13B: (S)-4-amino-7-fluorimidazo[1,5-a]quinoxalin-8-yl)(3-(4-(trifluoromethyl)phenyl)piperidin-1-yl)methanone

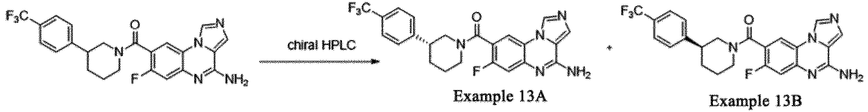

(4-amino-7-fluimidazo[1,5-*a*]quinoxalin-8-yl)(3-(4-(trifluoromethyl)phenyl)piperidin-1-yl)methanone (55 mg, 0.15 mmol) was separated by chiral HPLC (column: ChiralPak IG,100×40 mm I.D., 10 µm; Mobile phase: A for CO₂ and B for MeOH (0.1% NH₃H₂O), 50%/50%, flow rate:140 mL/min) to give prepeak (*R*)-4-amino-7-fluoroimidazo[1,5-*a*]quinoxalin-8-yl)(3-(4-(trifluoromethyl)phenyl)piperidin-1-yl)methanone (19 mg, 35%), which was a white solid and postpeak (*S*)-4-amino-7-fluoroimidazo[1,5-*a*]quinoxalin-8-yl)(3-(4-(trifluoromethyl)phenyl)piperidin-1-yl)methanone (22 mg. 40%), which was a white solid.

### Prepeak Example 13A: (R)-4-amino-7-fluorimidazo[1,5-a]quinoxalin-8-yl)(3-(4-(trifluoromethyl)phenyl)pip eridin-1-yl)methanone

LCMS (ESI) m/z: **458.2**[M+H]⁺
¹H NMR (400 MHz, DMSO-*d*₆) δ 9.13 (s, 1H), 8.30 (s, 1H), 7.91 (d, *J =* 10.2 Hz, 1H), 7.73 (d, *J =* 8.1Hz, 1H), 7.58 (t, *J =* 11.4 Hz, 4H), 7.40 (d, *J =* 8.0 Hz, 1H), 7.24 (d, *J =* 10.9 Hz, 1H), 4.61 (t, *J =* 14.3Hz, 1H), 3.54 (t, *J =* 16.5 Hz, 1H), 3.17 (d, *J =* 5.3 Hz, 1H), 2.87 (d, *J =* 11.9 Hz, 2H), 1.99 - 1.42 (m,4H).

### Postpeak Example 13B: (S)-4-amino-7-fluorimidazo[1,5-a]quinoxalin-8-yl)(3-(4-(trifluoromethyl)phenyl)pi peridin-1-yl)methanone

LCMS (ESI) m/z: **458.2**[M+H]⁺
¹H NMR (400 MHz, DMSO-*d*₆) δ 9.13 (s, 1H), 8.30 (s, 1H), 7.91 (d, *J =* 10.2 Hz, 1H), 7.73 (d, *J =* 8.1Hz, 1H), 7.58 (t, *J =* 11.4 Hz, 4H), 7.40 (d, *J =* 8.0 Hz, 1H), 7.24 (d, *J =* 10.9 Hz, 1H), 4.61 (t, *J =* 14.3Hz, 1H), 3.54 (t, *J =* 16.5 Hz, 1H), 3.17 (d, *J =* 5.3 Hz, 1H), 2.87 (d, *J =* 11.9 Hz, 2H), 1.99 - 1.42 (m,4H).

### Example 14

### (R)-(4-aminoimidazo[1,5-a]quinoxalin-8-yl)(2-(4-fluorophenyl)piperidin-1-yl)methanone

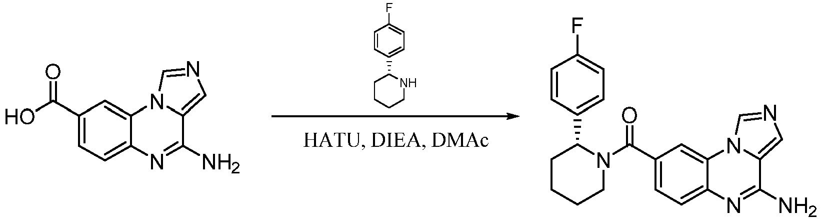

4-amino-10-hydroimidazo[1,5-*a*]quinoxalin-8-carboxylic acid (10 mg, 0.04 mmol), (2R)-2-(4-fluorophenyl)piperidine (8 mg, 0.05 mmol), *N,N,N',N*'-tetramethyl-O-(7-azabenzotriazol-1-yl)urea hexafluorophosphate (25 mg, 0.07 mmol), *N,N*-diisopropylethylamine (17 mg, 0.13 mmol) in dimethylformamide (1.00 mL) were stirred at room temperature for 2 h, and the reaction mixture was diluted with ethyl acetate (5 mL) and a saturated sodium bicarbonate solution (5 mL). The layers were separated and the aqueous layer was extracted with ethyl acetate (3 × 5 mL). The combined organic extracts were dried over anhydrous sodium sulfate, filtered and the crude product concentrated in vacuum was purified by high performance liquid chromatography (Columns: XBridge Shield RP18 OBD,30×150 mm, 5 µm; Mobile phase A: Water (10 mmol/L sodium bicarbonate), mobile phase B: Acetonitrile; Flow rate: 60 mL/min; Gradient: 20% B to 55% B, 55% B in 10 minutes; Wavelength: 220 nm RT1 (min): 8.48; Number of operations: 0) to give (*R*)-(4-aminoimidazo[1,5-a]quinoxalin-8-yl)(2-(4-fluorophenyl)piperid-1-yl) methanone (0.95 mg, yield: 6%), which was a white solid.
LCMS (ESI) m/z: 390.30[M+H]⁺
¹H NMR (400 MHz, DMSO-*d*₆) *δ* 9.17 (s, 1H), 8.30 (s, 1H), 7.91 (s, 1H), 7.46-7.38 (m, 5H), 7.26-7.21 (m, 2H), 3.23-3.22(m, 1H), 2.44-2.41 (m, 2H), 2.07(s,2H),1.67-1.64 (m, 1H), 1.56-1.55 (m, 2H), 1.23 (s, 1H), 0.85 (s, 1H).

### Example 15: Synthesis of (S)-(4-amino-7-fluoroimidazo[1,5-a]quinoxalin-8-yl)(2-(4-(trifluoromethyl)phenyl) morpholinyl)methanone

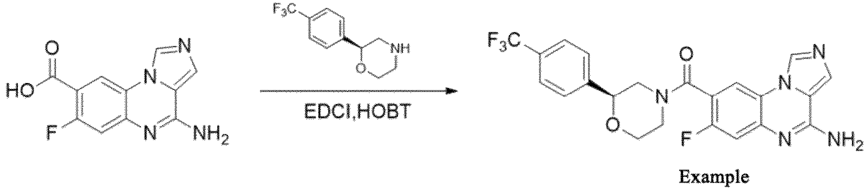

At room temperature, a compound 4-amino-7-fluoro-10-hydroimidazo[1,5-*a*]quinoxalin-8-carboxylic acid (387 mg, 1.563 mmol) was dissolved in DMF (3 mL) and 1-(3-dimethylaminopropyl)-3-ethylcarbodiimide hydrochloride (100 mg, 1.563 mmol) and 1-hydroxybenzotriazole (71 mg, 0.521 mmol) were added, and then DIEA (671 mg, 5.208 mmol) was added and the mixture was stirred to react at room temperature for 0.5 h. S-2-(4-trifluoromethylphenyl)morpholine (300.0 mg, 1.302 mmol) was then added and the mixture reacted at room temperature for 16 h. When LCMS indicated that the reaction was completed, water (40 mL) was poured, the mixture was extracted with ethyl acetate (40×3 mL), the organic phases were washed with a saturated salt (40 mL), dried over anhydrous sodium sulfate and filtered, the filtrate was concentrated under reduced pressure to give the crude product. The crude product was separated and purified by column chromatography (0-5% methanol/methylene chloride) to give (*S*)-(4-amino-7-fluoroimidazo[1,5-*a*]quinoxalin-8-yl)(2-(4-(trifluoromethyl)phenyl)morpholinyl)meth anone (280 mg, yield: 46.9%).
LCMS (ESI) m/z: 460.2 [M+H]⁺
¹H NMR (400 MHz, DMSO-*d*₆) δ 9.13 (s, 1H), 8.33 (d, *J =* 6.5 Hz, 1H), 7.94 (d, *J =* 4.6 Hz, 1H), 7.84 -7.58 (m, 5H), 7.51 (d, J = 8.0 Hz, 1H), 7.24 (t, *J =* 12.0 Hz, 1H), 4.65 (d, *J* = 9.5 Hz, 1H), 4.62 - 3.93 (m, 2H), 3.81 - 3.47 (m, 2H), 3.29 - 2.85 (m, 2H).

### Example 16 Synthesis of (R)-(4-amino-7-fluorimidazo[1,5-a]quinoxalin-8-yl)(2-(4-(trifluoromethyl)phenyl) pyrrolidin-1-yl)methanone

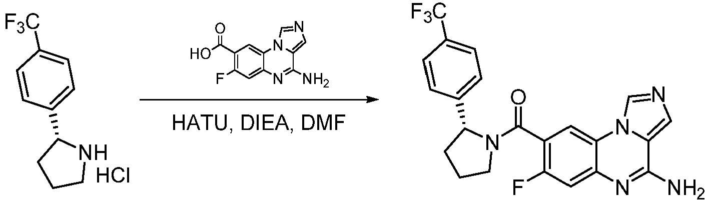

4-amino-7-fluoroimidazo[1,5-*a*]quinoxalin-8-carboxylic acid (97.70 mg, 0.40 mmol) and (*R*)-2-(4-(trifluoromethyl)phenyl)pyrrolidine hydrochloride (100.0 mg, 0.40 mmol) were dissolved in DMF (8.0 mL) and HATU (228 mg, 0.60 mmol) and DIEA (155 mg, 1.20 mmol) were added, and the reaction solution was stirred at room temperature for 16 h. After the reaction was completed, water (20 mL) was added thereto and the reaction solution was extracted with ethyl acetate (30 mL × 2), with organic phases combined,washed with water (20 mL×3) and a saturated salt (20 mL), dried over anhydrous sodium sulfate and filtered, and the filtrate was evaporated to dryness to give a crude product. The crude product was separated and purified by column chromatography (0-5% methanol/dichloromethane solution) to give (*R*)-4-amino-7-fluoro-N, 1-dimethyl-*N*-(7-(trifluoromethyl)pyran-4-yl)-1*H*-pyrazolo[4,3-*c*]quinolin-8-carboxylamide (130.0 mg, yield: 73%).
LCMS (ESI) m/z: 444.2[M+H]⁺
¹H NMR (400 MHz, CDCl₃) δ 8.97 (d, *J =* 163.2 Hz, 1H), 8.43 - 7.83 (m, 2H), 7.73 (d, *J =* 8.1Hz, 2H), 7.62 - 7.45 (m, 3H), 7.40 - 7.20 (m, 1H), 7.17 - 6.81 (m, 1H), 5.09 (dt, *J =* 129.5, 6.7 Hz, 1H),3.94 - 3.62 (m, 1H), 3.58 - 3.31 (m, 1H), 2.07 - 1.69 (m, 2H).

### Example 17 (S)-(4-amino-7-fluorimidazo[1,5-a]quinoxalin-8-yl)(2-(4-(trifluoromethyl)phenyl)pyrrolidin-1-y l)methanone

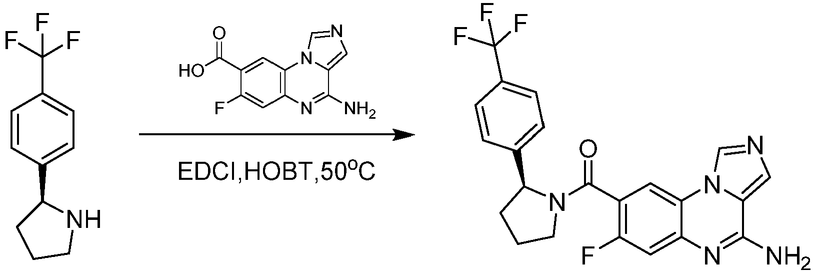

At room temperature, a compound 4-amino-7-fluoro-10-hydroimidazo[1,5-*a*]quinoxalin-8-carboxylic acid (137 mg, 0.558 mmol) was dissolved in DMF (3 mL) and 1-(3-dimethylaminopropyl)-3-ethylcarbodiimide hydrochloride (107 mg, 0.558 mmol) and 1-hydroxybenzotriazole (76 mg, 0.558 mmol) were added, and then DIEA (240 mg, 1.860 mmol) was added and the mixture was stirred to react at room temperature for 0.5 h. S-2-(4-trifluoromethylphenyl)pyrrolidine (100.0 mg, 0.465 mmol) was then added and the mixture reacted at room temperature for 16 h. When LCMS indicated that the reaction was completed, water (20 mL) was poured, the mixture was extracted with ethyl acetate (20 × 3 mL), the organic phases were washed with a saturated salt (20 mL), dried over anhydrous sodium sulfate and filtered, the filtrate was concentrated under reduced pressure to obtain the crude product. The crude product was separated and purified by column chromatography (0-5% methanol/methylene chloride) to give (*S*)-(-4-amino-7-fluoroimidazo[1, 5-*a*]quinoxalin-8-yl)(2-4-(trifluoromethyl)phenyl)pyrrolidin-1-yl)m ethanone (15 mg, yield: 7.28%).
LCMS (ESI) m/z: 444.2 [M+H]⁺
¹H NMR (400 MHz, DMSO-*d*₆) δ 8.99 (*d, J =* 163.3 Hz, 1H), 8.33 (d, *J =* 6.6 Hz, 1H), 7.97 -7.81 (m, 1H), 7.74 (d, *J =* 8.1 Hz, 1H), 7.66 -7.37 (m, 4H), 7.26 (d, *J =* 11.2 Hz, 1H), 7.22 -6.87 (m, 1H), 5.10 (ddd, *J* = 128.8, 7.9, 5.4 Hz, 1H), 3.91 -3.44 (m, 2H), 2.50 -1.70 (m, 4H).

### Example 18

### Synthesis of 2-(1,5-naphthyridin-3-yl)piperidin-1-yl)(4-aminoimidazo[1,5-a]quinoxalin-8-yl)methanone

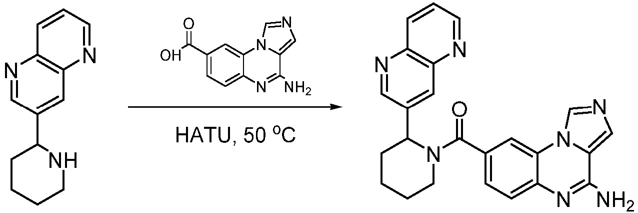

### Step I: Synthesis of 2-(1,5-naphthyridin-3-yl)piperidin-1-yl)(4-aminoimidazo[1,5-a]quinoxalin-8-yl)methanone

At room temperature, 3-(2-piperidinyl)-1,5-naphthyridine (50 mg, 0.2344 mmol) was dissolved in DMAC (4 mL), 4-aminoimidazo[1,5-*a*]quinoxalin-8-carboxylic acid (54 mg, 0.2344 mmol), HATU (182 mg, 0.4689 mmol) and *N,N*-diisopropylethylamine (93 mg, 0.7033 mmol) were stirred at 25°C for 2 h, the reaction solution was poured into water (20 mL), extracted with ethyl acetate (15 mL×3), the organic phases were combined and dried over anhydrous sodium sulfate and filtered and the filtrate concentrated under reduced pressure to give a crude product. The crude product was purified by silica gel column chromatography (methylene chloride: methanol=10:1) to give a product 2-(1,5-naphthyridin-3-yl)piperidin-1-yl)(4-aminoimidazo[1,5-*a*]quinoxalin-8-yl)methanone (4 mg, yield: 4%).
LCMS (ESI) m/z: 424.5 [M-H]⁺
¹H NMR (400 MHz, DMSO-*d₆*) δ 9.23 (s, 1H), 9.04 (dd, *J =* 4.4, 1.7 Hz, 2H), 8.46 (d, *J =* 8.4 Hz, 1H), 8.40 (s, 1H), 8.33 (s, 1H), 7.91 (s, 1H), 7.80 (dd, *J =* 8.5, 4.2 Hz, 1H), 7.45 (d, *J =* 14.2 Hz, 4H), 5.91 (s, 1H), 3.96 (s, 1H), 3.01 (s, 1H), 2.65 (d, *J =* 15.0 Hz, 1H), 2.10 (t, *J =* 12.2 Hz, 1H), 1.78 - 1.42 (m, 5H).

### Example 19

### Synthesis of (4-aminoimidazo[1,5-a]quinoxalin-8-yl)(2-(4-fluorophenoxy)phenyl)piperidin-1-yl)methanone

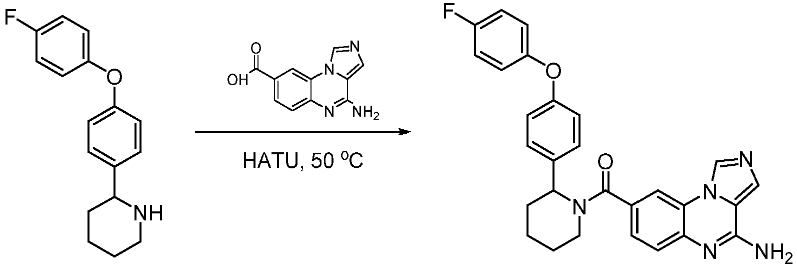

### Step I: Synthesis of (4-Aminoimidazo[1,5-a]quinoxalin-8-yl)(2-(4-fluorophenoxy)phenyl)piperidin-1-yl)met hanone

At room temperature, 2-(4-(4-fluorophenoxy)phenyl)piperidine (100 mg, 0.3685 mmol) was dissolved in DMAC (5 mL), 4-aminoimidazo[1,5-*a*]quinoxalin-8-carboxylic acid (84 mg, 0.3685 mmol), HATU (286 mg, 0.7371 mmol), and *N,N*-diisopropylethylamine (146 mg, 1.106 mmol) were added, the mixture was stirred at 25°C for 2 h, the reaction solution was poured into water (20 mL), extracted with ethyl acetate (15 mL ×3), with the organic phases combined, dried over anhydrous sodium sulfate and filtered and the filtrate was concentrated under reduced pressure to give a crude product. The crude product was purified by silica gel column chromatography (dichloromethane: methanol=10:1) to give a product (4-aminoimidazo[1,5-*a*]quinoxalin-8-yl)(2-(4-fluorophenoxy)phenyl)piperidin-1-yl)methanone(50 mg, yield: 28%), which was a white solid.

¹H NMR (400 MHz, DMSO-*d*₆) δ 9.16 (s, 1H), 8.28 (d, *J =* 1.8 Hz, 1H), 7.92 (s, 1H), 7.43 (d, *J =* 8.3 Hz, 4H), 7.25 (t, *J =* 8.8 Hz, 2H), 7.18 (dd, *J =* 8.3, 1.8 Hz, 1H), 7.16 - 7.08 (m, 3H), 6.95 - 6.84 (m, 2H), 5.60 (s, 1H), 3.85 (s, 1H), 2.86 (s, 1H), 2.40 (d, *J =* 13.9 Hz, 1H), 1.92 (t, *J =* 14.2 Hz, 1H), 1.74 - 1.48 (m, 3H), 1.39 (s, 1H).
LCMS (ESI) m/z: 482.5 [M+H]⁺

### Example 20

### Synthesis of (4-amino-7-fluimidazo[1,5-a]quinoxalin-8-yl) 2-(1-methylpyrazolo[3,4-b]pyridin-5-yl)piperid in-1-methanone (APRN-2102-32-732)

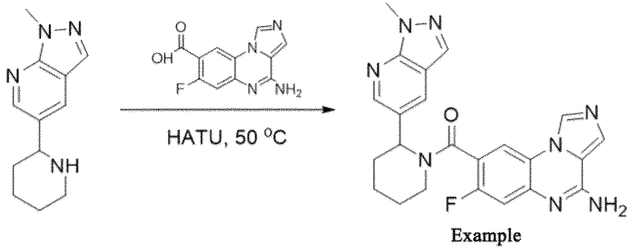

### Step I: Synthesis of (4-aminoimidazo[1,5-a]quinoxalin-8-yl) 2-(1-methylpyrazolo[3,4-b]pyridin-5-yl)piperidi n-1-methanone

At room temperature, 1-methyl-5-piperidin-2-yl-1*H*-pyrazolo[3,4-*b*]pyridine (100 mg, 0.461 mmol) was dissolved in DMAC (4 mL), 4-amino-7-fluoroimidazolo[1,5-*a*]quinoxalin-8-carboxylic acid (136 mg, 0.552 mmol), HATU (212 mg, 0.552 mmol) and *N,N*-diisopropylethylamine (238 mg, 1.844 mmol) at 50°C for 16 h, the reaction solution was poured into water (40 mL), extracted with ethyl acetate (20 mL×3), with the organic phases combined, dried over anhydrous sodium sulfate and filtered and the filtrate was concentrated under reduced pressure to give a crude product. The crude product was purified by silica gel hromatograpic column (methylene chloride: methanol=10:1) to give a product (4-amino-7-fluimidazo[1,5-*a*]quinoxalin-8-yl)2-(1-methylpyrazolo[3,4-*b*]p yridin-5-yl)piperidin-1-methanone (30 mg, yield:14.8%), which was a white solid.
LCMS (ESI) m/z: 441.2 [M+H]⁺
¹H NMR (400 MHz, DMSO-*d*₆) δ 9.20 (s, 1H), 8.77 - 8.39 (m, 2H), 8.19 (d, *J =* 22.1 Hz, 2H), 7.94 (s, 1H), 7.59 (s, 2H), 7.28 (d, *J =* 10.9 Hz, 1H), 6.14 (s, 1H), 4.08 (d, *J =* 6.9 Hz, 3H), 3.46 (s, 1H), 3.03 (s, 1H), 2.60 (d, *J =* 14.1 Hz, 1H), 1.98 (m, 1H), 1.75 - 1.46 (m, 4H).

### Example 21

### Synthesis of (4-aminoimidazo[1,5-a]quinoxalin-8-yl) 2-(5-(trifluoromethyl)pyridin-2-yl)piperidin-1-yl)metha none

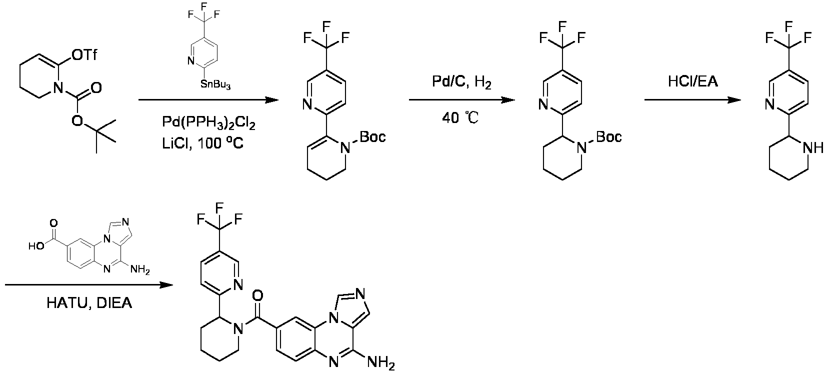

### Step I: Synthesis of tert-butyl 5'-trifluoromethyl-5,6-dihydro-[2,2'-bipyridine]-1(4H)-carboxylate

Under an atmosphere of N₂, 2-tributylstannylalkyl-5-trifluoromethylpyridine (400 mg, 0.906 mmol), lithium chloride (115 mg, 2.72 mmol), and bis-triphenylphosphine dichloride palladium (64.0 mg, 0.09 mmol) were successively added to a solution of tert-butyl 6-((trifluoromethylsulfonyl)oxy)-3,4-dihydropyridine-1(2*H*)-carboxylate (300 mg, 0.906 mmol) in toluene (10 mL) then the reaction mixture was stirred at 100°C for 16 h, the system was poured into water (50 mL), extracted with ethyl acetate (40 mL ×3), with the organic phases combined, dried over anhydrous sodium sulfate and filtered to remove anhydrous sodium sulfate, and the filtrate was concentrated to dryness in vacuum to give a crude product. The crude product was purified by silica gel column chromatography (petroleum ether: ethyl acetate=10:1) to give tert-butyl 5'-trifluoromethyl-5,6-dihydro-[2,2'-bipyridyl]-1(4*H*)-carboxylate, which was a brown solid (70 mg, 23.6% yield).
LCMS (ESI) m/z: 329.2 [M+H]⁺

### Step II: Synthesis of tert-butyl 2-(5-(trifluoromethyl)pyridin-2-yl)piperidin-1-carboxylate

Under an N₂ atmosphere, tert-butyl 5'-trifluoromethyl-5,6-dihydro-[2,2'-bipyridin]-1(4*H*)- carboxylate (70 mg, 0.213 mmol) was dissolved in ethanol (3 mL), then Pd/C (400 mg, 0.426 mmol) and ammonium formate (269 mg, 4.26 mmol) were added, and the mixture was purged with hydrogen three times using balloons and then stirred at 40°C for 8 hours to complete the reaction.. After LCMS showed that the reaction was completed, the mixture was filtered, and the filtrate was concentrated under reduced pressure to give a crude product tert-butyl 2-(5-(trifluoromethyl)pyridin-2-yl)piperidin-1-carboxylate (60 mg, crude product).
LCMS (ESI) m/z: 331.2 [M+H]⁺

### Step III: Synthesis of 2-(piperidin-2-yl)-5-trifluoromethylpyridine

At room temperature, the compound 2-(5-(trifluoromethyl)pyridin-2-yl)piperidin-1-tert-buty carboxylate (190 mg, 0.759 mmol) was dissolved in hydrochloric acid/ethyl acetate (5 mL). The mixture was stirred to react at room temperature for 3 h and concentrated under reduced pressure to give a crude product 2-(piperidin-2-yl)-5-trifluoromethylpyridine (50 mg, crude product), which was directly used in the next step without purification.
LCMS (ESI) m/z: 231.2 [M+H]⁺

### Step IV: Synthesis of 4-aminoimidazo[1,5-a]quinoxalin-8-yl)2-(5-(trifluoromethyl) pyridin-2-yl)piperidin-1-yl)methanone

At room temperature, 2-(piperidin-2-yl)-5-trifluoromethylpyridine (50 mg, 0.552 mmol) was dissolved in DMAC (4 mL), 4-aminoimidazo[1,5-*a*]quinoxalin-8-carboxylic acid (53 mg, 0.230 mmol), HATU (106 mg, 0.276 mmol) and *N,N*-diisopropylethylamine (119 mg, 0.922 mmol) were added, the mixture was stirred at 50°C for 16 h, after the reaction was completed, the reaction solution was poured into water (20 mL) and extracted with ethyl acetate (20 mL ×3), with the organic phasescombined, dried over anhydrous sodium sulfate and filtered, and the filtrate is concentrated under reduced pressure to give a crude product. The crude product was purified by silica gel column chromatography (methylene chloride: methanol=10:1) to give a product 4-aminoimidazo[1,5-*a*]quinoxalin-8-yl)2-(5-(trifluoromethyl)pyridin-2-yl)piperidin-1-yl)methanone (8 mg, yield: 7.91%), which was a pale yellow solid.
LCMS (ESI) m/z: 441.2 [M+H]⁺
¹H NMR (400 MHz, DMSO-*d*₆) δ 9.19 (s, 1H), 9.04 (d, *J* = 2.4 Hz, 1H), 8.38 - 8.19 (m, 2H), 7.92 (s, 1H), 7.69 (d, *J* = 8.3 Hz, 1H), 7.44 (s, 4H), 5.92 (s, 1H), 3.73 (s, 1H), 2.64 (s, 1H), 2.06 - 1.85 (m, 1H), 1.76 - 1.42 (m, 3H), 1.40 - 1.24 (m, 2H).

### Example 22 Synthesis of (4-aminoimidazo[1,5-a]quinoxalin-8-yl)2-(4-(trifluoromethoxy)phenyl)piperidin-1-yl methanone

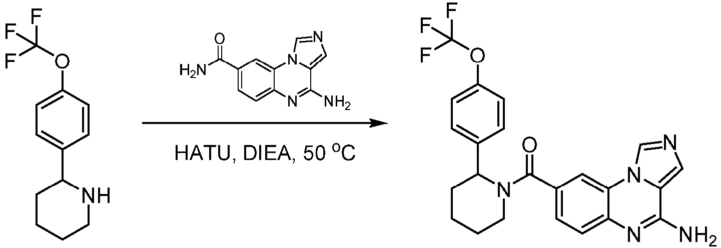

At room temperature, 2-(4-(trifluoromethoxy)phenyl)piperidine (100 mg, 0.4078 mmol) was dissolved in DMAC (5 mL), 4-aminoimidazo[1,5-*a*]quinoxalin-8-carboxylic acid (93 mg, 0.4078 mmol), HATU (316 mg, 0.8155 mmol), and N,N-diisopropylethylamine (161 mg, 1.223 mmol) were added, the mixture was stirred at 50°C for 2 h, the reaction solution was poured into water (20 mL), extracted with ethyl acetate (15 mL×3), with the organic phases combined, dried over anhydrous sodium sulfate and filtered and the filtrate was concentrated under reduced pressure to obtain a crude product. The crude product was purified by column chromatography on silica gel (dichloromethane: methanol=10:1) to give a product of (4-aminoimidazo[1,5-*a*]quinoxalin-8-yl)2-(4-(trifluoromethoxy)phenyl)piperidin-1-yl ketone (30 mg, yield: 16.2%), which was a white solid.
LCMS (ESI) m/z: 455.5 [M+H]⁺
¹H NMR (400 MHz, DMSO-*d*₆) δ 9.19 (s, 1H), 8.32 (d, *J* = 1.7 Hz, 1H), 7.92 (s, 1H), 7.54 - 7.38 (m, 9H), 5.63 (d, *J* = 4.5 Hz, 1H), 3.06 - 2.79 (m, 2H), 2.45 (d, *J* = 14.4 Hz, 1H), 1.98 (ddd, *J* = 13.8, 9.5, 4.8 Hz, 1H), 1.70 - 1.55 (m, 3H), 1.39 (d, *J* = 13.0 Hz, 1H).

### Example 23

### Synthesis of (4-aminoimidazo[1,5-a]quinoxalin-8-yl) 2-(1-methylpyrazolo[3,4-b]pyridin-5-yl)piperidin-1-me thanone

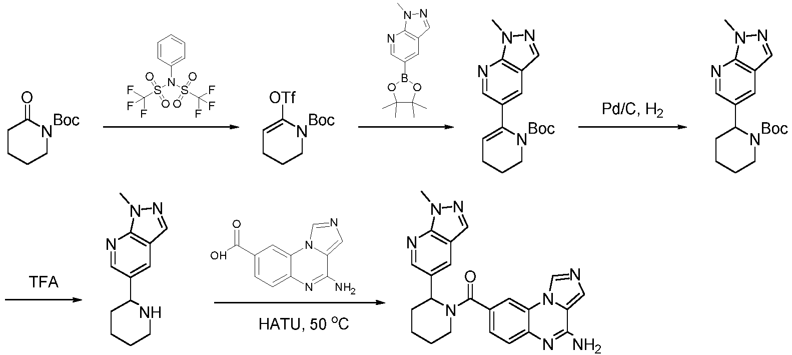

### Step I: Synthesis of tert-butyl 6-((trifluoromethylsulfonyl)oxy)-3,4-dihydropyridin-1(2H)-carboxylate

Under an N₂ atmosphere, lithium bistrimethylsilylamide (60 mL, 1.0 M, 50.25 mmol) was slowly added dropwise to a tetrahydrofuran (160 mL) solution of tert-butyl 2-oxopiperidine-1-carboxylate (10 g, 50.25 mmol), the mixture was stirred at -78°C to react for 1.5 h. Then 1,1,1-trifluoro-*N*-phenyl-*N*-((trifluoromethyl)sulfonyl) methanesulfonamide (29.5 g, 75.37 mmol) was slowly added at the temperature, the mixture was stirred at -78°C to react for 4 h, after the reaction was completed, the reaction solution was quenched with an ammonium chloride solution, poured into water (400 mL) and extracted with ethyl acetate (300 mL×3), with the organic phases combined and dried over anhydrous sodium sulfate and filtered and the filtrate was concentrated under reduced pressure to give a crude product. The crude product was purified by silica gel column chromatography (petroleum ether: ethyl acetate=5:1) to give tert-butyl 6-((trifluoromethylsulfonyl)oxy)-3,4-dihydropyridin-1(2*H*)-carboxylate (8.1 g, yield: 48.7%), which was a yellow oily liquid.

LCMS (ESI) m/z: 332.1 [M+H]⁺

### Step II: Synthesis of tert-butyl 6-(1-methylpyrazolo[3,4-b]pyridin-5-yl)-3,4-dihydropyridin-1(2H) carboxylate

Under an N₂ atmosphere, tert-butyl 6-((trifluoromethylsulfonyl)oxy)-3,4-dihydropyridin-1(2H)-carboxylate (300 mg, 0.906 mmol) was dissolved in a mixture of 1,4-dioxane and water (4:1, 6 mL). Subsequently, 1-methyl-5-(4,4,5,5-tetramethyl-1,3,2-dioxaborolane-2-yl)-1H-pyrazolo[3,4-b]pyridine (282 mg, 1.09 mmol), potassium carbonate (376 mg, 2.72 mmol), and [1,1'-bis(diphenylphosphine)ferrocene]dichloropalladium(II) (67.0 mg, 0.09 mmol) were added. The reaction mixture was stirred at 100°C for 16 hours and then poured into water(30 mL), extracted with ethyl acetate (20 mL×3), with the organic phases combined, dried over anhydrous sodium sulfate, and filtered to remove the anhydrous sodium sulfate, and the filtrate was concentrated to dryness in vacuum to give a crude product. The crude product was purified by silica gel column chromatography (petroleum ether:ethyl acetate=10:1) to give tert-butyl 6-(1-methylpyrazolo[3,4-*b*]pyridin-5-yl)-3,4-dihydropyridin-1(2H) carboxylate (250 mg, yield: 88.0%), which was a brown solid.
LCMS (ESI) m/z: 315.2 [M+H]⁺

### Step III: Synthesis of tert-butyl 2-(1-methyl-1H-pyrazolo[3,4-b]pyridin-5-yl)piperidin-1-carboxylate

Under an N₂ atmosphere, tert-butyl 6-(1-methylpyrazolo[3,4-b]pyridin-5-yl)-3,4-dihydropyridin-1(2H)-carboxylate (250 mg, 0.796 mmol) was dissolved in MeOH (5 mL). Pd/C (850 mg, 1.592 mmol) was then added, and the system was purged with hydrogen three times using balloons. The mixture was stirred at 25°C for 16 hours to complete the reaction. After LCMS showed that the reaction was completed, the mixture was filtered, and the filtrate was concentrated under reduced pressure to give a crude product tert-butyl 2-(1-methyl-1*H*-pyrazolo[3,4-*b*]pyridin-5-yl)piperidin-1-carboxylate (240 mg, crude product), which was directly used in the next step without purification.
LCMS (ESI) m/z: 317.2 [M-H]⁺

### Step IV: Synthesis of 1-methyl-5-piperidin-2-yl-1H-pyrazolo[3,4-b]pyridine

At room temperature, the compound tert-butyl 2-(1-methyl-1*H*-pyrazolo[3,4-*b*]pyridin-5-yl)piperidin-1-carbo xylate (240 mg, 0.759 mmol) was dissolved in hydrochloric acid/ethyl acetate (5 mL), the mixture was stirred at room temperature to react for 3h; after the reaction was completed, the mixture was concentrated under reduced pressure to give a crude product 1-methyl-5-piperidin-2-yl-1*H*-pyrazolo[3,4-*b*]pyridine (280 mg, crude product), which was direclty used in the next step without purification.
LCMS (ESI) m/z: 217.2 [M+H]⁺

### Step V: Synthesis of (4-aminoimidazo[1,5-a]quinoxalin-8-yl) 2-(1-methylpyrazolo[3,4-b]pyridin-5-yl)piperidin-1-methanone

At room temperature, 1-methyl-5-piperidin-2-yl-1*H*-pyrazolo[3,4-*b*]pyridine (100 mg, 0.461 mmol) was dissolved in DMAC (4 mL), 4-aminoimidazo[1,5-*a*]quinoxalin-8-carboxylic acid (127 mg, 0.552 mmol), HATU (212 mg, 0.552 mmol) and N,N-diisopropylethylamine (238 mg, 1.844 mmol) at 50°C for 16 h, the reaction solution was poured into water (20 mL), extracted with ethyl acetate (20 mL ×3), with the organic phases combined, dried over anhydrous sodium sulfate and filtered and the filtrate was concentrated under reduced pressure to obtain a crude product. The crude product was purified by chromatograpic column on silica gel (methylene chloride: methanol=10:1) to obtain a product of (4-aminoimidazo[1,5-*a*]quinoxalin-8-yl)2-(1-methylpyrazolo[3,4-*b*]pyridin-5-yl)piperi din-1-methanone (40 mg, yield:15.2%), which was a white solid.
LCMS (ESI) m/z: 427.2 [M+H]⁻
¹H NMR (400 MHz, DMSO-*d*₆) δ 9.22 (s, 1H), 8.58 (d, *J* = 2.1 Hz, 1H), 8.37 (s, 1H), 8.31 - 8.21 (m, 1H), 8.15 (s, 1H), 7.94 (s, 1H), 7.49 (d, *J* = 21.2 Hz, 4H), 5.80 (s, 1H), 4.08 (s, 3H), 2.74-2.55 (m, 2H), 2.11 - 1.94 (m, 1H), 1.80 - 1.43 (m, 4H), 1.36 - 1.21 (m, 1H).

### Example 24: Synthesis of (4-aminoimidazo[1,5-a]quinoxalin-8-yl)2-(quinolin-2-yl)piperidin-1-yl)methanon e

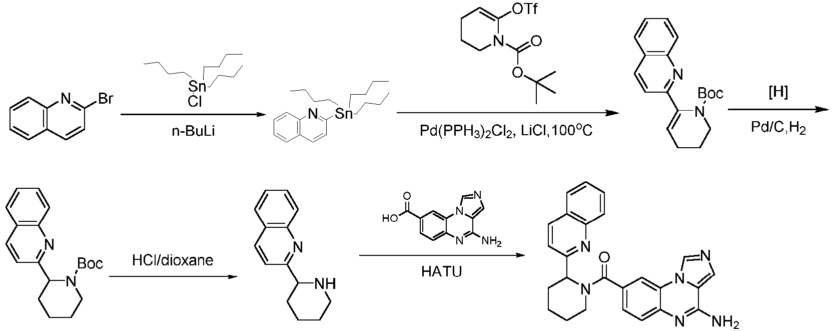

### Step I: Synthesis of 2-(tributylstannyl)quinoline

Under an N₂ atmopshere, 2-bromoquinoline (1 g, 4.81 mmol) was dissolved in tetrahydrofuran (20 mL), n-butyl lithium (3.6 mL, 1. 6 mol/L, 5.76 mmol) was slowly added dropwise to the system at -78°C, the system was stirred to react for 0.5 h at -78°C, then tributylstannane chloride (29.5 g, 75.37 mmol) was slowly added, and the system was stirred at -78°C for 2 h, after the reaction was completed, the reaction solution was quenched with an ammonium chloride solution, poured into water (400 mL), and extracted with ethyl acetate (300 mL×3), the organic phases were combined, dried over anhydrous sodium sulfate and filtered, and the filtrate was concentrated under reduced pressure to give a crude product 2-(tributylstannyl)quinoline (1.3 g, crude product), which was directly used in the next step without purification.
LCMS (ESI) m/z: 419.1 [M+H]⁺

### Step II: Synthesis of tert-butyl 6-(quinolin-2-yl)-3,4-dihydropyridin-1(2H)-carboxylate

Under an atmosphere of N₂, tert-butyl 6-((trifluoromethylsulfonyl)oxy)-3,4-dihydropyridin-1(2*H*)-carboxylate (300 mg, 0.906 mmol) was dissolved in toluene (10 mL), 2-(tributylstannyl)quinoline (600 mg, crude), lithium chloride (115 mg, 2.72 mmol), and bis-triphenylphosphine palladium dichloride(64.0 mg, 0.09 mmol) was added, and the mixture was reacted at 100°C for 16 hours. The mixyure was poured into water (50 mL), and extracted with ethyl acetate (40 mL ×3). The organic phases were combined and dried over anhydrous sodium sulfate, the anhydrous sodium sulfate was removed by filtration, and the filtrate was dried to obtain a crude product. The crude product was purified by silica gel column chromatography (petroleum ether:ethyl acetate=10:1) to give tert-butyl 6-(quinolin-2-yl)-3,4-dihydropyridin-1(2*H*)-carboxylate (200 mg, 71.2% yield), which was a brown solid.
LCMS (ESI) m/z: 311.2 [M+H]⁺

### Step III: Synthesis of tert-butyl 2-(quinolin-2-yl)piperidin-1-carboxylate

Under an N₂ atmosphere, tert-butyl 6-(quinolin-2-yl)-3,4-dihydropyridin-1(2*H*)-carboxylate (200 mg, 0.645 mmol) was dissolved in MeOH (5 mL), Pd/C (720 mg, 1.290 mmol) was added, and the system was purged with hydrogen three times using balloons. And the mixture was stirred to react at 40°C for 8 h. After LCMS showed that the reaction was completed, the mixture was filtered, and the filtrate was concentrated under reduced pressure to give tert-butyl 2-(quinolin-2-yl)piperidin-1-carboxylate (190 mg, crude product).
LCMS (ESI) m/z: 313.2 [M+H]⁺

### Step IV: Synthesis of 2-(piperidin-2-yl)quinoline

At room temperature, the compound tert-butyl 2-(quinolin-2-yl)piperidin-1-carboxylate (190 mg, 0.759 mmol) was dissolved in hydrochloric acid/ethyl acetate (5 mL) and the mixture was at room temperature to react for 3 h. After the reaction was completed, the mixture was concentrated under reduced pressure to give a crude 2-(piperidin-2-yl)quinoline (240 mg, crude product), which was directly used in the next step without purification.
LCMS (ESI) m/z: 213.2 [M+H]⁺

### Step V: Synthesis of (4-aminoimidazo[1,5-a]quinoxalin-8-yl)2-(quinolin-2-yl)piperidin-1-yl)methanone

At room temperature, 2-(piperidin-2-yl)quinoline (120 mg, 0.552 mmol) was dissolved in DMAC (4 mL), 4-aminoimidazo[1,5-*a*]quinoxalin-8-carboxylic acid (106 mg, 0.461 mmol), HATU (212 mg, 0.552 mmol) and *N,N*-diisopropylethylamine (238 mg, 1.844 mmol) were added, the mixture was stirred at 50°C for 16 h, after the reaction was completed, the reaction solution was poured into water (20 mL) and extracted with ethyl acetate (20 mL ×3), with the organic phases combined, dried over anhydrous sodium sulfate and filtered, and the filtrate was concentrated under reduced pressure to give a crude product. The crude product was purified by silica gel column chromatography (dichloromethane: methanol=10:1) to give a product (4-aminoimidazo[1,5-*a*]quinoxalin-8-yl)2-(quinolin-2-yl)piperidin-1-yl)methanone (10 mg, yield: 5.14%), which was a white solid.
LCMS (ESI) m/z: 423.2 [M+H]⁺
¹H NMR (400 MHz, DMSO-*d*₆) δ 9.22 (s, 1H), 8.62 - 8.31 (m, 2H), 8.26 - 7.93 (m, 3H), 7.90 - 7.21 (m, 7H), 6.05 (s, 1H), 2.10 - 1.91 (m, 1H), 1.80 - 1.26 (m, 6H).

### Example 25: Synthesis of (4-Amino-7-fluorimidazo[1,5-a]quinoxalin-8-yl)2-(quinolin-2-yl)piperidin-1-yl)m ethanone

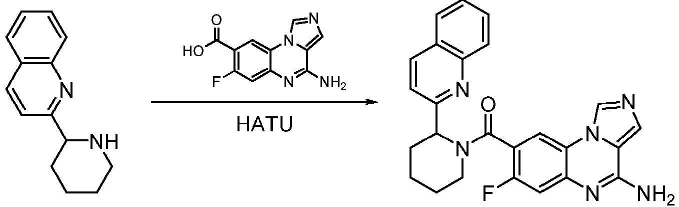

### Synthesis of (4-amino-7-fluorimidazo[1,5-a]quinoxalin-8-yl)2-(quinolin-2-yl)piperidin-1-yl)methanone

At room temperature, 2-(piperidin-2-yl)quinoline (120 mg, 0.552 mmol) was dissolved in DMAC (4 mL), 4-amino-7-fluoroimidazo[1,5-*a*]quinoxalin-8-carboxylic acid (114 mg, 0.461 mmol), HATU (212 mg, 0.552 mmol) and N,N-diisopropylethylamine (238 mg, 1.844 mmol) were added, the mixture was stirred at 50°C for 16 h, after the reaction was completed, the reaction solution was poured into water (20 mL) and extracted with ethyl acetate (20 mL ×3), with the organic phases combined, dried over anhydrous sodium sulfate and filtered, and the filtrate was concentrated under reduced pressure to obtain a crude product. The crude product was purified by column chromatography on silica gel (dichloromethane: methanol=10:1) to give a product of (4-amino-7-fluoroimidazo[1,5-*a*]quinoxalin-8-yl)2-(quinolin-2-yl)piperidin-1-yl)methanone (8 mg, yield: 3.94%), which was a white solid.
LCMS (ESI) m/z: 441.2 [M+H]⁺
¹H NMR (400 MHz, DMSO-*d*₆) δ 9.22 (s, 1H), 8.49 -8.28 (m, 2H), 8.12 -7.93 (m, 2H), 7.85 -7.71 (m, 1H), 7.69 -7.48 (m, 4H), 7.37 -7.23 (m, 1H), 6.12 (s, 1H), 5.19 -4.60 (m, 1H), 3.73 -3.48 (m, 1H), 2.89 (dd,*J* = 14.0 Hz, 1H), 2.69 (s, 1H), 2.09 -1.42 (m, 5H).

### Example 26 Synthesis of (4-aminoimidazo[1,5-a]quinoxalin-8-yl)(2-(benzothiazol-5-yl)piperidin-1-yl)metha none

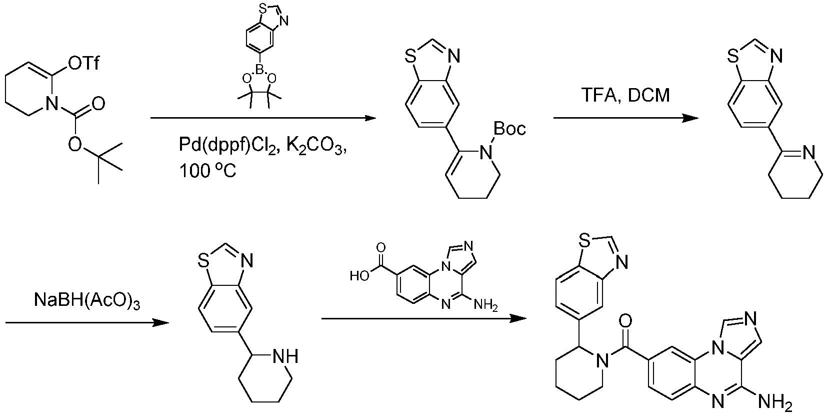

### Step I: Synthesis of tert-butyl 6-(benzothiazol-5-yl)-3,4-dihydropyridin-1(2H)-carboxylate

Under an N₂ atmosphere, tert-butyl 6-((trifluoromethylsulfonyl)oxy)-3,4-dihydropyridin-1(2*H*)-carboxylate (300 mg, 0.906 mmol) was dissolved in 1,4-dioxane and water (4:1,6 mL), 5-(4,4,5,5-tetramethyl-1,3,2-dioxaboran-2-yl)benzothiazole (284 mg, 1.09 mmol), potassium carbonate (376 mg, 2.72 mmol), and [1,1'-bis(diphenylphosphine)ferrocene]dichloropalladium (II) (67.0 mg, 0.09 mmol) were added to react at 100°C for 16 h. The system was poured into water (30 mL), extracted with ethyl acetate (20 mL×3), with the organic phases combined, dried over anhydrous sodium sulfate, and filtered to remove the anhydrous sodium sulfate, and the filtrate was concentrated to dryness in vacuum to obtain a crude product. The crude product was purified by column chromatography on silica gel (petroleum ether:ethyl acetate=10:1) to give tert-butyl 6-(benzothiazol-5-yl)-3,4-dihydropyridin-1(2*H*)-carboxylate (200 mg, yield: 69.8%), which was a brown solid.
LCMS (ESI) m/z: 317.2 [M+H]⁺

### Step II: Synthesis of 5-(3,4,5,6-tetrahydropyridin-2-yl)benzothiazole

At room temperature, tert-butyl 6-(benzothiazole-5-yl)-3,4-dihydropyridin-1(2*H*)-carboxylate (200 mg, 0.632 mmol) was dissolved in dichloromethane (2.5 mL), trifluoroacetic acid (0.5 mL) was added and the reaction solution was stirred at room temperature for 2 h. After the reaction was completed, the reaction solution was poured into water, a certain amount of a saturated sodium bicarbonate aqueous solution was added to adjust pH to 8 and the reaction solution was extracted with dichloromethane. The organic phases were combined, dried over anhydrous sodium sulfate and filtered, and the filtrate was concentrated under reduced pressure to give a crude product 5-(3,4,5,6-tetrahydropyridin-2-yl)benzothiazole (150 mg, crude product), which was directly used in the next step without purification.
LCMS (ESI) m/z: 217.2 [M-H]⁺

### Step III: Synthesis of 5-(piperidin-2-yl)benzothiazole

At room temperature, 5-(3,4,5,6-tetrahydropyridin-2-yl)benzothiazole (150 mg, 0.694 mmol) was dissolved in methanol, then sodium triacetylborohydride (284 mg, 1.314 mmol) was added and the reaction solution was stirred for 10 h at 50°C. After the reaction was completed, the reaction solution was concentrated under reduced pressure, poured into water and extracted with ethyl acetate, with the organic phases combined, dried over anhydrous sodium sulfate and filtered, and the filtrate was concentrated under reduced pressure to give a crude product 5-(piperidin-2-yl)benzothiazole (100 mg, crude product), which was directly used in the next step without purification.
LCMS (ESI) m/z: 219.2 [M+H]⁺

### Step IV: Synthesis of (4-aminoimidazo[1,5-a]quinoxalin-8-yl)(2-(benzothiazol-5-yl)piperidin-1-yl) methanone

At room temperature, 5-(piperidin-2-yl)benzothiazole (100 mg, 0.460 mmol) was dissolved in DMAC (4 mL), then 4-aminoimidazo[1,5-*a*]quinoxalin-8-carboxylic acid (127 mg, 0.552 mmol), 2-chloro-1-methylpyridine iodide (141 mg, 0.552 mmol) and *N,N*-diisopropylethylamine (238 mg, 1.844 mmol) were added successively and the mixture was stirred at 50°C for 16 h, after the reaction was completed, the reaction solution was poured into water (20 mL) and extracted with ethyl acetate (20 mL×3), with the organic phases combined, dried over anhydrous sodium sulfate and filtered, and the filtrate concentrated under reduced pressure to give a crude product. The crude product was purified by silica gel column chromatography (dichloromethane: methanol=10:1) to give a product (4-aminoimidazo[1,5-*a*]quinoxalin-8-yl)(2-(benzothiazol-5-yl)piperidin-1-yl)methanone (3 mg, yield: 1.53%), which was a pale yellow solid.
LCMS (ESI) m/z: 429.2 [M+H]⁺
¹H NMR (400 MHz, DMSO-*d*₆) δ 9.43 (s, 1H), 9.21 (s, 1H), 8.37 (d, *J =* 1.6 Hz, 1H), 8.22 (d, *J =* 8.4 Hz, 1H), 8.05 (s, 1H), 7.92 (s, 1H), 7.59 -7.35 (m, 5H), 5.78 (s, 1H), 2.94 (s, 1H), 2.60 (s, 1H), 2.10 -96 (m, 1H), 1.80 -1.41 (m, 5H)

### Example 27 Synthesis of

### (4-aminoimidazo[1,5-a]quinoxalin-8-yl)2-(7-fluoroquinolin-2-yl)piperidin-1-methanone

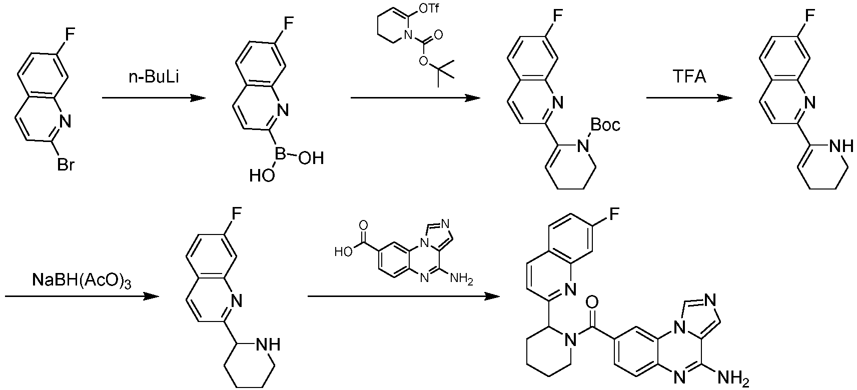

### Step I: Synthesis of 7-fluoroquinolin-2-boronic acid

At room temperature, 2-bromo-7-fluoroquinoline (1 g, 4.424 mmol) was dissolved in anhydrous tetrahydrofuran (20 mL). n-butyl lithium (3.5 mL, 8.848 mmol) was slowly added dropwise into the system at -78°C. After the mixture was stirred for 10 min, isopropyl pinacol borate (1.6 g, 8.848 mmol) was added to react while stirring at -78°C for 2 h. After the reaction was completed, after quenching with a saturated monium chloride solution (1 mL), the mixture was concentrated under reduced pressure to obtain a crude product. The crude product was washed with ethyl acetate and filtered and the filter cake was washed twice with ethyl acetate to give a white solid (0.5 g, crude product).
LCMS (ESI) m/z: 192.0 [M+H]⁺

### Step II: tert-butyl 6-(7-fluoroquinolin-2-yl)-3,4-dihydropyridin-1(2H)-carboxylate

At room temperature, 7-fluoroquinolin-2-boronic acid (346 mg, 1.811 mmol) and tert-butyl 6-((trifluoromethylsulfonyl)oxy)-3,4-dihydropyridin-1(2*H*)-carboxylate (300 mg, 0.906 mmol) were dissolved in a mixed solvent (10 mL, 4:1) of 1,4-dioxane and water, and [1,1'-bis(diphenylphosphine)ferrocene] palladium dichloride (68 mg, 0.091 mmol) and potassium carbonate (383 mg, 2.72 mmol) were added, and the system was purged with nitrogen three times using balloons and the mixture was reacted at 100°C for 14 h. After the reaction was completed, the reaction solution was poured into water and extracted with ethyl acetate (20 mL × 3),with the organic phases combined, dried over anhydrous sodium sulfate and filtered, and the filtrate concentrated under reduced pressure was purified by column chromatography (petroleum ether:ethyl acetate=1:1) to give tert-butyl 6-(7-fluoroquinoline-2-yl)-3,4-dihydropyridin-1(2*H*)-carboxylate (150 mg, yield: 50%).
LCMS (ESI) m/z: 329.2 [M+H]⁺

### Step III: Synthesis of 7-fluoro-2-(1,4,5,6-tetrahydropyridin-2-yl)quinoline

At room temperature, tert-butyl 6-(7-fluoroquinolin-2-yl)-3,4-dihydropyridin-1(2*H*)-carboxylate (150 mg, 0.457 mmol) was dissolved in dichloromethane (5 mL), trifluoroacetic acid (0.5 mL) was added and the reaction solution was stirred at room temperature for 2 h. After the reaction was completed, the reaction solution was poured into water, a certain amount of a saturated aqueous sodium bicarbonate solution was added to adjust pH to 8 and the mixture was extracted with dichloromethane, with organic phases combined, dried over anhydrous sodium sulfate and filtered, and the filtrate was concentrated under reduced pressure to give a crude product (100 mg, crude product), which was directly used in the next step without purification.
LCMS (ESI) m/z: 229 [M-H]⁺

### Step IV: Synthesis of 7-fluoro-2-(piperidin-2-yl)quinoline

At room temperature, 7-fluoro-2-(1,4,5,6-tetrahydropyridin-2-yl)quinoline (100 mg, 0.438 mmol) was dissolved in methanol, sodium triacetylborohydride (284 mg, 1.314 mmol) was added and the reaction solution was stirred for 10 h at 50°C. After the reaction was completed, the reaction solution was concentrated under reduced pressure, poured into water and extracted with ethyl acetate, with the organic phases combined, dried over anhydrous sodium sulfate and filtered, and the filtrate was concentrated under reduced pressure to obtain a crude product (100 mg), which was used directly in the next step without purification.
LCMS (ESI) m/z: 231.1 [M+H]⁺

### Step V: (4-aminoimidazo[1,5-a]quinoxalin-8-yl) 2-(7-fluoroquinolin-2-yl)piperidin-1-methanone

At room temperature, 7-fluoro-2-(piperidin-2-yl)quinoline (100 mg, 0.369 mmol) was dissolved in DMAC (5 mL), 4-aminoimidazo[1,5-*a*]quinoxalin-8-carboxylic acid (84 mg, 0.369 mmol), HATU (286 mg, 0.737 mmol) and N,N-diisopropylethylamine (146 mg, 1.11 mmol) were added, after the mixture was stirred at 25° for 2 h, the reaction solution was poured into water (20 mL) and extracted with ethyl acetate (15 mL×3), with the organic phases combined, dried over anhydrous sodium sulfate and filtered and the filtrate was concentrated under reduced pressure to give a crude product. The crude product was purified by silica gel column chromatography on (dichloromethane: methanol=10:1) to give a product (4-aminoimidazo[1,5-*a*]quinoxalin-8-yl)(2-(4-fluorophenoxy)phenyl)piperi din-1-yl)methanone (50 mg, yield: 28%).
LCMS (ESI) m/z: 441.2 [M+H]⁺

### Example 28 Synthesis of (4-amino-7-fluorimidazo[1,5-a]quinoxalin-8-yl)2-(quinoxalin-6-yl)piperidin-1-met hanone

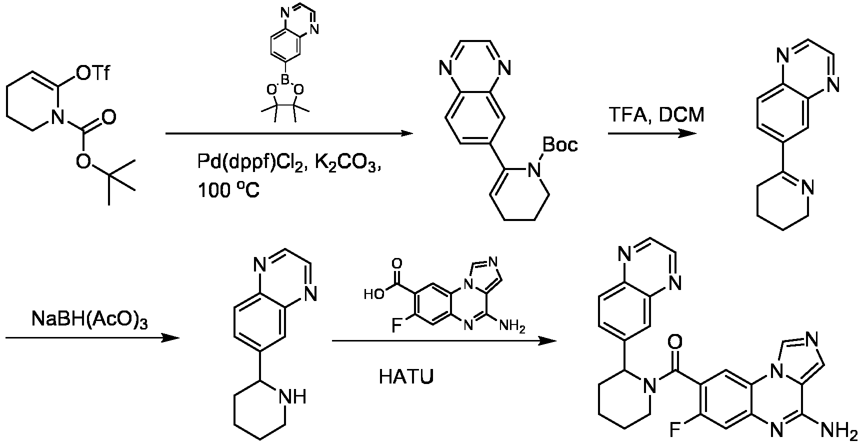

### Step I: Synthesis of tert-butyl 6-(quinoxalin-6-yl)-3,4-dihydropyridin-1(2H)-carboxylate

Under an N₂ atmosphere, tert-butyl 6-((trifluoromethylsulfonyl)oxy)-3,4-dihydropyridin-1(2*H*)-carboxylate (300 mg, 0.906 mmol) was dissolved in 1,4-dioxane and water (4:1, 6 mL), 6-(4,4,5,5-tetramethyl-1,3,2-dioxaboran-2-yl)quinoxaline (280 mg, 1.09 mmol), potassium carbonate (376 mg, 2.72 mmol), and [1,1'-bis(diphenylphosphine)ferrocene]dichloropalladium (II) (67.0 mg, 0.09 mmol) were added to react at 100°C for 16 h. The system was poured into water (30 mL), extracted with ethyl acetate (20 mL× 3), with the organic phases combined, dried over anhydrous sodium sulfate, and filtered to remove the anhydrous sodium sulfate, and the filtrate was concentrated to dryness in vacuum to obtain a crude product. The crude product was purified by column chromatography on silica gel (petroleum ether:ethyl acetate=10:1) to give tert-butyl ester 6-(quinoxalin-6-yl)-3,4-dihydropyridin-1(2*H*)-carboxylate (200 mg, yield: 70.9%), which was a brown solid.
LCMS (ESI) m/z: 312.2 [M+H]⁺

### Step II: Synthesis of 6-(3,4,5,6-tetrahydropyridin-2-yl)quinoxaline

At room temperature, tert-butyl 6-(quinoxalin-6-yl)-3,4-dihydropyridin-1(2*H*)-carboxylate (200 mg, 0.643 mmol) was dissolved in dichloromethane (2.5 mL), trifluoroacetic acid (0.5 mL) was added and the reaction solution was stirred at room temperature for 2 h. After the reaction was completed, the reaction solution was poured into water, a certain amount of a saturated sodium bicarbonate aqueous solution was added to adjust pH to 8 and the reaction solution was extracted with dichloromethane,with the organic phases combined, dried over anhydrous sodium sulfate and filtered, and the filtrate was concentrated under reduced pressure to give 6-(3,4,5,6-tetrahydropyridin-2-yl)quinoxaline (150 mg, crude product), which was directly used in the next step without purification.
LCMS (ESI) m/z: 212.2 [M+H]⁺

### Step III: Synthesis of 6-(piperidin-2-yl)quinoxaline

At room temperature, 6-(3,4,5,6-tetrahydropyridin-2-yl)quinoxaline (150 mg, 0.710 mmol) was dissolved in methanol, then sodium triacetylborohydride (307 mg, 1.314 mmol) was added and the reaction solution was stirred for 10 h at 50°C. After the reaction was completed, the reaction solution was concentrated under reduced pressure, poured into water and extracted with ethyl acetate, with the organic phases combined, dried over anhydrous sodium sulfate and filtered, and the filtrate was concentrated under reduced pressure to give 6-(piperidin-2-yl)quinoxaline (100 mg, crude product), which was directly used in the next step without purification.
LCMS (ESI) m/z: 214.2 [M+H]⁺

### Step IV: Synthesis of (4-amino-7-fluoroimidazo[1,5-a]quinoxalin-8-yl) 2-(quinoxalin-6-yl)piperidine-1-methanone

At room temperature, 6-(piperidin-2-yl)quinoxaline (100 mg, 0.469 mmol) was dissolved in DMAC (4 mL), 4-amino-7-fluimidazo[1,5-*a*]quinoxalin-8-carboxylic acid (136 mg, 0.552 mmol), 2-chloro-1-methylpyridine iodide (141 mg, 0.552 mmol) and *N,N*-diisopropylethylamine (238 mg, 1.844 mmol) were added, and the mixture was stirred at 50°C for 16 h, after the reaction was completed, the reaction solution was poured into water (20 mL) and extracted with ethyl acetate (20 mL ×3), with the organic phases combined, dried over anhydrous sodium sulfate and filtered, and the filtrate was concentrated under reduced pressure to give a crude product. The crude product was purified by silica gel column chromatography on (dichloromethane: methanol=10:1) to give a product (4-amino-7-fluimidazo[1,5-*a*]quinoxalin-8-yl)2-(quinoxalin-6-yl)piperidin-1-methanone (3 mg, yield: 1.53%).

¹H NMR (400 MHz, DMSO-*d*₆) δ 9.23 (s, 1H), 8.99 (d, J = 7.1 Hz, 2H), 8.44 (d, J = 6.5 Hz, 1H), 8.22 - 8.09 (m, 1H), 8.02 - 7.75 (m, 2H), 7.66 - 7.21 (m, 3H), 4.92 (d, J = 225.1 Hz, 1H), 3.56 (d, J = 13.7 Hz, 1H), 3.12 (s, 1H), 2.71 (d, J *=* 14.6 Hz, 1H), 2.03 (s, 1H), 1.83 - 1.38 (m, 4H).
LCMS (ESI) m/z: 424.2 [M+H]⁺

### Biochemical Evaluation

### I. Inhibitory Activity Test of Compounds on Tumor Cell Proliferation

### Test case 1: Inhibitory activity of compounds on the proliferation of HCT-116 MTAP(-/-)deleted cells

Materials and cells: HCT-116 MTAP(-/-)deleted cells were purchased from Botron Healthcare (China); RPMI-1640 medium, fetal bovine serum and penicillin-streptomycin were purchased from Thermo Fisher (United States); 384-well plates were purchased from PerkinElmer (United States); Cell-Titer Glo kit was purchased from Promega (United States).

Cell culture: HCT116 MTAP(-/-)deleted cells were cultured with RPMI 1640 medium containing 10% fetal bovine serum +1% penicillin-streptomycin at 37°C in 5% CO₂. Only cells in the logarithmic growth phase could be used for the test.

Test of inhibitory activity on cell proliferation: The Cell-Titer Glo kit was used to test the proliferation inhibitory activity of the compounds on HCT-116 MTAP(-/-)deleted cells. The cell concentration was adjusted, It was inoculated at 40 µL per well into a 384-well plate and incubated overnight at 37°C in 5% CO₂. 40 nL of the compound was added to each well to reach a final concentration of 0-10,000 nM (starting concentration 10,000 nM, 3-fold dilution, 10 points), and the cell plate with 0.1% DMSO content was incubated at 37°C and 5% CO₂ for 8 d. 40 µL of Cell-Titer Glo reagent was added to test cell viability. The test results were shown in Table 1.

### Test case 2: Test on proliferation inhibitory activity of compound on HCT-116 Wild Type cells

Materials and cells: HCT-116 wild-type cells were purchased from Botron Healthcare (China); RPMI-1640 medium, fetal bovine serum and penicillin-streptomycin were purchased from Thermo Fisher (United States); 384-well plates were purchased from PerkinElmer (United States); Cell-Titer Glo kit was purchased from Promega (United States).

Cell culture: HCT-116 wild-type cells were cultured with a RPMI-1640 medium containing 10% fetal bovine serum+1% penicillin-streptomycin at 37°C in 5% CO₂. Only cells in the logarithmic growth phase could be used for the test.

Test of cell proliferation activity: Cell-Titer Glo kit was used to test the proliferation inhibitory activity of the compound on HCT-116 wild-type cells. The cell concentration was adjusted, 40 µL per well was inoculated into a 384-well plate and incubated overnight at 37°C in 5% CO₂. 40 nL of the compound was added to each well to reach a final concentration of 0-10,000 nM (starting concentration 10,000 nM, 2-fold dilution, 10 points), and the cell plate with 0.1% DMSO contentwas incubated at 37°C and 5% CO₂ for 8 d. 40 µL of Cell-Titer Glo reagent was added to test cell viability. The test results were shown in Table 1.

Table 1 below showed the inhibitory activity of Example compound on proliferation of HCT116 MTAP(-/-)deleted cells and HCT116 wild type cells.

**Table 1**

| Compound | IC₅₀ (nM) | |
|---|---|---|
| | HCT-116 MTAP(-/-)deleted | HCT-116 wild type |
| Example 1 | 3885 | 10806 |
| Example 2 | 106.1 | 7509.5 |
| Example 2A | >10000 | >10000 |
| Example 2B | 160 | 7930 |
| Example 3 | 4750.7 | >20000 |
| Example 4 | 18.5 | 1082 |
| Example 4A | 6.5 | 511.8 |
| Example 4B | 2779 | 6516.8 |
| Example 5 | 954 | >10000 |
| Example 6 | 28.76 | 2721.6 |
| Example 7 | 24.3 | 1686.9 |
| Example 8 | 395.8 | >10000 |
| Example 9 | >3000 | >10000 |
| Example 10 | 794.9 | >10000 |
| Example 11 | 16.9 | 3184.4 |
| Example 12 | 3.2 | 91.1 |
| Example 13 | 458.2 | 4182 |
| Example 13A | 516.2 | >10000 |
| Example 13B | >3000 | >10000 |
| Example 14 | 671.4 | >10000 |
| Example 15 | 932.8 | >10000 |
| Example 16 | 1300 | >10000 |
| Example 17 | >3000 | >10000 |
| Example 18 | 1399.6 | >10000 |
| Example 19 | 472.4 | 6383 |
| Example 20 | 83.3 | >10000 |
| Example 21 | 80.4 | >10000 |
| Example 22 | 45.2 | 8128 |
| Example 23 | 741.3 | >10000 |
| Example 24 | 242.5 | >10000 |

Although preferred embodiments have been described hereinabove, it will be apparent to those skilled in the art that modifications may be made without departing from the present invention. Such modifications are considered to be possible variants encompassed within the scope of the present invention.

## Claims

1. A compound, a pharmaceutically acceptable salt, an ester, a prodrug, a stereoisomer or an isotopic derivative thereof, having the structure of formula (I) below: 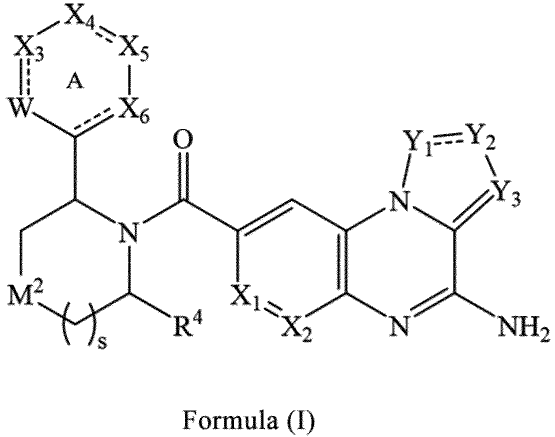
wherein W represents N or CR^{W};
wherein X₃ represents N or CR^{X3}; X₄ represents N or CR^{X4}; X₅ represents N or CR^{X5}; and X₆ represents N or CR^{X6};
wherein, when X₃ represents CR^{X3}, R^{X3} represents hydrogen, deuterium, C₁-C₆ alkyl, deuterided C₁-C₆ alkyl, C₂-C₆ alkenyl, C₂-C₆ alkynyl, halogen, -OR^{a}, -SR^{a}, -S(O)₂R^{a}, -S(O)R^{a}, -P(O) R^{a}R^{b}, -CN, -SF₅, -NR^{a}R^{b}, C₁-C₆ haloalkyl, C₁-C₆ haloalkoxy, hydroxy(C₁-C₆ alkyl) or 0-4 substituents selected from: deuteride, halogen, C₁-C₆ alkyl, C₂-C₆ alkenyl, C₂-C₆ alkynyl, C₃-C₁₀ cycloalkyl, -OR^{a}, oxo, hydroxy(C₁-C₆ alkyl), NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, OC(O)NR^{a}R^{b}, -NR^{a}COR^{b} or -CONR^{a}R^{b} substituted C₃-C₁₀ cycloalkyl, C₆-C₁₀ cycloalkenyl, 4- to 10-membered heterocycloalkyl, 6- to 10-membered heterocycloalkenyl, C₆-C₁₀ aryl, 5- to 10-membered heteroaryl;
wherein, when X₄ represents CR^{X4}, R^{X4} represents hydrogen, deuterium, C₁-C₆ alkyl, deuterided C₁-C₆ alkyl, C₂-C₆ alkenyl, C₂-C₆ alkynyl, halogen, -OR^{a}, -SR^{a}, -S(O)₂R^{a}, -S(O)R^{a}, -P(O) R^{a}R^{b}, -CN, -SF₅, -NR^{a}R^{b}, C₁-C₆ haloalkyl, C₁-C₆ haloalkoxy, hydroxy(C₁-C₆ alkyl) or 0-4 substituents selected from: deuterided, halogen, C₁-C₆ alkyl, C₂-C₆ alkenyl, C₂-C₆ alkynyl, C₃-C₆ cycloalkyl, -OR^{a}, oxo, hydroxy (C₁-C₆ alkyl), NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, OC(O)NR^{a}R^{b}, -NR^{a}COR^{b} or -CONR^{a}R^{b} substituted C₃-C₁₀ cycloalkyl, C₆-C₁₀ cycloalkenyl, 4- to 10-membered heterocycloalkyl, 6- to 10-membered heterocycloalkenyl, C₆-C₁₀ aryl, 5- to 10-membered heteroaryl;
wherein, when X₅ represents CR^{X5}, R^{X5} represents hydrogen, deuterium, C₁-C₆ alkyl, deuterided C₁-C₆ alkyl, C₂-C₆ alkenyl, C₂-C₆ alkynyl, halogen, -OR^{a}, -SR^{a}, -S(O)₂R^{a}, -S(O)R^{a}, -P(O) R^{a}R^{b}, -CN, -SF₅, -NR^{a}R^{b}, C₁-C₆ haloalkyl, C₁-C₆ haloalkoxy, hydroxy(C₁-C₆ alkyl) or 0-4 substituents selected from: deuterium, halogen, C₁-C₆ alkyl, C₂-C₆ alkenyl, C₂-C₆ alkynyl, C₃-C₁₀ cycloalkyl, -OR^{a}, oxo, hydroxy(C₁-C₆ alkyl), NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, OC(O)NR^{a}R^{b}, -NR^{a}COR^{b} or -CONR^{a}R^{b} substituted C₃-C₁₀ cycloalkyl, C₆-C₁₀ cycloalkenyl, 4- to 10-membered heterocycloalkyl, 6- to 10-membered heterocycloalkenyl, C₆-C₁₀ aryl, 5- to 10-membered heteroaryl;
wherein, when X₆ represents CR^{X6}, R^{X6} represents hydrogen, deuterium, C₁-C₆ alkyl, deuterated C₁-C₆ alkyl, C₂-C₆ alkenyl, C₂-C₆ alkynyl, halogen, -OR^{a}, -SR^{a}, -S(O)₂R^{a}, -S(O)R^{a}, -P(O) R^{a}R^{b}, -CN, -SF₅, -NR^{a}R^{b}, C₁-C₆ haloalkyl, C₁-C₆ haloalkoxy, hydroxy(C₁-C₆ alkyl) or 0-4 substituents selected from: deuterium, halogen, C₁-C₆ alkyl, C₂-C₆ alkenyl, C₂-C₆ alkynyl, C₃-C₁₀ cycloalkyl, -OR^{a}, oxo, hydroxy (C₁-C₆ alkyl), NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, OC(O)NR^{a}R^{b}, -NR^{a}COR^{b} or -CONR^{a}R^{b} substituted C₃-C₁₀ cycloalkyl, C₆-C₁₀ cycloalkenyl, 4- to 10-membered heterocycloalkyl, 6- to 10-membered heterocycloalkenyl, C₆-C₁₀ aryl, 5- to 10-membered heteroaryl;
wherein ring A may also be optionally fused with a 5- to 6-membered saturated or unsaturated ring at the chemical bond between X₃ and X₄, which may contain 0-3 heteroatoms selected from O, N and S;
wherein ring A may also be optionally fused with a 5- to 6-membered saturated or unsaturated ring at the chemical bond between X₄ and X₅, which may contain 0-3 heteroatoms selected from O, N and S;
wherein ring A may also be optionally fused with a 5- to 6-membered saturated or unsaturated ring at the chemical bond between X₅ and X₆, which may contain 0-3 heteroatoms selected from O, N and S;
wherein, when W represents CR^{W}, R^{W} is selected from hydrogen, deuterium, C₁-C₆ alkyl, deuterated C₁-C₆ alkyl, halogen, -OR^{a}, -SR^{a}, -S(O)₂R^{a}, -S(O)R^{a}, -CN, -SF₅, -NR^{a}R^{b}, C₁-C₆ haloalkyl, hydroxy(C₁-C₆ alkyl), and C₁-C₆ haloalkoxy;
wherein X₁ represents N or CR^{X1};
whereinX₂ represents N or CR^{X2};
wherein Y₁ represents CR^{Y1}R^{Y1'}, NR^{Y1}, O, S, Se;
whereinY₂ represents CR^{Y2}R^{Y2'}, NR^{Y2}, O, S, Se;
whereinY₃ represents CR^{Y3}R^{Y3'}, NR^{Y3}, O, S, Se;
wherein R^{X1} and R^{X2} each independently represent hydrogen, deuterium, C₁-C₆ alkyl, deuterated C₁-C₆ alkyl, C₁-C₆ haloalkyl, C₁-C₆ haloalkoxy, hydroxy(C₁-C₆ alkyl), C₂-C₆ alkenyl, C₂-C₆ alkynyl, -OR^{a}, -SR^{a}, -S(O)₂R^{a}, -S(O)R^{a}, -CN, -OC(O)R^{a}, -OCONR^{a}R^{b}, halogen, -SO₃R^{a}, -NR^{a}R^{b} and -SF₅;
wherein R^{Y1}, R^{Y1'}, R^{Y2}, R^{Y2'}, R^{Y3}, and R^{Y3'} each independently represent absent, hydrogen, deuterium, C₁-C₆ alkyl, deuterated C₁-C₆ alkyl, C₁-C₆ haloalkyl, C₁-C₆ haloalkoxy, hydroxy(C₁-C₆ alkyl), C₂-C₆ alkenyl, C₂-C₆ alkynyl, C₃-C₆ cycloalkyl, -OR^{a}, -SR^{a}, -S(O)₂R^{a}, -S(O)R^{a}, -CN, -OC(O)R^{a}, -OCONR^{a}R^{b}, halogen, -SO₃R^{a}, -NR^{a}R^{b}, and -SF₅;
Wherein M² represents CR^{M1}R^{M2}, S, O and NR^{M1};
Wherein R^{M1} and R^{M2} each independently represent hydrogen, deuterium, C₁-C₆ alkyl, halogen, or R^{M1} and R^{M2}, together with the atom to which they are attached, form a 3- to 14-membered saturated or unsaturated ring which may optionally contain 0-2 heteroatoms selected from O, S, N;
wherein R⁴ represents hydrogen, C₁-C₆ alkyl, deuterated C₁-C₆ alkyl, C₃-C₆ cycloalkyl or halogen;
wherein -̅ -̅ -̅ represents a single bond or a double bond;
wherein s represents an integer of 0 to 3;
wherein R^{a} and R^{b} each independently represent hydrogen, deuterium, halogen, C₁-C₆ alkyl, C₃-C₆ cycloalkyl, halogenated (C₁-C₆ alkyl), or R^{a} and R^{b}, together with the atom to which they are attached, form a 3- to 14-membered saturated or unsaturated ring which may optionally contain 0-2 heteroatoms selected from O, S, N.

2. The compound, and the pharmaceutically acceptable salt, ester, prodrug, stereoisomer or isotopic derivative thereof according to claim 1, having the structure of formula (II) below: 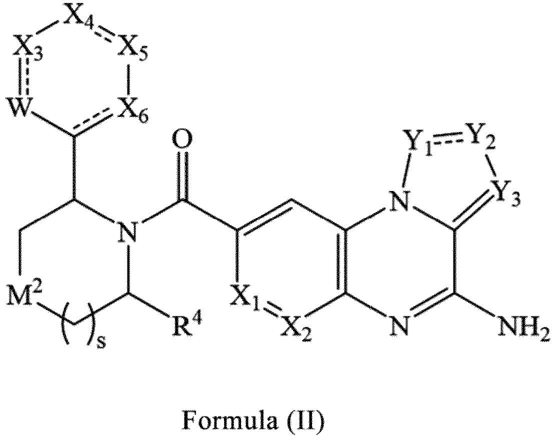
where W represents N or CR^{W};
where X₃ represents N or CR^{X3}; X₄ represents N or CR^{X4}; X₅ represents N or CR^{X5}; and X₆ represents N or CR^{X6},
wherein, when X₃ represents CR^{X3}, R^{X3} represents hydrogen, deuterium, C₁-C₆ alkyl, deuterated C₁-C₆ alkyl, C₂-C₆ alkenyl, C₂-C₆ alkynyl, halogen, -OR^{a}, -SR^{a}, -S(O)₂R^{a}, -S(O)R^{a}, -P(O) R^{a}R^{b}, -CN, -SF₅, -NR^{a}R^{b}, C₁-C₆ haloalkyl, C₁-C₆ haloalkoxy, hydroxy(C₁-C₆ alkyl) or 0-4 substituents selected from: deuteride, halogen, C₁-C₆ alkyl, C₂-C₆ alkenyl, C₂-C₆ alkynyl, C₃-C₁₀ cycloalkyl, -OR^{a}, oxo, hydroxy(C₁-C₆ alkyl), NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, OC(O)NR^{a}R^{b}, -NR^{a}COR^{b} or -CONR^{a}R^{b} substituted C₃-C₁₀ cycloalkyl, C₆-C₁₀ cycloalkenyl, 4- to 10-membered heterocycloalkyl, 6- to 10-membered heterocycloalkenyl, C₆-C₁₀ aryl, 5- to 10-membered heteroaryl;
wherein, when X₄ represents CR^{X4}, R^{X4} represents hydrogen, deuterium, C₁-C₆ alkyl, deuterated C₁-C₆ alkyl, C₂-C₆ alkenyl, C₂-C₆ alkynyl, halogen, -OR^{a}, -SR^{a}, -S(O)₂R^{a}, -S(O)R^{a}, -P(O) R^{a}R^{b}, -CN, -SF₅, -NR^{a}R^{b}, C₁-C₆ haloalkyl, C₁-C₆ haloalkoxy, hydroxy(C₁-C₆ alkyl) or 0-4 substituents selected from: deuteride, halogen, C₁-C₆ alkyl, C₂-C₆ alkenyl, C₂-C₆ alkynyl, C₃-C₆ cycloalkyl, -OR^{a}, oxo, hydroxy (C₁-C₆ alkyl), NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, OC(O)NR^{a}R^{b}, -NR^{a}COR^{b} or -CONR^{a}R^{b} substituted C₃-C₁₀ cycloalkyl, C₆-C₁₀ cycloalkenyl, 4- to 10-membered heterocycloalkyl, 6- to 10-membered heterocycloalkenyl, C₆-C₁₀ aryl, 5- to 10-membered heteroaryl;
wherein, when X₅ represents CR^{X5}, R^{X5} represents hydrogen, deuterium, C₁-C₆ alkyl, deuterated C₁-C₆ alkyl, C₂-C₆ alkenyl, C₂-C₆ alkynyl, halogen, -OR^{a}, -SR^{a}, -S(O)₂R^{a}, -S(O)R^{a}, -P(O) R^{a}R^{b}, -CN, -SF₅, -NR^{a}R^{b}, C₁-C₆ haloalkyl, C₁-C₆ haloalkoxy, hydroxy(C₁-C₆ alkyl) or 0-4 substituents selected from: deuteride, halogen, C₁-C₆ alkyl, C₂-C₆ alkenyl, C₂-C₆ alkynyl, C₃-C₁₀ cycloalkyl, -OR^{a}, oxo, hydroxy(C₁-C₆ alkyl), NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, OC(O)NR^{a}R^{b}, -NR^{a}COR^{b} or -CONR^{a}R^{b} substituted C₃-C₁₀ cycloalkyl, C₆-C₁₀ cycloalkenyl, 4- to 10-membered heterocycloalkyl, 6- to 10-membered heterocycloalkenyl, C₆-C₁₀ aryl, 5- to 10-membered heteroaryl;
wherein, when X₆ represents CR^{X6}, R^{X6} represents hydrogen, deuterium, C₁-C₆ alkyl, deuterated C₁-C₆ alkyl, C₂-C₆ alkenyl, C₂-C₆ alkynyl, halogen, -OR^{a}, -SR^{a}, -S(O)₂R^{a}, -S(O)R^{a}, -P(O) R^{a}R^{b}, -CN, -SF5, -NR^{a}R^{b}, C₁-C₆ haloalkyl, C₁-C₆ haloalkoxy, hydroxy(C₁-C₆ alkyl) or 0-4 substituents selected from: deuteride, halogen, C₁-C₆ alkyl, C₂-C₆ alkenyl, C₂-C₆ alkynyl, C₃-C₁₀ cycloalkyl, -OR^{a}, oxo, hydroxy(C₁-C₆ alkyl), NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, OC(O)NR^{a}R^{b}, -NR^{a}COR^{b} or -CONR^{a}R^{b} substituted C₃-C₁₀ cycloalkyl, C₆-C₁₀ cycloalkenyl, 4- to 10-membered heterocycloalkyl, 6- to 10-membered heterocycloalkenyl, C₆-C₁₀ aryl, 5- to 10-membered heteroaryl;
wherein, when W represents CR^{W}, R^{W} is selected from hydrogen, deuterium, C₁-C₆ alkyl, deuterated C₁-C₆ alkyl, halogen, -OR^{a}, -SR^{a}, -S(O)₂R^{a}, -S(O)R^{a}, -CN, -SF₅, -NR^{a}R^{b}, C₁-C₆ haloalkyl, hydroxy(C₁-C₆ alkyl), and C₁-C₆ haloalkoxy;
wherein X₁ represents N or CR^{X1};
whereinX₂ represents N or CR^{X2};
wherein Y₁ represents CR^{Y1}R^{Y1'}, NR^{Y1}, O, S, Se;
whereinY₂ represents CR^{Y2}R^{Y2'}, NR^{Y2}, O, S, Se;
wherein Y₃ represents CR^{Y3}R^{Y3'}, NR^{Y3}, O, S, Se;
wherein R^{X1} and R^{x2} each independently represent hydrogen, deuterium, C₁-C₆ alkyl, deuterated C₁-C₆ alkyl, C₁-C₆ haloalkyl, C₁-C₆ haloalkoxy, hydroxy(C₁-C₆ alkyl), C₂-C₆ alkenyl, C₂-C₆ alkynyl, -OR^{a}, -SR^{a}, -S(O)₂R^{a}, -S(O)R^{a}, -CN, -OC(O)R^{a}, -OCONR^{a}R^{b}, halogen, -SO₃R^{a}, -NR^{a}R^{b} and -SF₅;
wherein R^{Y1}, R^{Y1'}, R^{Y2}, R^{Y2'}, R^{Y3}, and R^{Y3'} each independently represent absent, hydrogen, deuterium, C₁-C₆ alkyl, deuterated C₁-C₆ alkyl, C₁-C₆ haloalkyl, C₁-C₆ haloalkoxy, hydroxy(C₁-C₆ alkyl), C₂-C₆ alkenyl, C₂-C₆ alkynyl, C₃-C₆ cycloalkyl, -OR^{a}, -SR^{a}, -S(O)₂R^{a}, -S(O)R^{a}, -CN, -OC(O)R^{a}, -OCONR^{a}R^{b}, halogen, -SO₃R^{a}, -NR^{a}R^{b}, and -SF₅;
wherein M² represents CR^{M1}R^{M2}, S, O and NR^{M1};
wherein R^{M1} and R^{M2} each independently represent hydrogen, deuterium, C₁-C₆ alkyl, halogen, or R^{M1} and R^{M2}, together with the atom to which they are attached, form a 3- to 14-membered saturated or unsaturated ring which may optionally contain 0-2 heteroatoms selected from O, S, N;
wherein R⁴ represents hydrogen, C₁-C₆ alkyl, deuterated C₁-C₆ alkyl, C₃-C₆ cycloalkyl or halogen;
wherein-̅ -̅ -̅ represents a single bond or a double bond;
wherein s represents an integer of 0 to 3;
wherein R^{a} and R^{b} each independently represent hydrogen, deuterium, halogen, C₁-C₆ alkyl, C₃-C₆ cycloalkyl, C₁-C₆ haloalkyl, or R^{a} and R^{b}, together with the atom to which they are attached, form a 3- to 14-membered saturated or unsaturated ring which may optionally contain 0-2 heteroatoms selected from O, S, N.

3. The compound, and the pharmaceutically acceptable salt, ester, prodrug, stereoisomer or isotopic derivative thereof according to claim 1 or 2, wherein X₁ represents CR^{X1} or N, and wherein R^{X1} represents hydrogen, deuterium, halogen, -CN, C₁-C₆ alkyl, deuterated C₁-C₆ alkyl, C₁-C₆ alkoxy, and C₁-C₆ haloalkyl.

4. The compound, and the pharmaceutically acceptable salt, ester, prodrug, stereoisomer or isotopic derivative thereof according to any one of claims 1 to 3, wherein X² represents CH, CD.

5. The compound, and the pharmaceutically acceptable salt, ester, prodrug, stereoisomer or isotopic derivative thereof according to any one of claims 1 to 4, wherein X₃ represents CH, CD or N.

6. The compound,and the pharmaceutically acceptable salt, ester, prodrug, stereoisomer or isotopic derivative thereof according to any one of claims 1 to 5, wherein X₄ represents CR^{X4} or N, wherein R^{X4} represents hydrogen, deuterium, C₁-C₆ alkyl, deuterated C₁-C₆ alkyl, C₁-C₆ alkoxy, C₁-C₆ haloalkyl, C₁-C₆ haloalkoxy, halogen, SF₅, SO₃R^{a}, -SR^{a}, -P(O)R^{a}R^{b} or cyano or substituents selected from 0 to 4: deuteride, halogen, C₁-C₆ alkyl, C₂-C₆ alkenyl, C₂-C₆ alkynyl, C₃-C₆ cycloalkyl, -OR^{a}, oxo, hydroxy(C₁-C₆ alkyl), NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, OC(O)NR^{a}R^{b}, -NR^{a}COR^{b} or -CONR^{a}R^{b} substituted C₃-C₁₀ cycloalkyl, C₆-C₁₀ cycloalkenyl, 4-10 membered heterocycloalkyl, 6- to 10-membered heterocycloalkenyl, C₆-C₁₀ aryl, 5- to 10-membered heteroaryl.

7. The compound, and the pharmaceutically acceptable salt, ester, prodrug, stereoisomer or isotopic derivative thereof according to any one of claims 1 to 6, wherein X₅ represents CR^{X5} or N, and wherein R^{X5} represents hydrogen, deuterium, C₁-C₆ alkyl, C₁-C₆ alkoxy, C₁-C₆ haloalkyl, C₁-C₆ haloalkoxy, halogen, SF₅, or cyano.

8. The compound, and the pharmaceutically acceptable salt, ester, prodrug, stereoisomer or isotopic derivative thereof according to any one of claims 1 to 7, wherein X₆ represents CH, CD or N.

9. The compound, and the pharmaceutically acceptable salt, ester, prodrug, stereoisomer or isotopic derivative thereof according to any one of claims 1 to 8, wherein the chemical bond between Y₁ and Y₂ is a double bond.

10. The compound, and the pharmaceutically acceptable salt, ester, prodrug, stereoisomer or isotopic derivative thereof according to claim 9, wherein Y₁ represents CR^{Y1}, wherein R^{Y1} represents hydrogen, deuterium, C₁-C₆ alkyl, deuterated C₁-C₆ alkyl, C₃-C₆ cycloalkyl, C₁-C₆ alkoxyl, C₁-C₆ haloalkyl, C₁-C₆ haloalkoxy, halogen, SF₅, -S(O)₂CH₃ or cyano.

11. The compound, and the pharmaceutically acceptable salt, ester, prodrug, stereoisomer or isotopic derivative thereof according to claim 8 or 9, wherein Y₂ represents N.

12. The compound, and the pharmaceutically acceptable salt, ester, prodrug, stereoisomer or isotopic derivative thereof according to any one of claims 1 to 11, wherein Y₃ represents CH or CD.

13. The compound, and the pharmaceutically acceptable salt, ester, prodrug, stereoisomer or isotopic derivative thereof according to any one of claims 1 to 12, wherein R⁴ represents hydrogen or C₁-C₆ alkyl or deuterated C₁-C₆ alkyl.

14. The compound, and the pharmaceutically acceptable salt, ester, prodrug, stereoisomer or isotopic derivative thereof according to any one of claims 1 to 13, wherein M² represents O, NH, S.

15. The compound, and the pharmaceutically acceptable salt, ester, prodrug, stereoisomer or isotopic derivative thereof according to any one of claims 1 to 14, wherein M² represents CR^{M1}R^{M2}; wherein R^{M1} and R^{M2} each independently represent hydrogen, deuterium, C₁-C₆ alkyl, halogen, or R^{M1} and R^{M2}, together with the atom to which they are attached, form a 3- to 14-membered saturated or unsaturated ring.

16. The compound, and the pharmaceutically acceptable salt, ester, prodrug, stereoisomer or isotopic derivative thereof according to any one of claims 1 to 15, selected from any of the following compound structures:

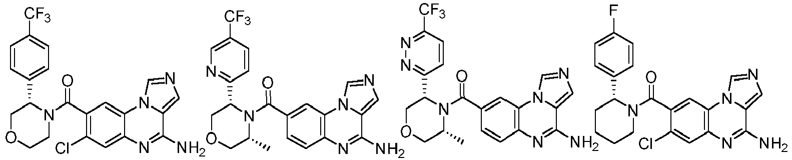

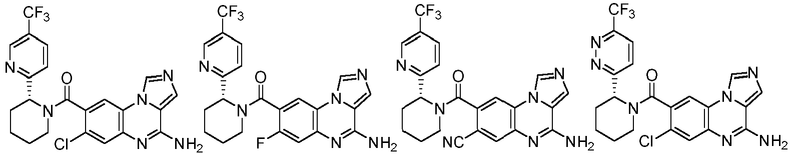

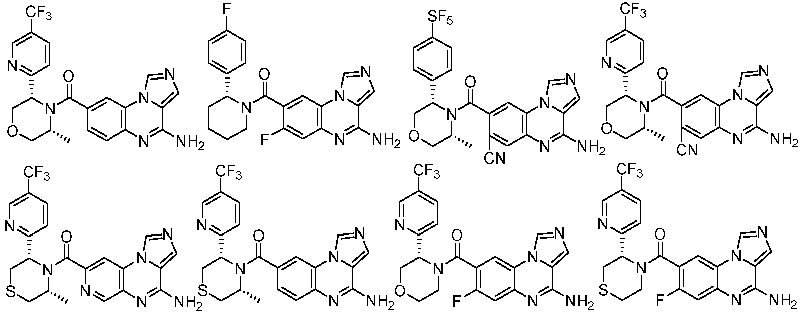

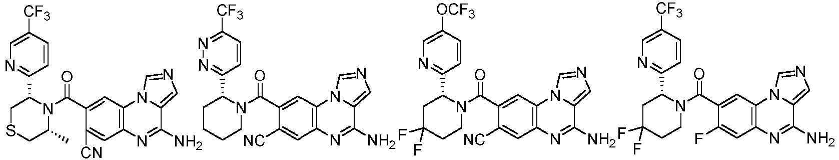

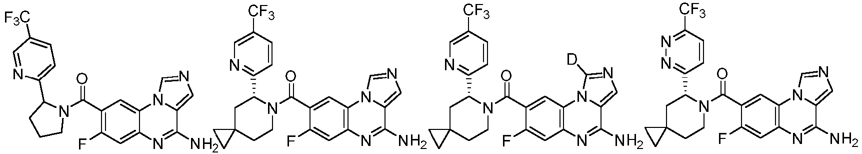

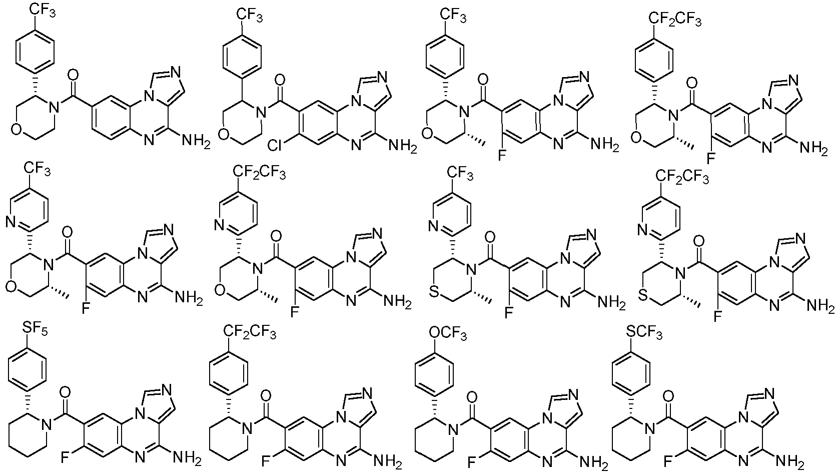

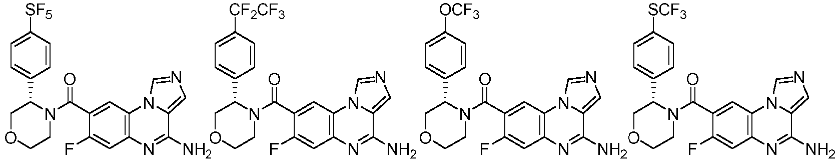

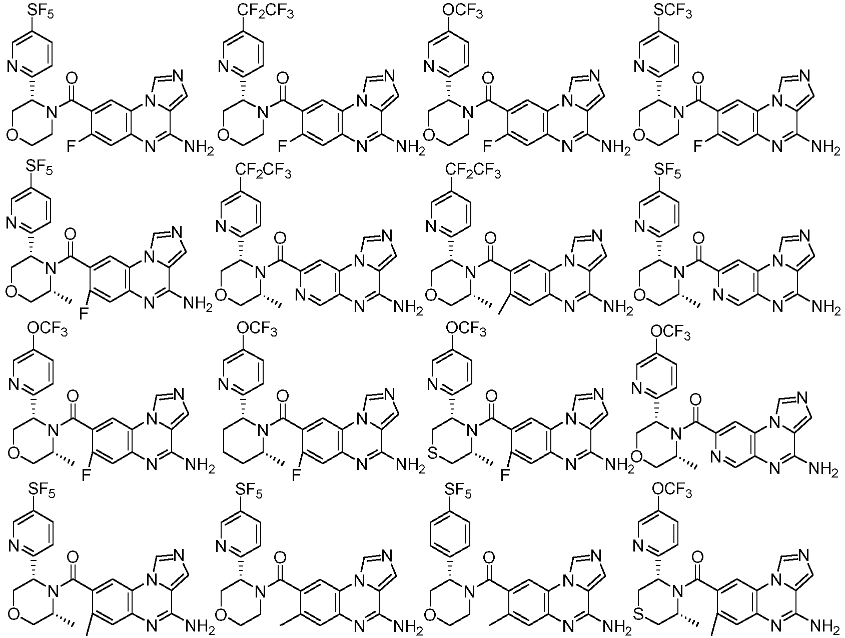

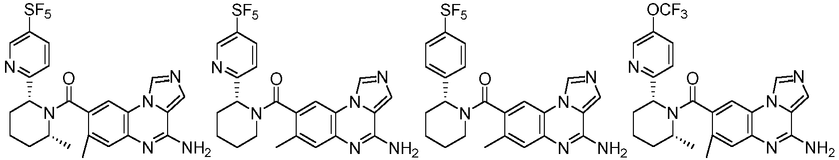

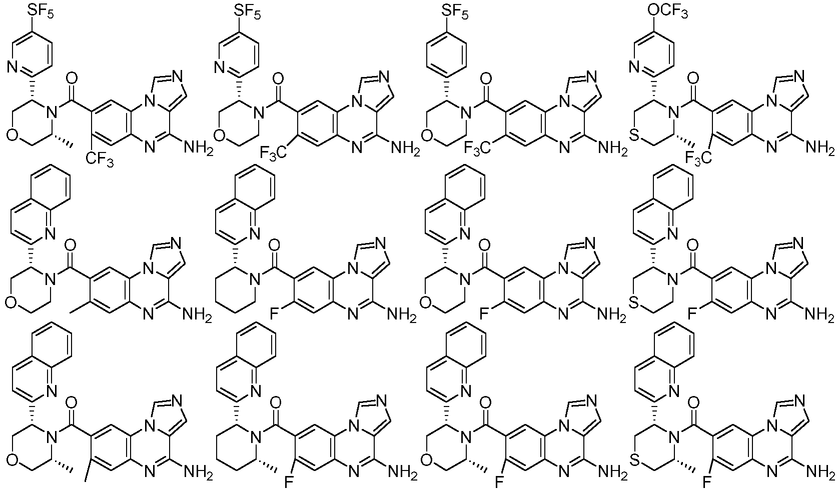

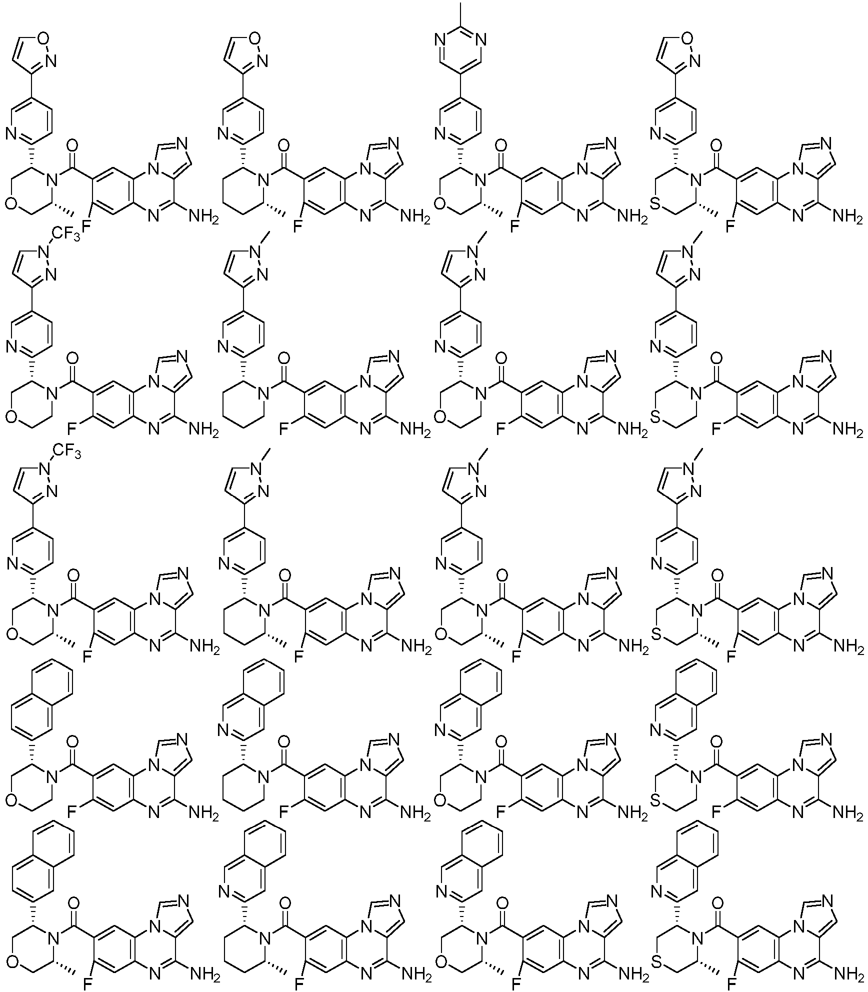

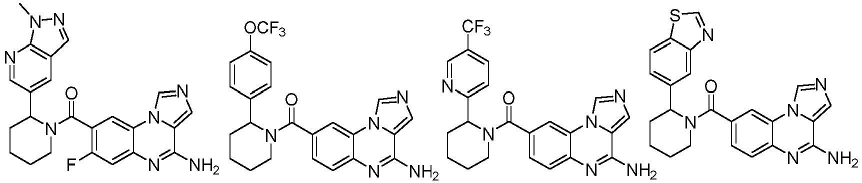

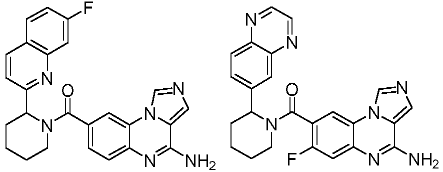

17. An isotopic derivative of the compound according to any one of claims 1-16, wherein at least one or more hydrogen atoms contained in the isotopic derivative are deuterium atoms.

18. A pharmaceutical composition, comprising the compound, and the pharmaceutically acceptable salt, ester, prodrug, stereoisomer or isotopic derivative according to any one of claims 1 to 17 and a pharmaceutically acceptable carrier.

19. The pharmaceutical composition according to claim 18, further comprising a second active substance, wherein the second active substance is an anti-tumor drug comprising one or more of chemotherapeutic drugs, targeted tumor therapy drugs and tumor therapy antibody drugs.

20. The compound, and the pharmaceutically acceptable salt, ester, prodrug, stereoisomer or isotopic derivative thereof according to any one of claims 1 to 19, a method for treating a disease by inhibiting the action of PRMT5.

21. The method according to claim 20, wherein the disease is a tumor.
